# EUROPEAN PATENT SPECIFICATION

(11) **EP 1 438 420 B1**
(45) Date of publication and mention of the grant of the patent: **16.09.2009**
(21) Application number: 02782079.4
(22) Date of filing: 24.09.2002
(51) Int. Cl.: G01N 33/92

(54) **METHODS OF USING QUANTITATIVE LIPID METABOLOME DATA**
VERFAHREN ZUR VERWENDUNG VON QUANTITATIVEN LIPIDMETABOLONDATEN
PROCEDE D'UTILISATION DE DONNEES QUANTITATIVES CONCERNANT LE METABOLOME LIPIDIQUE

(30) Priority: 24.09.2001 US 324728 P; 25.01.2002 US 352129 P
(43) Date of publication of application: 21.07.2004
(73) Proprietor: Lipomics Technologies, Inc., West Sacramento, CA 95691 (US)
(72) Inventor: WATKINS, Steven, M., Davis, CA 95616 (US)
(74) Representative: Marshall, Cameron John
(86) International application number: PCT/US2002/030348
(87) International publication number: WO 2003/028271

(56) References cited:
- WO-A-03/005628
- KOLETZKO B ET AL: "FATTY ACID COMPOSITION OF MATURE HUMAN MILK IN NIGERIA" ZEITSCHRIFT FUER ERNAEHRUNGSWISSENSCHAFT, STEINKOPF VERLAG, DARMSTADT, DE, vol. 30, no. 4, 1991, pages 289-297, XP009046785 ISSN: 0044-264X
- ROMO G A ET AL: "Milk composition and apparent digestibilities of dietary fatty acids in lactating dairy cows abomasally infused with cis or trans fatty acids" JOURNAL OF DAIRY SCIENCE, vol. 83, no. 11, November 2000 (2000-11), pages 2609-2619, XP002388189 ISSN: 0022-0302
- MADSEN ARNE ET AL: "Influence of dietary fat on carcass fat quality in pigs: A review" ACTA AGRICULTURAE SCANDINAVICA SECTION A ANIMAL SCIENCE, vol. 42, no. 4, 1992, pages 220-225, XP009068782 ISSN: 0906-4702
- VOLPE ET AL.: 'Regulation of palmitic acid synthesis in cultured glial cells: effects of glucocorticoid on fatty acid synthetase, acetyl-CoA carboxylase, fatty acid and sterol synthesis' JOURNAL OF NEUROCHEMISTRY vol. 27, no. 4, 1976, pages 841 - 845, XP002971984
- GELLHORN ET AL.: 'Lipid biosynthesis in adipose tissue during aging and in diabetes' ANNALS OF THE NEW YORK ACADEMY OF SCIENCES vol. 131, no. 1, 1965, pages 344 - 356, XP002971985
- DATABASE ANSWER [Online] AWAI: 'Abnormal metabolism of fatty acids in diabetes', XP002971986 Database accession no. 1969:25614 -& SOGO RINSHO vol. 17, no. 3, 1968, pages 549 - 564, XP002971987
- WATKINS S.M.: 'Comprehensive lipid analysis: a powerful metanomic tool for predictive and diagnostic medicine' ISRAEL MEDICAL ASSOCIATION JOURNAL vol. 2, no. 9, 2000, pages 722 - 724, XP002971988

## Description

### FIELD OF THE DISCLOSURE

This disclosure relates to the collection of quantitative and compositional data on lipid metabolites, their analysis and linkage of individual metabolites or sets of metabolites or ratios of metabolites to conditions, diseases, and treatments, relating to or influencing weight change, particularly weight gain.

### BACKGROUND OF THE DISCLOSURE

Genomics has fundamentally transformed biological research and is providing astonishing insight into the molecular basis for disease. By determining the genetic sequence of each individual, it will soon be possible to understand the basis for many human diseases at the level of DNA, the blueprint of biology. Genomics can therefore transform medicine from a science based on patient categories into one in which each individual is assessed based on their genetic composition.

Although the potential for genomics to eliminate human disease was anticipated, it is now understood that genomics has many limitations. With few exceptions, diseases are not the simple consequences of genes. Instead, disease almost invariably results from a complex interplay between genes, the environment, and nutrition. Exempting a few overtly genetic diseases, genes can at best predict only the potential for a specific disease outcome. Further, the usefulness of genomic strategies in treating disease is limited.

The bioinformatic analysis of proteins, or the proteome, is widely considered the key correlate to the genome for moving bioinformatic knowledge into clinical and commercial practice. Knowing that proteins carry out the actions of biology, the medical and biotechnology industries are relying on the commercial value of the proteome. The vision is to develop diagnostic tests that detect and measure thousands of proteins in human samples simultaneously, providing information on the presence or risk for specific disease defects. Proteomics will undeniably advance individual health care. However, at present, there are no rapid, quantitative technologies for assembling information about the proteome, much less technologies for bringing the power of proteomics to the individual or even to a doctor's office.

Further complicating the matter, it is estimated that there are possibly 20-times more actual proteins than genes, for instance due to alternative splicing of messages and post-translational protein modifications, and that each regulatory protein could have many potential forms of activation. Gaining a true and comprehensive understanding of protein composition as it relates to health is still a long way from being technically feasible. Once these technologies are reliable and cost effective, they still must be able to demonstrate that the presence or concentration of a protein actually causes a change in the concentration of metabolites or indicates the presence (or absence) of a phenotype.

What is needed is a system for identifying hallmark profiles of metabolism that are capably of integrating and interpreting the influence of genes, nutrition, environment, pharmaceuticals, and toxins on phenotype. Despite optimism from biotechnology that genes and proteins will provide this capability, the truth is that genes, gene expression, and protein concentrations provide data only on the potential for a metabolic or phenotypic effect. Ultimately, it is the concentrations of the metabolites themselves that define phenotype and that exactly reflect the complete metabolic actions of a cell or organism. Until now, no system existed for generating and analyzing such information.

Obesity is a major public health problem affecting nearly a quarter of the adults in the United States, or over 39.8 million people in the U.S. alone; over half of the adult population is overweight (NIDDK statistics available from the National Institutes of Health, 2000). According to the Wealth Health Organization, the number of obese people worldwide has increased from 200 million in 1995 to 300 million in 2000, including 115 million in developing countries. Overweight and obesity are related to more than $99.2 billion per year in related health care costs in the United States alone (direct and indirect costs, Wolf &.Colditz, Obes Res. 6:97-106, 1998). There are few existing therapies capable of modulating weight gain in a safe and efficacious manner. Developing safe therapeutic interventions to prevent obesity will provide a substantial benefit to society. However, weight gain or loss is usually a slow phenotype to develop in response to intervention, and metabolic profiles capable of predicting weight gain in response to interventions, such as therapeutic, nutritional, environmental and genetic intervention, will be highly useful for assessing the effects of these interventions and in developing new forms of intervention.

Koletzko et al. (1991), Zeitschrift für Ernährungswissenschaft 30:289-297 reports significantly higher proportions of saturated fatty acids, in particular lauric acid (C12:0) and myristic acid (C14:0), in the milk of some Nigerian women compared to German women, presumably due to increased *de novo* fatty acid synthesis in the African women consuming a high carbohydrate and low-fat diet.

### SUMMARY OF THE DISCLOSURE

The present invention relates to a method of assessing de novo fatty acid synthesis as defined by the claims. The current disclosure details methods for screening metabolomic data to identify and interpret individual metabolomic profiles as indicative of metabolic status, including particularly weight gain or loss, obesity, and de novo fatty acid synthesis. In various embodiments, this assessment can be used as a means, among other things, to carry out the following:
(1) diagnose disease or health;
(2) assess the metabolic response to a treatment, disease, or condition;
(3) identify the underlying metabolic cause(s) of a phenotype;
(4) predict phenotype based on metabolic profile;
(5) deduce genes, hormones, or other biological factors responsible for producing a metabolic profile;
(6) deduce metabolic targets of dietary components, toxins, pharmaceuticals, environmental or other conditions on the basis of a changed metabolomic profile; and
(7) assess the relative activity of one or more metabolic enzymes between an individual or group of individuals and another individual or group of individuals.

This disclosure deals specifically with using a quantitative assessment of lipid metabolites, including using quantitative assessments for the purposes listed above. In particular embodiments, provided herein are methods of using quantitative lipid metabolomic data (expressed in absolute or relative terms, or in terms of specific ratios between metabolites) to analyze, detect, and predict *de novo* fatty acid synthesis. This is enabled by the identification of lipid metabolites that serve as *de novo* fatty acid synthesis markers. These markers include palmitic acid (16:0), palmitoleic acid (16:1n7), oleic acid (18:1n9), vaccenic acid (18:1n7) and combinations of two or more of these compounds. In particular embodiments, palmitoleic acid is used alone or in combination with other metabolites to provide information about *de novo* fatty acid synthesis in a system or subject. Specific ratios that are correlated with *de novo* fatty acid synthesis include ratios between palmitoleic and palmitic acids (16:1n7 to 16:0), between stearic and palmitic acids (18:0 to 16:0), between oleic (18:1n9) and stearic (18:0), between total n7 desaturated fatty acids and total saturated fatty acids, and between total n7 desaturated fatty acids and total n9 desaturated fatty acids in any particular lipid class.

Also provided in various described embodiments are methods of assessing *de novo* fatty acid synthesis in an organism or a tissue (*e*.*g*., adipose, liver or muscle tissue) of the organism; methods to determine if a pharmaceutical, nutritional, genetic, toxicological or environmental treatment, regimen or dosage influences *de novo* fatty acid synthesis, and/or weight gain or loss; methods to assess a therapeutic or pharmaceutical agent for its potential effectiveness, efficacy or side effects relating to *de novo* fatty acid synthesis, and/or weight gain or loss; methods to screen individuals for compatibility or incompatibility with a pharmaceutical, nutritional, toxicological or environmental treatment; methods to assess the rate or amount of *de novo* fatty acid synthesis as a component of a metabolic status of a research animal; methods of assessing a change in the *de novo* fatty acid synthesis in the organism; and methods of determining whether a treatment or other intervention will cause weight gain or loss in an organism. Also provided are similar methods relating to specific disease conditions, including for instance diabetes, hypo- and hyper-thyroidism, menopause, immuno-tolerance, auto-immunity, chronic inflammation, hormonal dysregulation, and/or cardiovascular disease.

In any of the provided methods, a comparison of or analysis of data can involve a statistical or computer-mediated analysis. Also, any of the provided methods can optionally further involve generating a printed report.

The foregoing and other features and advantages will become more apparent from the following detailed description of several embodiments, which proceeds with reference to the accompanying figures.

### BRIEF DESCRIPTION OF THE FIGURES

**Figure 1** provides a schematic overview of *de novo* fatty acid anabolism in animals. The fatty acids depicted here are those that can be synthesized *de novo* from acetyl-CoA via the activities of fatty acid synthase, along with various desaturases and elongases. Lines represent enzyme actions including fatty acid synthase (FAS), elongation (elongase), and delta-9 (Δ9), delta-5 (Δ5) and delta-6 (Δ6) desaturases.
**Figure 2** is a pair of bar graphs showing the body (FIG 2a) and adipose (FIG 2b) weights of mice treated with rosiglitazone (hatched bars), and corresponding controls (solid bars).
**Figure 3** is a pair of bar graphs showing the body (FIG 3a) and adipose (FIG 3b) weights of mice treated with CL316,243 (hatched bars), and corresponding controls(solid bars).

### DETAILED DESCRIPTION

### I. Abbreviations

- **CE:**: cholesterol ester
- **CL:**: cardiolipin
- **DAG:**: diacylglycerides
- **FAME:**: fatty acid methyl ester
- **FFA:**: free fatty acid
- **LMP:**: lipid metabolite profile
- **LY:**: lyso-phosphatidylcholine
- **MAG:**: monoacylglycerides
- **PC:**: phosphatidylcholine
- **PE:**: phosphatidylethanolamine
- **PI**:: phosphatidylinositol
- **PS:**: phosphatidylserine
- **PS/I**:: phosphatidylinositol / phosphatidylserine
- **SP:**: sphingomyelin
- **TAG:**: triacylglycerol

### II. Explanations of Specific Terms

Unless otherwise noted, technical terms are used according to conventional usage. Definitions of common terms in molecular biology may be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments of the invention, the following explanations of specific terms are provided:

**Biological Sample:** Any biological material, such as a cell, a collection of cells (*e*.*g*., cultured cells), a tissue sample, a biopsy, or an organism. Biological samples also include blood and blood products (*e*.*g*., plasma) and other biological fluids (*e*.*g*., tears, sweat, saliva and related fluids, urine, tears, mucous, semen, and so forth). Tissue samples can be from any organ or tissue in the body, include heart, liver, muscle, adipose, brain, lung, testes, and brain.

Biological samples may be from individual subjects (*e*.*g*., animals, such as humans, mice, rats, guinea pigs, monkeys, cats, dogs, pigs, horses, cows, fruit flies, or worms) and/or archival repositories. The samples may be acquired directly from the individuals, from clinicians (for instance, who have acquired the sample from the individual), or directly from archival repositories.

***De novo* fatty acid synthesis:** The biochemical processes of producing fatty acids from substrate(s) within an organism. These processes stand in contrast to the accumulation of fatty acids within an organism that takes place following the consumption of foods containing fatty acids.

For the purpose of this disclosure, *de novo* fatty acid synthesis does not refer to modifications to dietary fatty acids that are performed endogenously by a subject. As used herein, *de novo* fatty acid synthesis refers to the process of creating fatty acids from acetyl-CoA substrate. This process can ultimately yield a variety of fatty acid structures. A non-limiting list of fatty acids that can be produced *de novo* include: palmitic acid, myristic acid, stearic acid, palmitoleic acid, oleic acid, vaccenic acid and nervonic acid, and combinations of two or more of these compounds.

The primary enzymes involved in *de novo* fatty acid synthesis include fatty acid synthase, acetyl-CoA carboxylase, stearoyl-CoA desaturase (Δ9-desaturase) and elongase. The biosynthesis of fatty acids is largely similar among plants and animals. Both are capable of producing fatty acids de novo from acetyl CoA via the concerted action of acetyl CoA carboxylase and fatty acid synthase. The first step in the *de novo* synthesis of fatty acids involves the production of malonyl CoA from acetyl CoA, a reaction catalyzed by acetyl CoA carboxylase. Acetyl CoA carboxylase carries out two partial reactions, each catalyzed at distinct sites, which first carboxylate the reaction cofactor biotin and second, transfer the carboxyl group to acetyl CoA.

In animals, acetyl CoA carboxylase is found in the cytosol and appears to be regulated by a number of factors including long chain acyl CoA, providing sensitivity to both *de novo* production of acyl chains and diet. The second general step in the production of fatty acids is activation of both malonyl CoA and the primary unit of condensation, acetyl CoA. The activated malonyl complex then enters a cycle of elongation catalyzed by the soluble enzyme complex, fatty acid synthase. Fatty acid synthase lengthens the acyl chain by two carbons per cycle of activity, using acetyl CoA as the condensing unit. This series of reactions generally culminates in the production of palmitic acid. The cycle is terminated when thioesterase hydrolyzes the growing acyl chain and releases a fatty acyl CoA. Upon removal from the fatty acid synthase complex, fatty acids can be further modified by elongases or the stearoyl-CoA desaturase to produce other forms of *de novo* synthesized fatty acids.

**Informatics:** A global term used to describe the statistical or mathematical analysis of a large collection of data. This data is usually produced by modem, "high throughput" scientific techniques. Such "high-throughput" techniques include genomic analyses and proteomic analyses, as well as metabolomic analyses. Informatics is also a term used to describe the field of study focused on developing statistical and mathematical techniques for analyzing the large biological datasets. Informatics stands in contrast to standard "reductionist" research, wherein investigators design the experimental system to aid in the interpretation of the results. Informatics focuses on obtaining and analyzing large amounts of accurate data that are often from uncontrolled populations or experimental models. Informatic analyses generally test hypotheses *in silico* rather than at the laboratory bench. This method of investigation is suited to genomics, where sequences from disparate sources are integrated easily into one database because the genetic code is essentially universal. Because metabolomic data is influenced by the environment, and can be different depending on the time and conditions under which the sample is taken, a metabolomic database involves providing for considerably more complexity than is seen in a genomic database.

***In silico* research:** Literally referring to "in computer" systems, *in silico* research involves methods to test biological models, drugs and other interventions using computer models rather than laboratory (*in vitro*) and animal (*in vivo*) experiments. *In silico* methods can involve analyzing an existing database, for instance a database that includes one or more records that include quantitative analysis of a metabolite (*e*.*g*., a lipid metabolite). Analysis of such databases may include mining, parsing, selecting, identifying, sorting, or filtering of the data in the database. Data in the database can also be subjected to one or more computational analyses, such as statistical conversions. The data may be subjected to a clustering algorithm, discrimination algorithm, difference test, correlation, regression algorithm or other statistical modeling algorithm.

Using *in silico* research, drug targets can be identified and validated, candidate drugs can be selected, tested, and prioritized, and experimental strategies can be assessed. *In silico* systems complement laboratory-based research, yet increase productivity and efficiency by minimizing the need for *in vitro* and *in vivo* laboratory experiments.

In certain embodiments provided herein, *in silico* diagnostic systems are used. In particular, this disclosure provides *in silico* diagnostic methods for assessing a condition related to *de novo* fatty acid synthesis. Such methods involve assessing data in a database, such as a lipomic database. The data in the database usually includes a quantity of at least one marker of *de novo* fatty acid synthesis from a biological sample from one or more individuals. The quantity of at least one marker of *de novo* fatty acid synthesis from the biological sample is correlated with *de novo* fatty acid synthesis. In specific examples of these methods, markers of *de novo* fatty acid synthesis are palmitoleic acid, vaccenic acid, palmitic acid, stearic acid, oleic acid, myristic acid, or a combination of any two or more thereof.

**Lipid:** As used herein, the term lipid refers to a class of water-insoluble, or partially water insoluble, oily or greasy organic substances, that are extractable from cells and tissues by nonpolar solvents, such as chloroform or ether. The most abundant kinds of lipids are the fats or triacylglycerols, which are major fuels for most organisms. Another class of lipids is the polar lipids, which are major components of cell membranes. The following table (Table 1) provides one way of grouping major types of lipids; these have been grouped according to their chemical structure:

**Table 1:**

| **Lipid type** | **Representative examples or sub-groups** |
|---|---|
| Triacylglycerols | |
| Waxes | |
| Phosphoglycerides | phosphatidylethanolamine |
| | phosphatidylcholine |
| | phosphatidylserine |
| | phosphatidylinositol |
| | cardiolipin |
| Sphingolipids | sphingomyelin |
| | cerebrosides |
| | gangliosides |
| Sterols and their fatty acid esters | *(see Table 3)* |

Lipid metabolites may also be broken down into other recognized classes, such as those shown in Table 2:

**Table 2**

| **SCIENTIFIC NAME** | **ABBREVIATION** |
|---|---|
| Lyso-Phosphatidylcholine | LY |
| Sphingomyelin | SP |
| Phosphatidylcholine | PC |
| Phosphatidylserine | PS |
| Phosphatidylinositol | PI |
| Phosphatidylethanolamine | PE |
| Phosphatidylglycerol | PG |
| Cardiolipin | CL |
| Free Fatty Acids | FFA |
| Monoacylglycerides | MAG |
| Diacylglycerides | DAG |
| Triacylglycerides | TAG |
| Cholesterol Esters | CE |

Also included in the term lipid are the compounds collectively known as sterols. Table 3 shows representative sterols.

**Table 3:**

| **SCIENTIFIC NAME** | **MOLECULAR FORMULA** | **COMMON NAME** |
|---|---|---|
| 5b-cholestan-3b-ol | C₂₇H₄₈O | coprostanol |
| 5a-cholestan-3b-ol | C₂₇H₄₈O | dihydrocholesterol |
| 5-cholesten-3b-ol | C₂₇H₄₆O | cholesterol |
| 5,24-cholestadien-3b-ol | C₂₇H₄₄O | desmosterol |
| 5-cholestan-25a-methyl-3b-ol | C₂₈H₄₂O | campesterol |
| 5-cholestan-24b-methyl-3b-ol | C₂₈gH₄₂O | dihydrobrassicasterol |
| 5-cholesten-24b-ethyl-3b-ol | C₂₉H₅₀O | b-sitosterol |
| 5,22-cholestadien-24b-ethyl-3b-ol | C₂₉H₄₈O | stigmasterol |

**Metabolite:** A biomolecule that has a functional and/or compositional role (such as a component of a membrane) in a biological system, and which is not a molecule of DNA, RNA, or protein. Examples of metabolites include lipids, carbohydrates, vitamins, co-factors, pigments, and so forth. Metabolites can be obtained through the diet (consumed from the environment) or synthesized within an organism. Proteins exist in large part to break down, modify, and synthesize metabolites. Metabolites are not only directly responsible for health and disease, but their presence in a biological system is the result of a variety of factors including genes, the environment, and direct nutrition. By profiling the metabolite composition of a biological sample, for instance using the methods described herein, data on genotype, metabolism, and diet can be obtained in great detail. This data can be linked to clinical information and used to identify the true biochemical basis for health and disease.

Lipids are perhaps the most important subset of metabolites, because dietary lipids and lipid metabolism are clearly linked to the incidence and progression of several major degenerative diseases, including heart disease, diabetes, obesity, auto-immunity, and chronic inflammation. Moreover, because lipids are the only major nutrients that survive digestion intact, highly accurate information on individual nutrition can be gained from a lipid metabolite profile. Thus, a lipid metabolomic approach provides information encompassing the entire spectrum of factors that influence disease.

Each fatty acid may be found as a component of any lipid class, and in such combination is a different metabolite than it is on its own (free) or as a component in any other lipid class. Thus, palmitoleic acid in cholesterol esters is a distinct metabolite from palmitoleic acid in triacylglycerides, and so on. By way of example, if a system is used in which lipids are categorized into thirteen classes (as shown in Table 2), and there an analysis determines the concentration of 38 fatty acids in each class, then 13 x 38, or 494 specific metabolite concentrations may be determined.

**Metabolomics:** Analysis of metabolite concentrations in a biological sample in a comprehensive fashion. There are several levels of metabolomics - these can be differentiated for instance based on the scope of the individual metabolite profile, where scope refers to the number or type of metabolites measured in the individual analysis. Thus, lipid metabolomics is the study or analysis of a set of individual lipid metabolites. Carbohydrate metabolomics is the study or analysis of a set of individual carbohydrate metabolites. The set of data produced from analysis of an individual sample is referred to herein as an individual **lipid metabolite/metabolic profile ("lipomic profile**") of that sample. Certain examples of lipid metabolite profiles include a highly comprehensive set of metabolite measurements (a profile) by multi-parallel analyses.

The comparison of two metabolite profiles of similar scope (*i*.*e*., containing information about the same or a similar or overlapping set or subset of metabolites) from cells/tissues/subjects that have been differently treated, or that are genetically different or different based on disease state or condition, provides information on the metabolic effects of the difference.

A metabolome is a data set that includes concentrations of metabolites in a biological system (*e*.*g*., a cell, tissue, biological fluid, or whole subject) under specific conditions; a multidimensional metabolome includes such data from like samples over a variety of conditions (*e*.*g*., time points, treatment points, different drug or other treatments, and so forth).

Quantitative metabolomic data as discussed herein include molar quantitative data, mass quantitative data, and relational data by either moles or mass (mole % or weight %, respectively) for individual metabolites, or subsets of metabolites. Quantitative aspects of metabolomic samples may be provided and/or improved by including one or more quantitative internal standards during the analysis, for instance one standard for each lipid class (in a lipomic profile). Internal standards described herein enable true quantification of each fatty acid from each lipid class, whereas traditional lipid analysis methods produce data in either a percent-of-total format or as a mixed population of lipid metabolites. Provided internal standards are designed to reflect any loss of fatty acid due to oxidation, discrimination, or cross-contamination.

Ratios of lipid metabolites may also be used to reflect or assess changes in lipid metabolism. These ratios require only relational data when lipid metabolites contained in the numerator and the denominator are all taken from the same lipid class. However if lipid metabolite ratios are calculated from metabolites not present in the same lipid class, the data used to calculate the ratio should be quantitative.

Truly quantitative data can be integrated from multiple sources (whether it is work from different labs, samples from different subjects, or merely samples processed on different days) into a single seamless database, regardless of the number of metabolites measured in each discrete, individual analysis.

**Metabolite fingerprint (or linked profile):** A distinct or identifiable pattern of metabolite levels, or ratios of such levels, for instance a pattern of high and low metabolites of a defined set. A representative "set" is a biogenerative pathway; a non-inclusive list of other sets includes biodegenerative pathways, disease sets (linked to a specific disease), fitness sets (linked to a level or type of fitness of the subject), and so forth. In specific embodiments, the metabolite levels in the fingerprint are absolute metabolite concentrations. Metabolite fingerprints (also referred to as linked profiles, *e*.*g*., a disease-linked profile or toxin-linked profile) can be linked to a tissue or cell type, to a particular stage of normal tissue growth or disease progression, to a dietary limitation or supplementation, or to any other distinct or identifiable condition that influences metabolite levels (*e*.*g*., concentrations) in a predictable or associatable way. Metabolite fingerprints can include relative as well as absolute levels of specific metabolites, but absolute levels (*e*.*g*., concentrations) are preferred in many embodiments. Specific examples of metabolite fingerprints are lipid metabolite fingerprints.

**Nutritional treatment or intervention:** The terms nutritional intervention or treatment include any process of providing a specific food, fluid (*e*.*g*, beverage) or supplement to an subject. This process can be intentional, as takes place in a nutritional trial, or it can be assessed retroactively by questioning the subject or by otherwise determining the nutritional status of a subject A "nutritional component" is any molecule or plurality of molecules consumed by a subject through the diet

**PharmaceuticaUtherapeutic agent:** Any agent, such as a protein, peptide (*e*.*g*., hormone peptide), other organic molecule or inorganic molecule or compound, or combination thereof, that has one or more effects on a biological system, such as a desired therapeutic or prophylactic effect when properly administered to a subject

**Subject:** Living multi-cellular vertebrate organisms, a category that includes both human and non-human mammals.

Unless otherwise explained, all technical and scientific terms used herein have the same meaning as commonly understood by one of ordinary skill in the art to which this invention belongs. The singular terms "a," "an," and "the" include plurals unless context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of the present invention, suitable methods and materials are described below. In case of conflict, the present specification, including explanations of terms, will control. In addition, the materials, methods, and examples are illustrative only and not intended to be limiting.

### III. Overview of Several Embodiments

Bioinformatic strategies that embrace the study of metabolites have potential for (1) identifying hallmarks of individual metabolism, (2) identifying the metabolic hallmarks of disease, and (3) determining the complex and integrative effects of metabolism, genes, nutrition, and environment on health. Further, metabolomics will enalite researchers, clinicians, and even individuals to monitor the individual metabolic response of a person or research animal to a treatment. This approach has the potential to allow individuals to assess how a specific intervention, such as change in diet or application of pharmaceutical agent, affects their metabolism.

This disclosure provides in certain embodiments methods of assessing *de novo* fatty acid synthesis in an organism or a tissue of the organism, for instance a research animal or human. In some embodiments, such methods involve quantifying a marker of *de novo* fatty acid synthesis in a biological sample from the organism, wherein the marker of *de novo* fatty acid synthesis includes palmitoleic acid, vaccenic acid, palmitic acid, stearic acid, oleic acid, myristic acid, or a combination of any two or more thereof. In specific examples of provided methods, a ratio of two metabolites is used. These markers are useful when they are quantified as free fatty acids or as part of other lipid molecules, for instance glycerolipids, such as triacylglycerides, cholesterol esters, and so forth.

In various embodiments, the biological sample is a fluid or tissue sample, or a cell or extract prepared from a cell, cell culture, or cell preparation. Contemplated fluid samples include blood and blood products (*e*.*g*., plasma) and other biological fluids (*e*.*g*., tears, sweat, saliva and related fluids, urine, tears, mucous, semen, and so forth). Contemplated tissue samples may be any tissue sample taken from a subject, including heart, liver, adipose, brain, and muscle tissue. It is specifically contemplated that *in vitro* cultured cells can be used, for instance immortalized cells taken from a subject.

Certain disclosed embodiments are methods of assessing *de novo* fatty acid synthesis in adipose tissue, liver tissue or muscle tissue. In some such specific embodiments, the method is a method to assess *de novo* fatty acid synthesis in adipose tissue, and a marker of *de novo* fatty acid synthesis is quantified from the free fatty acid fraction of a blood product. In other specific embodiments, the method is a method to assess *de novo* fatty acid synthesis in liver tissue, and a marker of *de novo* fatty acid synthesis is quantified from the phosphatidylcholine, triacylglyceride, or cholesterol ester fraction of a blood product.

Also provided are methods to determine if a pharmaceutical, developmental, nutritional, genetic, toxicological or environmental treatment, regimen or dosage influences *de novo* fatty acid synthesis, which methods involve quantifying a marker of *de novo* fatty acid synthesis in a biological sample from the organism.

Further embodiments are methods to assess or identify a therapeutic or pharmaceutical agent for its potential effectiveness, efficacy or side effects relating to *de novo* fatty acid synthesis, which methods involve quantifying a marker of *de novo* fatty acid synthesis in a biological sample from the organism.

Also provided are methods to screen individuals for compatibility or incompatibility with a pharmaceutical, nutritional, toxicological or environmental treatment, which methods involve quantifying a marker of *de novo* fatty acid synthesis in a biological sample from the organism.

Still further embodiments are methods to assess the rate or amount of *de novo* fatty acid synthesis as a component of a metabolic status of an animal, such as a companion or research animal, which methods involve quantifying a marker of *de novo* fatty acid synthesis in a biological sample from the organism. In specific examples of such methods, the metabolic status of the research animal is a normal or baseline metabolic state.

Optionally, in some of the provided embodiments, the methods further involve comparing the assessment of *de novo* fatty acid synthesis from the organism to an assessment of *de novo* fatty acid synthesis from another organism (from the same or a different species) or an assessment compiled for a population of organisms (from one or a mixture of species that may be the same or different from the test organism or subject). Some examples of such methods further involve scoring the test or subject organism based on the comparison, for instance to provide a score that is correlated to weight gain or loss, growth, or wasting, or a score based on metabolite concentrations, such as a percentile rank. In some examples of provided methods, the comparison is a comparison between the quantity of one or more markers of *de novo* fatty acid synthesis.

Also provided herein are methods of assessing a change in the *de novo* fatty acid synthesis in the organism, wherein the methods involve taking at least two biological samples from the organism, one of which is taken before and one after an event. In various specific embodiments, the event involves passage of time (*e*.*g*., minutes, hours, days, weeks, months, or years), treatment with a therapeutic agent (or putative or potential therapeutic agent), treatment with a pharmaceutical agent (or putative or potential pharmaceutical agent), treatment with a nutritional regimen, treatment with a genetic modification, exposure to a toxic or potentially toxic compound, exposure to an environmental condition, treatment with a laboratory procedure, exercise (*e*.*g*., an increase, decrease, or change in an exercise regimen), or the appearance of a phenotypic state.

In certain of the provided methods, the quantity of a marker of *de novo* fatty acid synthesis is correlated to a propensity, risk, or metabolic basis for weight gain or loss of the organism, and the method is a method for determining the propensity, risk, or metabolic basis for weight gain or loss of the organism. Optionally, such methods may further involve correlating the quantity of the marker of *de novo* fatty acid synthesis with *de novo* fatty acid synthesis in adipose, wherein the marker of *de novo* fatty acid synthesis is quantified from the free fatty acid fraction of a blood product. Other provided methods involve correlating the quantity of the marker of *de novo* fatty acid synthesis with *de novo* fatty acid synthesis in the liver, wherein the marker of *de novo* fatty acid synthesis is quantified from the phosphatidylcholine, triacylglyceride, or cholesterol ester fraction of a blood product.

In still other provided methods, the quantity of a marker of *de novo* fatty acid synthesis is correlated to a propensity, risk, or metabolic basis for diabetes, obesity, cardiovascular disease, and/or hormonal dysregulation of the organism, and the method is a method for determining the propensity, risk, or metabolic basis for such.

In specific provided embodiments, it is a ratio of quantities of metabolites or metabolite categories (*e*.*g*., all n7 or all n9 or all saturated fatty acids) that is so correlated.

Also provided are methods of determining whether a treatment or other intervention will cause weight gain or loss in an organism, which methods involve taking at least two biological samples from the organism, wherein the two samples are taken before and after a nutritional, pharmacological, genetic, environmental or toxicological intervention treatment, and wherein a change in the quantity of a marker of *de novo* fatty acid synthesis measured in these samples is correlated with a likelihood of weight gain or loss.

Optionally, the provided methods for examining weight gain or loss in an organism may further involve comparing the assessment of *de novo* fatty acid synthesis from the organism to an assessment of *de novo* fatty acid synthesis from another organism or compiled for a population of organisms. Specific examples of such methods further involve predicting a propensity of the individual organism for weight gain based on the comparison.

In embodiments of the provided methods, the quantity of a marker of *de novo* fatty acid synthesis is reported as an absolute or relative concentration. For instance, methods are provided wherein correlating the quantity of a marker of *de novo* fatty acid synthesis involves using the absolute or relative concentration of the marker of *de novo* fatty acid synthesis in a mathematical or statistical equation for determining the amount of *de novo* fatty acid synthesis. In specific examples, the marker of *de novo* fatty acid synthesis is a ratio of the quantity of two individual metabolites, which themselves might separately be markers of *de novo* fatty acid synthesis.

Also disclosed are *in silico* diagnostic methods, particularly methods for determining an effect related to *de novo* fatty acid synthesis. In some embodiments, these methods include assessing a condition related to *de novo* fatty acid synthesis, which involves assessing data in a database, wherein the data in the database includes a quantity of at least one marker of *de novo* fatty acid synthesis from a biological sample from the individuals. The quantity of the at least one marker of *de novo* fatty acid synthesis from the biological sample is correlated with *de novo* fatty acid synthesis. Alternatively, a ratio of the quantities of two metabolites may be used in such methods. In various embodiments, markers of *de novo* fatty acid synthesis include palmitoleic acid, vaccenic acid, palmitic acid, stearic acid, oleic acid, myristic acid, or a combination of any two or more thereof. As contemplated herein, assessing data in the database can include mining, parsing, selecting, identifying, sorting, and/or filtering the data or otherwise subjecting it to an analysis algorithm.

Certain of the disclosed *in silico* diagnostic methods are methods of assessing weight gain or growth or the potential for weight gain or growth, which methods involve correlating *de novo* fatty acid synthesis with weight gain or growth or the potential for weight gain or growth.

In specific examples of the *in silico* diagnostic methods, the quantity of a marker of *de novo* fatty acid synthesis is reported as an absolute or relative concentration. Some of these methods further involve using the absolute or relative concentration of the marker of *de novo* fatty acid synthesis in a mathematical or statistical equation for determining the amount of *de novo* fatty acid synthesis. In specific examples, the marker of *de novo* fatty acid synthesis is a ratio of the quantity of two individual metabolites, which themselves might separately be markers of *de novo* fatty acid synthesis.

Some of the provided methods involve using the absolute or relative concentration of the marker of *de novo* fatty acid synthesis in a mathematical or statistical equation for determining a propensity for a future body weight, a cause of current body weight, or a change in body weight.

In other provided methods, correlating the quantity of the marker of *de novo* fatty acid synthesis involves using the absolute or relative concentration of the marker of *de novo* fatty acid synthesis in an mathematical or statistical equation for determining a propensity for a change in body composition, a propensity for a change in total body fat, a change in body composition, or a change in total body fat.

In still other of the provided methods, correlating the quantity of the marker of *de novo* fatty acid synthesis involves using the absolute or relative concentration of the marker of *de novo* fatty acid synthesis in an mathematical or statistical equation for determining a propensity for growth or a cause of growth. In specific examples, the marker of *de novo* fatty acid synthesis is a ratio of the quantity of two individual metabolites, which themselves might separately be markers of *de novo* fatty acid synthesis.

In some examples of the provided methods, more metabolites are assessed than merely markers of *de novo* fatty acid synthesis. Thus, some of the *in silico* diagnostic methods specifically involve assessing a concentration or relative concentration of a lipid metabolite (or a non-lipid metabolite) other than palmitoleic acid, vaccenic acid, palmitic acid, stearic acid, oleic acid or myristic acid.

In certain examples of the provided methods, assessing data in a database involves using palmitoleic acid, vaccenic acid, palmitic acid, stearic acid, oleic acid, all n7 fatty acids, all n9 fatty acids, all unsaturated fatty acids, or a combination of two or more thereof as part of a clustering algorithm, discrimination algorithm, difference test, correlation, regression algorithm or other statistical modeling algorithm. Such methods can be used for instance to determine or predict an effect of a condition on *de novo* fatty acid synthesis in a subject.

In still other provided methods, the quantity of a marker of *de novo* fatty acid synthesis (reported in either an absolute or relative concentration, or a ratio thereof) is correlated to a propensity, risk, or metabolic basis for growth or wasting of a subject. Thus, some of the provided methods are methods for determining the propensity, risk, or metabolic basis for growth or wasting of a subject, which methods involve quantifying a marker of *de novo* fatty acid synthesis in a biological sample of the subject, wherein the marker of *de novo* fatty acid synthesis is palmitoleic acid, vaccenic acid, palmitic acid, stearic acid, oleic acid or myristic acid, or a combination of two or more thereof. In some of these embodiments the assessment of *de novo* fatty acid synthesis from the organism is compared to an assessment of *de novo* fatty acid synthesis from another organism or compiled for a population of organisms. Specific examples of such methods further involve predicting a propensity of the organism for growth or wasting based on the comparison.

In specific examples of such methods, the quantity of the marker of *de novo* fatty acid synthesis is further correlated with *de novo* fatty acid synthesis in adipose, and the marker of *de novo* fatty acid synthesis is quantified from the free fatty acid fraction of a blood product. In other examples, the quantity of the marker of *de novo* fatty acid synthesis is correlated with *de novo* fatty acid synthesis in the liver, and the marker of *de novo* fatty acid synthesis is quantified from the phosphatidylcholine, triacylglyceride, or cholesterol ester fraction of a blood product.

Also provided are methods of determining whether a treatment will cause growth or wasting. These methods involve taking at least two biological samples from the organism, wherein the two samples are taken before and after a nutritional, pharmacological, genetic, environmental or toxicological intervention treatment, and wherein a change in the quantity of the marker of *de novo* fatty acid synthesis is correlated with a likelihood of growth or wasting.

In still other of the provided methods in which the quantity of a marker of *de novo* fatty acid synthesis is correlated to a propensity, risk, or metabolic basis for growth or wasting of a subject, correlating the quantity of the marker of *de novo* fatty acid synthesis involves using the absolute or relative concentration of the marker of *de novo* fatty acid synthesis in an mathematical or statistical equation for determining the amount of *de novo* fatty acid synthesis.

One specific provided embodiment is a method of determining whether or to what extent a condition influences *de novo* fatty acid synthesis. This method involves subjecting a subject to the condition, taking a biological sample from the subject, analyzing the biological sample to produce a test lipomic profile for the subject, which profile comprises a total quantity (in absolute or relative terms) of at least one marker for *de novo* fatty acid biosynthesis, and comparing the test lipomic profile for the subject with a control lipomic profile, which profiles comprise a total quantity of the at least one marker for *de novo* fatty acid biosynthesis. From this comparison, conclusions are drawn about whether or to what extent the condition influences *de novo* fatty acid synthesis based on differences or similarities between the test lipomic profile and the control lipomic profile. Specific examples of markers for *de novo* fatty acid synthesis that are used in this method include palmitoleic acid, vaccenic acid, palmitic acid, stearic acid, oleic acid, all n7 fatty acids, all n9 fatty acids, all unsaturated fatty acids, and combinations of two or more thereof. As contemplated for this embodiment, a condition to which the subject is subjected can include but is not limited to a genotype, such as a genetic knockout, a dietary limitation or supplementation, a disease or disease state, application of a toxin or suspected toxin, application of a pharmaceutical or therapeutic agent or candidate agent, an increase in exercise, a decrease in exercise, or a change in an exercise regimen of the subject.

In specific examples of such methods, the control lipomic profile is a compiled lipomic profile assembled from a plurality of individual lipomic profiles. In other examples, the control lipomic profile is a pre-condition lipomic profile from the subject.

Also provided is a method of determining drug or treatment effectiveness or side effects, which method involves applying a drug or treatment to a subject, taking a biological sample from the subject, and analyzing the biological sample to produce a test lipomic profile for the subject, which profile comprises a total quantity of at least one marker for *de novo* fatty acid synthesis. The test lipomic profile for the subject is compared with a control lipomic profile, which profile comprises a total quantity of the at least one marker for *de novo* fatty acid synthesis; and conclusions are drawn about the effectiveness or side effects of the drug or treatment based on differences or similarities between the test lipomic profile and the control lipomic profile. Specific examples of markers for *de novo* fatty acid synthesis that are used in this method include palmitoleic acid, vaccenic acid, palmitic acid, stearic acid, oleic acid, myristic acid, and combinations of two or more thereof. As contemplated for this embodiment, a drug or treatment that is applied to the subject can include a hormone or hormone treatment, a drug or treatment relates to controlling obesity or diabetes, a drug or treatment relates to controlling cardiovascular disease, a drug or treatment relates to modifying lipid metabolism, a nutritional intervention, or an exercise program.

Further provided embodiments are methods of assessing fatty acid synthesis, which methods specifically include quantifying palmitoleic acid in a biological sample (such as a blood product). In examples of these methods, the palmitoleic is quantified from the free fatty acid fraction of a blood product and the method is a method to assess *de novo* fatty acid synthesis in adipose tissue.

Another disclosed method involves quantifying palmitoleic acid and palmitic acid in a biological sample from an organism, for instance, within a specific lipid class. Such methods may further include generating a ratio indicator of *de novo* fatty acid synthesis, wherein the ratio indicator is the ratio of the quantity of palmitoleic acid to the quantity of palmitic acid.

Yet a further disclosed method involves quantifying stearic acid and palmitic acid in a biological sample from an organism, for instance, within a specific lipid class. Such methods in some circumstances may further include generating a ratio indicator of *de novo* fatty acid synthesis, wherein the ratio indicator is the ratio of the quantity of stearic acid to the quantity of palmitic acid.

A still further disclosed method involves quantifying total n7 fatty acids and total saturated fatty acids in a biological sample from an organism, for instance, within a specific lipid class. Such methods may further include generating a ratio indicator of *de novo* fatty acid synthesis, wherein the ratio indicator is the ratio of the quantity of total n7 fatty acids to the quantity of total saturated fatty acids.

Also provided are methods involving quantifying total n7 fatty acids and total n9 fatty acids in a biological sample from an organism, for instance, within a specific lipid class. Such methods may further include generating a ratio indicator of *de novo* fatty acid synthesis, wherein the ratio indicator is the ratio of the quantity of total n7 fatty acids to the quantity of total n9 fatty acids.

In any of the provided methods, a comparison of or analysis of data can involve a statistical or computer-mediated analysis.

Any of the provided methods can further involve generating a printed report, for instance a report of some or all of the data, of some or all of the conclusions drawn from the data, or of a score or comparison between the results from a subject or individual and other individuals or a control or baseline.

### IV. Lipomic Assessment of Metabolism, Development and Phenotype

The massively parallel measurement of gene expression has become a standard approach for identifying the genetic and metabolic basis for a biological difference. Essentially, the expression of genes as mRNA from two samples or groups of samples are measured and compared to determine which genes are more or less expressed in a relative sense between the samples. These data are then interpreted as the basis for altered metabolism. This approach has many advantages, the most salient being that the expression of every gene can be measured simultaneously and that each gene transcript can be linked directly to a metabolic pathway. However, there are several disadvantages to the approach as well, namely that increased gene expression does not guarantee a change in the metabolic status of a subject. The mere presence of mRNA does not indicate that (1) the mRNA will be translated into a protein, (2) that the protein will be activated, or (3) that the protein will be present at the appropriate site to catalyze the desired reaction. Additionally, assays that rely on relative or comparison data are not intrinsically quantitative, and thus, not easily organized into a seamless and minable database.

By contrast, the highly parallel and quantitative assessment of metabolites allows for the creation of an infinitely expandable and minable database. Perhaps even more importantly, where the concentrations of metabolites change, it is the unequivocal consequence of altered metabolism. Therefore, constructing and mining a metabolomic database will allow the identification of metabolite hallmarks of metabolic processes. This database and the resulting knowledge created from it can be used to develop diagnostics, markers, or profiles of specific metabolic processes. Where these processes are linked with phenotype, the metabolomic measurements themselves can be used as diagnostics or predictive diagnostics for the phenotype. Once the metabolomic profile of a specific metabolic process is known that profile can be used to identify the targets of nutritional components, pharmaceuticals, toxins, environmental influences and the functions of genes and proteins. As an example, a thiadolidinedione drug binds the PPARγ receptor and elicits a series of metabolic responses. At the level of metabolic control, the binding of the PPARγ receptor can induce hormone synthesis or secretion, and induce the binding of DNA transcription factors among other possible affects. Each of these metabolic control mechanisms can control the concentration, activity, or specificity of individual enzymes. These enzymes in turn modulate the concentrations of metabolites, and it is the metabolites that in turn cause and define phenotype. Thus, by generating quantitative data on the metabolome, it is possible to "fingerprint" (profile) the effects of (1) single enzymes, (2) metabolic control mechanisms, such as hormones, and (3) treatments or affectors such as pharmaceuticals or nutritional components. In addition, the quantitative metabolomic profile serves as a marker or diagnostic for the action and efficacy of any treatment that affects lipid metabolism.

Ratios of fatty acids or lipid classes can be informative to changes in lipid metabolism. As the activity of one or more enzymes change, the ratio of substrate lipid metabolites used to product lipid metabolites can indicate the direction of the change. Increases in enzyme activity will be reflected by increases in the ratio of a product to its substrate(s). Likewise, decreases in enzyme activity will be reflected by decreases in the ratio of a product to its substrate(s). These ratios can be ratios of two lipid metabolites or ratios of complex relationships among metabolites. Further, the lipid metabolites do not need to be direct product-substrate metabolites of specific enzymes (or a specific enzyme), but can be ratios of any two or more metabolites. In this respect, it is important that the data used to generate ratios is quantitative, as mole percentage data are not appropriate for comparing two lipid metabolites that are present within different lipid classes.

### V. Individual Markers and Profiles

A quantitative assessment of fatty acids allows for the investigation of fatty acid concentration in both absolute and relative terms. Only quantitative data will allow for the investigation of the molar relationships among all fatty acids, regardless of which lipid class the fatty acid is acylated into. Additionally, quantitative data enables the creation of an expandable database of lipid metabolites that can be investigated *in silico.* Quantitative data is also easily converted into relative data (mole percentage, weight percentage, etc.) for investigating fatty acid metabolism within an individual lipid class. By converting quantitative data to mole percentage data, for instance, an investigator can identify the relative abundance of the fatty acid within the class. This approach can make identifying changes in lipid class metabolism easier than investigating the data in quantitative terms. Thus, the assessment of *de novo* fatty acid synthesis by measuring fatty acid composition can be approached both from quantitative and relational data formats, although it is suggested that the data is collected in quantitative terms.

There are many ways to collect quantitative or relational data on lipid metabolites, and the analytical methodology does not affect the utility of metabolite concentrations in predicting phenotype or assessing metabolism. One method described herein for generating quantitative and mole percentage data on fatty acids in lipid classes involves gas chromatography coupled with flame ionization detection. Other methods for generating data on lipid metabolites include but are not limited to high-performance liquid chromatography, mass spectrometry, capillary electrophoresis, thin layer chromatography, immunoassay, RNA switches, nuclear magnetic resonance, etc. The specific methodology used to generate the quantitative lipid metabolite date is essentially irrelevant to this disclosure, which is focused on the use of lipid metabolite data, for instance to identify metabolic process or to identify or predict phenotype, including the specific embodiments described herein.

### A. Markers for Endogenous Fatty Acid Biosynthesis and Accompanying Phenotypes

It has been found using the methods disclosed herein that assessment or measurement of the absolute or relative concentration of palmitoleic acid (16:1n7) or its immediate elongation product, vaccenic acid (18:1n7), in biological samples can be used as a measurement of *de novo* fatty acid synthesis. Assessment of *de novo* fatty acid synthesis by measuring other fatty acids including palmitic acid (16:0), stearic acid (18:0), oleic acid (18:1n9), myristoleic acid (14:0), all n7, all n9, or all saturated fatty acids, can also be used for the same purposes. Each of these markers, alone or in combination with other markers, can be used to analyze and predict phenotypes and manifestations linked to *de novo* fatty acid in much the same way as cholesterol is used as a marker for heart disease. Similarly, markers or profiles of sets of markers can be correlated to the activity of one or more specific enzymes involved in *de novo* fatty acid synthesis.

In some embodiments, the lipid class in which these *de novo* fatty acid synthesis markers are found is an indication of the location of the increased or decreased fatty acid synthesis.

Measurement of these compounds, either from biological samples or *in silico* from a table or database, can be, among other things, used for:
(1) the assay of the activity of one or more of the enzymes involved in *de novo* fatty acid synthesis;
(2) the bulk process of *de novo* fatty acid synthesis itself
(3) the measurement of processes in which *de novo* fatty acid synthesis is a component (either as a direct assay of the process or as a constituent part of a profile to assay this process);
(4) phenotypes or the propensity to express a phenotype that results from or is related to *de novo* fatty acid synthesis, such as weight gain or loss, growth and hypo- or hyperlipidemia, and
(5) identification and testing/characterization of compounds or non-compound influences (such as exercise, dietary changes, nutritional treatment, and so forth) regarding their ability to influence (*e*.*g*., treat, detect, analyze, ameliorate, reverse, and/or prevent changes in) *de novo* lipid biosynthesis.

These measurements can serve as assessments of individuals or populations and as assessments of the results of an intervention by pharmacological, nutritional, toxicological, environmental, or genomic means. Further, these markers and profiles can be used to mine, parse, sort, filter, or otherwise investigate a database of lipid metabolites.

### Introduction

Fatty acids found in the tissue and body fluids of animals are present there because of diet or the biosynthesis of fatty acids *de novo* from acetyl-CoA. The accumulation of fatty acids within lipids is thus a competitive process, where *de novo* synthesized fatty acids compete for acylation into the many of the same lipid pools as diet-derived fatty acids. The distinction between diet- and *de novo*-synthesized fatty acids is not always clear, for instance oleic acid (18:1n9) is both a major unsaturated fatty acid in the diet and a major unsaturated fatty acid produced *de novo.* However, there are distinctions among fatty acid that allow investigators to assess the relative role of endogenous lipid synthesis in the total lipid composition of a human or a research animal. As an example, humans and other animals can not synthesize linoleic acid, and thus, when it is present in tissues or body fluids it is invariably there because linoleic acid is present in the diet. Conversely, the fatty acid palmitoleic acid (16:1n7) is not common in the diet, and is a primary product of *de novo* fatty acid synthesis, and thus, the presence of palmitoleic acid in tissues or body fluids is the result of *de novo* fatty acid synthesis.

In animals, *de novo* fatty acid synthesis occurs predominantly in the liver, skeletal muscle and in the abdominal adipose tissue (Semenkovich et al., Prog. Lipid Res. 36(1):43-53, 1997). The combined action of a group of enzymes known as fatty acid synthase produce saturated fatty acids by subsequent reaction cycles, with each cycle adding an acetate group to the growing acyl chain. Thioesterases specific for fatty acid chain length remove the fatty acids from this cyclic synthesis. The dominant product of *de novo* fatty acid synthesis is palmitic acid (16:0). Other fatty acids including stearic acid (18:0) and myristic acid (14:0) are also produced by fatty acid synthase but at much lower concentrations. Each of these fatty acids can be desaturated by the stearoyl-CoA desaturase to a Δ9-unsaturated fatty acid. Examples of these fatty acids include palmitoleic acid (16:1n7) and oleic acid (18:1n9).

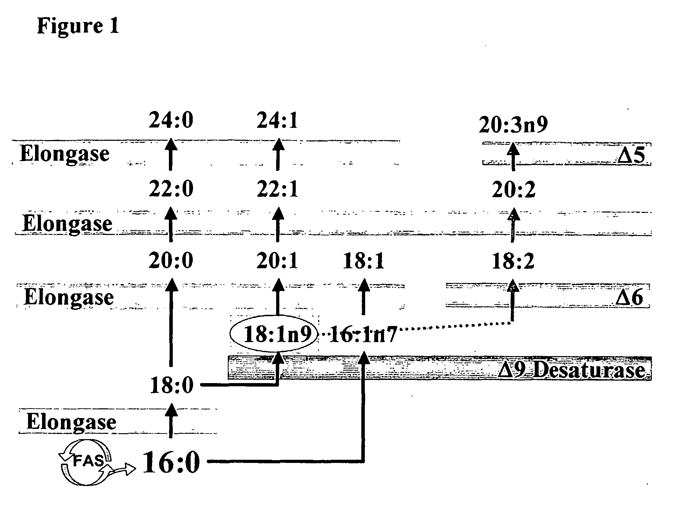

All of the fatty acids produced *de novo* by animals are candidates for markers of *de novo* fatty acid synthesis; however, the inventor has discovered that a few fatty acids are particularly well suited as biomarkers or components of metabolomic profiles of *de novo* fatty acid synthesis. Figure 1 shows the metabolism of *de novo* synthesized fatty acids within animals. The major fatty acids produced by *de novo* synthesis are palmitic acid (16:0), stearic acid (18:0), myristic acid (14:0), palmitoleic acid (16:1n7), oleic acid (18:1n9) and vaccenic acid (18:1n7). all of the major fatty acids produced *de novo* are saturated or monounsaturated fatty acids. The only polyunsaturated fatty acid of significance produced *de novo* in animals is mead acid (eicosatrienoic acid; 20:3n9). All of the above mentioned fatty acids have some value in assessing the activity and regulation of *de novo* fatty acid synthesis, however, in particular circumstances each of these components also has significant drawbacks.

The saturated fatty acid 16:0 is the primary product of fatty acid synthase and would seem at first glance to be the ideal marker of *de novo* fatty acid synthesis. However, there are two drawbacks to using the measurement of 16:0 to assess *de novo* fatty acid synthesis. The first is that 16:0 is a major component of the diet, and thus, if the measurement of 16:0 from biological tissues is to be used to assess *de novo* fatty acid synthesis, the diet of the experimental subject must be controlled or known. This is also the case for 18:1n9 and 18:0, which are major dietary fatty acids. The second drawback is more complicated and it is applicable to each of the saturated fatty acids including 14:0, 16:0, and 18:0. Although saturated fatty acids are the direct products of fatty acid synthase, they are not substantially enriched relative to other fatty acids in biological tissues as a result of *de novo* fatty acid synthesis. This is because the lipid classes that fatty acids are esterified into are tightly regulated with respect to their saturated fatty acid content. As an example, the sn-1 position (the position in which a fatty acid is inserted by glycerol phosphate acyltransferase, a major enzyme involved in glycerolipid biosynthesis) in phospholipids contains more than 80% of its fatty acids as saturated fatty acids, while in most phospholipids, there are virtually no saturated fatty acids in the sn-2 position. The sn-2 position is composed of both mono- and polyunsaturated fatty acids. Thus, since the pool for saturated fatty acids in phospholipids is already predominantly comprised of saturated fatty acids, there can be no substantial enrichment of phospholipids with saturated fatty acids. This example extends to most other lipid classes as well. For the saturated fatty acid products of *de novo* fatty acid synthesis to substantially alter the lipid classes of tissues or bodily fluids, they must first be desaturated to their monounsaturated fatty acid derivatives.

The primary desaturase in *de novo* fatty acid synthesis is the Δ9 desaturase or stearoyl CoA-desaturase. This desaturase is common to both plants and animals, and is unfailingly the first desaturase to act on a saturated fatty acid. The Δ9 desaturation of palmitic acid produces the n7 family of fatty acids, 16:1n7 and 18:1 n7. These fatty acids are both rare in the diet and highly indicative of *de novo* fatty acid synthesis.

Monounsaturated fatty acids, and particularly those belonging to the n7 family of fatty acids, are typically a more appropriate subset of the metabolome to investigate as reflective of *de novo* fatty acid synthesis than are saturated fatty acids. Oleic acid (18:1 n9) may be used in a similar fashion to 16:1n7, but only when the diet is carefully controlled, because oleic acid is the most common unsaturated fatty acid consumed in the diet. In fact, in a highly controlled experimental system, the concentration of 18: 1n9 or its elongation products may be equal or superior measurements than 16:1n7 or 18:1n7 for assessing *de novo* fatty acid synthesis.

Although palmitoleic acid is produced *de novo* by both plants and animals by the Δ9 desaturation of palmitic acid, it is a negligible component of most animal and vegetable lipids. Fish oil and pork fat are two noteworthy sources of palmitoleic acid; however, both fats generally contain less than 3% of their fatty acids as palmitoleic acid.

The primary purpose of *de novo* fatty acid synthesis is to convert soluble energy in the form of carbohydrates and acetyl-CoA to insoluble energy in the form of fats for long-term storage. *De novo* fatty acid synthesis is thus one response to an energy surplus, and is typically accompanied by weight gain, particularly adipose tissue accumulation. It has recently been reported that the injection of a pharmacological inhibitor of fatty acid synthase caused significant weight loss in mice (Loftus et al., Science, 288:2379-2381, 2000). Ceasing the injections of fatty acid synthase inhibitor was followed by a rapid gain of weight in the same mice. These data indicate that metabolic markers of fatty acid synthesis should be excellent markers of the propensity to gain weight.

Several nutritional interventions are known to modulate *de novo* lipogenesis. Dietary polyunsaturated fatty acids have been shown to decrease the expression of hepatic fatty acid synthase and Δ9 desaturase mRNA (Jump et al. J. Lipid Research 35:1076-1084, 1994). Alternatively, dietary carbohydrates and plasma glucose increase *de novo* lipogenesis in both liver and adipose. Fasting has tissue-specific effects on *de novo* lipogenesis, with lipogenesis depressed in adipose and increased in liver (Kersten et al. 103, 1489-1498, 1999). This is likely because adipose tissue must mobilize fatty acids from triacylglycerides to meet the increased energy demands of peripheral tissues in the fasted state, while the liver must deal with the increased circulating free fatty acids by repackaging them into triacylglycerides for transport to tissues. This repackaging of previously formed free fatty acids is important to distinguish from the *de novo* synthesis of fatty acids. Although the term lipogenesis encompasses both the synthesis of fatty acids and the assembly of those fatty acids into triacylglycerides, only the synthesis of fatty acids itself is truly representative of physiological states in which there is an energy surplus or a propensity for weight gain. For the purposes of this application, *de novo* fatty acid synthesis refers to only the synthesis of fatty acids, and not the creation of triacylglycerides.

Included herein is an example (rosiglitazone-treated mice) in which plasma total triacylglycerides decreased, yet *de novo* fatty acid synthesis in both the liver and in adipose were increased. The rosiglitazone-treated mice from this study gained weight relative to their controls, yet had diminished lipid concentrations in their plasma. Despite the decreased plasma lipids, a quantitative metabolomic assessment of the plasma and other tissues revealed that these mice showed clear signs of *de novo* fatty acid synthesis, as all tissues and plasma contained increased concentrations of palmitoleic acid. This example clearly demonstrates that triacylglyceride concentrations are not sufficient to assess whether an intervention will increase fatty acid synthesis or cause weight gain, and that only an assessment of palmitoleic acid or other markers of *de novo* fatty acid synthesis described herein can predict these metabolic and phenotypic outcomes.

Hormones also modulate *de novo* lipogenesis. Insulin increases hepatic *de novo* lipogenesis, while glucagon and growth hormone decrease lipogenesis (Kersten, EMBO Reports, 2(4):282-286, 2001). Growth hormone has been shown to depress insulin sensitivity in adipose tissue, resulting in decreased fatty acid synthase expression in adipose (Yin et al., Biochem. J. 331, 815-820, 1998). The phenotypic effects of this are decreased adipose and increased lean muscle mass. Leptin is best known for is repression of food intake, however, it has also been shown to decrease the expression of fatty acid synthase and genes involved in triacylglycerides synthesis (Soukas et al., Genes Dev. 14, 963-980, 2000). The effect of leptin and other hormones may be mediated by the sterol regulatory element binding protein (SREBP-1). The hormone acylation stimulating protein (ASP) acts not at the level of fatty acid synthesis, but rather by up-regulating the synthesis of triacylglycerides. Thus, ASP action may be discriminated from the action of those hormones that induce both fatty acid synthesis and triacylglycerides synthesis by the absence of an increase in *de novo* synthesized fatty acids within the newly formed triacylglycerides.

Sterol regulatory element-binding proteins (SREBPs) are membrane-bound transcription factors that control the expression of genes involved in lipid biosynthesis including fatty acid synthase and sterol CoA desaturase (SCD) (Horton et al., J. Clin. Invest. 109:1125-1131, 2002). There are two genes encoding three SREBP proteins SREBP-1a, SREBP-1c and SREBP-2. SREBP-1c c preferentially activates fatty acid synthase and SCD, thereby activating fatty acid biosynthesis without activating sterol biosynthesis. SREBP-2 preferentially activates the biosynthesis of cholesterol without activating fatty acid biosynthesis. Thus, agents that target the expression, processing or actions of SREBP proteins can influence *de novo* fatty acid biosynthesis and weight gain. The markers described herein serve as effective diagnostic and prognostic markers for the actions of interventions that may influence SREBP-mediated fatty acid biosynthesis and weight gain. Likewise, activities and influences of hormones that regulate SREBP actions, such as insulin, estrogen, growth factor, for instance, can be assessed by the methods described herein.

### Quantitative Metabolomics

A quantitative assessment of fatty acids allows for the investigation of fatty acid concentration in both absolute and relative terms. Only quantitative data will allow for the investigation of the molar relationships among all fatty acids, regardless of which lipid class the fatty acid is acylated into. Additionally, quantitative data enables the creation of an expandable database of lipid metabolites that can be investigated *in silico.* Quantitative data is also easily converted into relative data (mole percentage, weight percentage, etc.) for investigating fatty acid metabolism within an individual lipid class. By converting quantitative data to mole percentage data, for instance, an investigator can identify the relative abundance of the fatty acid within the class. This approach can make identifying changes in lipid class metabolism easier than investigating the data in quantitative terms. Thus, the assessment of *de novo* fatty acid synthesis by measuring fatty acid composition can be approached both from quantitative and relational data formats, although it is suggested that the data is collected in quantitative terms.

It is the fatty acids themselves (16:1n7, etc) that indicate the modulation of *de novo* fatty acid synthesis, not the concentrations of the lipid classes. However, the presence of these fatty acids in lipid classes can indicate the location of the modulated *de novo* fatty acid synthesis. In blood, for instance, free fatty acids are derived almost exclusively from adipose, while in blood from fasted subjects, triacylglycerides, phospholipids and most cholesterol esters are derived from the liver. Thus, as an example, the presence of a marker of *de novo* fatty acid synthesis in the free fatty acid fraction of plasma indicates increased synthesis in adipose tissue.

An increased concentration (or concentration relative to other major fatty acids) of palmitoleic acid (16:1n7) in tissue, blood, serum or plasma cholesterol esters, free fatty acids, triacylglycerides, phosphatidylcholine, phosphatidylethanolamine, phosphatidylserine or phosphatidylinositol indicates increased *de novo* fatty acid synthesis. A decreased concentration (or concentration relative to other major fatty acids) of palmitoleic acid (16:1n7) in tissue, blood, serum or plasma cholesterol esters, free fatty acids, triacylglycerides, phosphatidylcholine, phosphatidylethanolamine, phosphatidylserine, or phosphatidylinositol indicates decreased *de novo* fatty acid synthesis.

*De novo* fatty acid synthesis can be detected by the methods described herein, even when total plasma triacylglycerides or other tissue or plasma lipid classes are decreased in concentration. It is the individual *de novo* synthesized lipids that are important, not the total class concentration.

### Ratios of Markers

Using methods described herein, it has been found that specific ratios between lipid metabolites, or between classes of metabolites, can be closely correlated with specific biological conditions or propensities. Representative specific ratios that have been identified include the following:
1) The ratio of palmitoleic acid to palmitic acid (16:1 n7/16:0) increases in lipid classes with increases in fatty acid synthesis, and therefore marks lipogenesis and weight gain.
2) The ratio of stearic acid to palmitic acid (18:0/16:0) decreases in lipid classes with increases in fatty acid synthesis, and therefore marks lipogenesis and weight gain in some systems.
3) The ratio of total n7 fatty acids to total saturated fatty acids increases in lipid classes with increases in fatty acid synthesis, and therefore marks lipogenesis and weight gain.
4) The ratio of total n7 fatty acids to total n9 fatty acids increases in lipid classes with increases in fatty acid synthesis, and therefore marks lipogenesis and weight gain.
5) The ratio of 18: 1n9 to 18:0 is positively correlated with weight gain.

Marker ratios can be calculated for different lipid categories, such as liver triacylglycerides, cholesterol esters, free fatty acids, plasma triacylglycerides, cholesterol esters and free fatty acids, and adipose triacylglycerides, to determine the propensity for weight gain.

### Markers of de novo fatty acid synthesis as markers for phenotype

As mentioned above, the primary purpose of *de novo* fatty acid synthesis is to convert soluble energy in the form of carbohydrates and acetyl-CoA to insoluble energy in the form of fats for long-term storage. Thus, increased *de novo* fatty acid synthesis itself is a hallmark of energy surplus, weight gain and adipose accumulation. Weight gain or lipid synthesis and accumulation are important phenotypes in a number of clinically relevant situations. A non-limiting list of examples includes individual responses to a pharmaceutical intervention, such as birth control pills or insulin sensitizing drugs, wherein weight gain is considered a negative phenotype, and the treatment of acquired immune deficiency syndrome (AIDS) patients, chemotherapy patients, and the elderly, in which weight gain may be considered a positive phenotype.

### VI. Methods of Screening for a Compound

This disclosure further relates in some embodiments to novel methods for screening compounds (such as test compounds) for their ability to treat, detect, analyze, ameliorate, reverse, and/or prevent changes in *de novo* fatty acid synthesis-linked diseases, disorders or conditions, such as diabetes, weight gain, weight loss (*e*.*g*., wasting), obesity, hypo- and hyper-thyroidism, menopause, immuno-tolerance, auto-immunity, aging, and/or cardiovascular disease. In particular, the present disclosure provides methods for identifying compounds that can be used to treat, detect, analyze, ameliorate, reverse, and/or prevent changes in lipid-linked diseases, disorders or conditions, such as diabetes, weight gain, weight loss (*e.g.*, wasting), obesity, hypo- and hyper-thyroidism, menopause, immuno-tolerance, auto-immunity, aging, and/or cardiovascular disease. The compounds of interest can be tested by exposing a cell, system, or subject to the test compounds (alone or in combination), then examining the effect(s) that the compounds have on one or more fatty acid or lipid metabolites, or metabolite fingerprint. If a compound affects one or more fatty acid or lipid metabolites, for instance by increasing or decreasing that metabolite, or by increasing or decreasing a set of metabolites (such as a set that is indicative of a pathway fingerprint or other linked profile), the compound is then further evaluated for its ability to influence one or more lipid-related or lipid-influenced conditions. Specifically, provided herein are lipid metabolites that are linked to de novo fatty acid synthesis, which include palmitoleic acid, vaccenic acid, palmitic acid, stearic acid, oleic acid, all n7 fatty acids, all n9 fatty acids, or a combination of two or more thereof.

Similarly, the systems described herein for screening for a compound can be used to screen for a non-compound influence, such as a dietary change, lifestyle change, nutritional treatment or intervention.

One provided aspect involves a screening method to identify a compound (or other influence) effective for treating, detecting, preventing, reversing, analyzing, or ameliorating diabetes, weight gain, weight loss (*e*.*g*., wasting), obesity, hypo- and hyper-thyroidism, menopause, immuno-tolerance, auto-immunity, aging, and/or cardiovascular disease, which method includes ascertaining the compound's effects on one or more lipid metabolites (for instance, on the quantity of such metabolites, such as palmitoleic acid, vaccenic acid, palmitic acid, stearic acid, oleic acid, all n7 fatty acids, all n9 fatty acids, all unsaturated fatty acids, or a combination of two or more thereof), or a ratio of lipid metabolites (for instance, palmitoleic acid to palmitic acid, stearic acid to palmitic acid, total n7 fatty acids to total saturated fatty acids, total n7 fatty acids to total n9 fatty acids, or oleic acid to stearic acid, or myristoleic to myristate (14: 1n5 to 14:0), measured either as free fatty acids or components of other lipids, in a system (such as a cell, organ, organism, or subject) contacted with the compound. In some embodiments, the screening method further includes determining whether the compound exhibits toxicity toward a cell in cell culture.

By screening compounds in this fashion, potentially beneficial and improved compounds or other influences for treating, detecting, analyzing, ameliorating, reversing, and/or preventing changes in lipid-related or lipid-influenced diseases, disorders, or conditions can be identified more rapidly and with greater precision than possible in the past.

### VII. Use of Identified Compounds to Treat, Detect, Analyze, Ameliorate, Reverse, and/or Prevent Changes in Lipid-Linked Disease, Disorder or Condition

With the provision herein of methods for identifying compounds that influence the one or more lipid metabolites in a system, and by such alterations of lipid metabolites influence or signal a change in a lipid-linked disease, disorder or condition, the benefits of using the identified compound to cure, detect, analyze, ameliorate, prevent, or treat diseases, disorders, and conditions that involve (directly or indirectly) lipid metabolism, and more particularly de novo fatty acid synthesis, are now made clear. Such diseases, disorders, and conditions include, but are not limited to, diabetes, weight gain, weight loss (*e*.*g*., wasting), obesity, hypo- and hyper-thyroidism, menopause, immuno-tolerance, auto-immunity, aging, and cardiovascular disease.

The invention is illustrated by the following non-limiting Examples of certain specific embodiments.

### EXAMPLES

### Example 1: Palmitoleic Acid as a Marker for Endogenous Fatty Acid Biosynthesis, and Accompanying Phenotypes

This example provides specific methods that have been used to examine tissues taken from mice treated with either rosiglitazone (trial 1), a thiazolidinedione, or CL316,243 (trial 2), a β-3 adrenergic agonist It is understood that the described methods can be used to analyze lipid metabolites from other subjects, including particularly animals other than mice.

### Methods:

### Samples

Mouse tissue and plasma samples were a generous donation to Lipomics Technologies, Inc., from Dr. Edward Leiter of the Jackson Laboratory (Bar Harbor, ME). Samples included the plasma, heart, liver and inguinal adipose of mice treated with pharmaceuticals or their corresponding controls.

In trial 1, prediabetic male F1 mice (from a cross of the obese NZO and lean NON mouse strains) were fed a control diet with or without the presence of the PPARs-y agonist rosiglitazone for 4 weeks (at 0.2 g rosiglitazone per kg body weight).

In trial 2, male, inbred NON mice were fed a control with or without the presence of the β-3 adrenergic agonist Cm316,243 for four weeks (at 0.001% CL316,243 by weight in the dietary chow).

In both studies, five treated and five control mice were used. Following the treatments and the killing of the mice, tissues and plasma were taken, chilled to-80 °C and shipped to the analysis laboratory at Lipomics Technologies in a frozen state.

### Tissue Processing and Extraction

Tissue processing and lipid extraction, including provision of internal standards, was carried out essentially as described in Watkins et al. (J. Lipid Res. Papers in Press, 10.1184/jlf.M200169-JLF200, published August 16,2002), and in co-owned international application PCT/US0221426, entitled "GENERATING, VIEWING, INTERPRETING, AND UTILIZING A QUANTITATIVE DATABASE OF METABOLITES".

### Separation of Lipid and Phospholipid Classes

The following description provides representative methods for separation of lipid and phospholipid classes. One of ordinary skill in the art will understand that modifications can be made to these methods.

The separation of lipid classes was performed in some instance by preparative thin-layer chromatography (TLC). To remove any residual metal or other damaging contaminants on the TLC plates, each plate was washed prior to use. Washing the plates is a three-step process that involves impregnating each plate with ethylenediamine tetraacetic acid (EDTA) and rinsing the plates once with methanol and once with chloroform. Each plate is first impregnated with Mum EDTA, pH 5.5, by ascending development using the method of Ruiz (J. Lipid Res. 38, 1482-1489, 1997). After each plate was completely developed, it was dried in air overnight. Once dry, each plate was developed in methanol, dried, and developed in chloroform in the same direction as the development with EDTA. The washed plates were then dried in air. Just prior to use, each plate was activated by heating to 110 °C for 10 minutes.

To prepare the TLC chamber for chromatography, Whatman (Clifton, NJ) filter paper was cut into 20 × 80-cm strips and wrapped around the inside wall of a 30 × 60 × 10-cm glass development chamber. One hundred milliliters of the appropriate mobile phase was added to the chamber, and the chambers were sealed and allowed to equilibrate. Chambers were considered equilibrated when the solvent front had completely ascended the filter paper. The mobile phase employed for the separation of phospholipid classes (lyso-phospholipids, sphingomyelin, phosphatidylcholine, phosphatidylserine, phosphatidylethanolamine and cardiolipin) was a modification of the solvent system described by Holub and Skeaf ("Nutritional regulation of cellular phosphatidylinositol," in Methods in Enzymology, ed. Conn, P. M. (Academic Press, Inc., Orlando), pp. 234-243, 1987) consisting of chloroform/methanol/acetic acid/water (100:67:7:4, by volume).

For the separation of neutral lipid classes (free fatty acids, free sterols, triacylglycerides and cholesterol esters), a solvent system consisting of petroleum ether/diethyl ether/acetic acid (80:20:1, by volume) was used (Mangold, Thin Layer Chromatography- A Laboratory Handbook (Springer-Verlag, New York), 1969).

After the TLC plate was activated, the sample extracts were spotted onto the activated plate. As a general rule, samples were spotted at an estimated concentration such that no single lipid class was present at more than 25 µg per centimeter of plate width following chromatography. This ensured that the plate was not overloaded and minimized the risk of cross-contamination between lipid classes (cross-contamination is readily identified during sample analysis as each lipid class contains unique internal standards).

Lipid class separations were performed on TLC plates with a 10-cm separation length, while PL class separations were performed on TLC plates with a 20-cm separation length. Because lipid visualization reagents invariably degrade certain analytes, most notably the polyunsaturated fatty acids, the identification of individual lipid classes was performed by comparison with authentic lipid standards chromatographed in reference lanes. Each reference lane was spotted with a mixture of authentic lipid standards (obtained from Avanti Polar Lipids, Alabaster, AL), and when the amount of sample is not limiting, the sample extract was also spotted onto the reference lanes. Once the TLC plates were spotted and the tanks were equilibrated, the plates were transferred into the tank containing the appropriate mobile phase, and the sample was chromatographed until the mobile phase ascended to 1-cm below the top of the plate.

Once the TLC plate was developed, the reference lipids were visualized by cutting the reference lanes from the plate, dipping the reference lanes in 10% cupric sulfate/8% phosphoric acid and charring the reference lanes at 300 °C. The charred reference lanes were used to identify the location of lipid classes on the analytical plate. Each sample was scraped from the plate using a clean razor blade and the silica scrapings were placed in a 2-mL glass vial for derivitization. Great care was taken to develop this process so that it meets the following criteria:
(1) reference standards co-migrate with sample analytes with great accuracy;
(2) chromatographic separation between the lipid classes is maximized to avoid any cross-contamination problems; and
(3) the portion of the plate containing analytes is not exposed to environmental stresses such as air, light or any reagent that would cause the degradation of specific analytes.

The silica scrapings containing the free sterol fraction were exposed to a fluid extractant consisting of one milliliter of cbIoroform:methanol (2:1 vol:vol). The mixture was mixed vigorously and allowed to sit for 15 minutes, then 0.3 mL of 0.01 M potassium chloride was added, and the solution once again mixed vigorously. The organic fraction containing free sterols was separated from the polar fraction of the mixture by centrifugation. The extract including free sterols was removed from the mixture and completely dried down under a stream of nitrogen. A 20-µL aliquot of chloroform was used to transfer the reconstituted free sterols to a conical insert in preparation for free sterol separation via capillary gas chromatography. No derivitization was necessary to prepare the free sterols for gas chromatographic analysis.

### Derivatization and Chromatography

Derivatization and chromatographic analysis were carried out essentially as described in Watkins et al. (J. Lipid Res. Papers in Press. 10.1184/jlf.M200169-JLF200, published August 16, 2002), and in co-owned international application PCT/US02/21426, entitled "GENERATING, VIEWING, INTERPRETING, AND UTILIZING A QUANTITATIVE DATABASE OF METABOLITES".

### Integration and Data Handling

Following chromatography, each chromatogram was integrated, for instance using Hewlett-Packard (Wilmington, DE) ChemStation™ software. At the beginning of each batch of samples, a standard mixture was run, containing a known concentration of each of the fatty acids listed in Table 4. Each fatty acid in its methyl ester form is present in this standard mixture. The quantitative standard was used to set a calibration table that automatically corrected the areas associated with each fatty acid methyl ester from the samples for injection discrimination and injector non-linearity.

After chromatogram integration, the chromatogram from each sample was visually checked to ensure proper integration, and the data was sent electronically to an Excel 2000 (Microsoft Corporation, Redmond, WA) spreadsheet. This spreadsheet contains sample identification information, quality control algorithms and algorithms required to convert the raw chromatogram data to mass or concentration data.

**Table 4:**

| **SCIENTIFIC NAME** | **SCIENTIFIC ABBR.** | **COMMON NAME** |
|---|---|---|
| **- S A T U R A T E D -** | | |
| Tetradecanoic Acid | 14:0 | Myristic Acid |
| Pentadecanoic Acid | 15:0 | - |
| Hexadecanoic Acid | 16:0 | Palmitic Acid |
| Heptadecanoic Acid | 17:0 | Margaric Acid |
| Octadecanoic Acid | 18:0 | Stearic Acid |
| Eicosanoic Acid | 20:0 | Arachidic Acid |
| Docosanoic Acid | 22:0 | Behenic Acid |
| Tetracosanoic Acid | 24:0 | Lignoceric Acid |

| **- D 9 D E S A T U RA S E F A M I L Y -** | | |
|---|---|---|
| 9-Tetradecenoic Acid | 14:1n5 | Myristoleic Acid |
| 9-Hexadecenoic Acid | 16:1n7 | Palmitoleic Acid |
| 11-Octadecenoic Acid | 18:1n7 | Vaccenic Acid |
| 9-Octadecenoic Acid | 18: 1n9 | Oleic Acid |
| 11-Eicosenoic Acid | 20:1n9 | Eicosenoic Acid |
| 5,8,11-Eicosatrienoic Acid | 20:3n9 | Mead Acid |
| 13-Docosenoic Acid | 22: 1n9 | Erucic Acid |
| 15-Tetracosenoic Acid | 24: 1n9 | Nervonic Acid |

| **- O M E G A 3 F A M I L Y -** | | |
|---|---|---|
| 9,12,15-Octadecatrienoic Acid | 18:3n3 | a-Linolenic Acid |
| 6,9,12,15-Octadecatetraenoic Acid | 18:4n3 | - |
| 11,14,17-Eicosatrienoic Acid | 20:3n3 | Eicosatrienoic Acid (ETA) |
| 8,11,14,17-Eicosictetraenoic Acid | 20:4n3 | - |
| 5,8,11,14,17-Eicosapentaenoic Acid | 20:5n3 | Eicosapentaenoic Acid (EPA) |
| 7,10,13,16,19-Docosapentaenoic | Acid 22:5n3 | Docosapentaenoic Acid (DPA) |
| 4,7,10,13,16,19-Docosahexaenoic | Acid 22:6n3 | Docosahexaenoic Acid (DHA) |
| 6,9,12,15,18,21 -Tetracoshexaenoic | Acid 24:6n3 | Tetracosahexaenoic Acid |

| **- O M E G A 3 F A M I L Y -** | | |
|---|---|---|
| 9,12,15-Octadecatrienoic Acid | 18:3n3 | a-Linolenic Acid |
| 6,9,12,15-Octadecatetraenoic Acid | 18:4n3 | - |
| 11,14,17-Eicosatrienoic Acid | 20:3n3 | Eicosatrienoic Acid (ETA) |
| 8,11,14,17-Eicosictetraenoic Acid | 20:4n3 | - |
| 5,8, 11,14,17-Eicosapentaenoic Acid | 20:5n3 | Eicosapentaenoic Acid (EPA) |
| 7,10,13,16,19-Docosapentaenoic | Acid 22:5n3 | Docosapentaenoic Acid (DPA) |
| 4,7,10,13,16,19-Docosahexaenoic | Acid 22:6n3 | Docosahexaenoic Acid (DHA) |
| 6,9,12,15,18,21-Tetracoshexaenoic | Acid 24:6n3 | Tetracosahexaenoic Acid |

| **- O M E G A 6 F A M I L Y -** | | |
|---|---|---|
| 9,12-Octadecadienoic Acid | 18:2n6 | Linoleic Acid |
| 6,9,12-Octadecat.de.noic Acid | 18:3n6 | g-Linolenic Acid |
| 11,14-Eicosadienoic Acid | 20:2n6 | Eicosadienoic Acid |
| 8,11,14-Eicosatrienoic Acid | 20:3n6 | Homo-g-Linolenic Acid |
| 5,8,11,14-Eicosicaletraenoic Acid | 20:4n6 | Arachidonic Acid |
| 13,16-Docsadienoic Acid | 22:2n6 | Docosadienoic Acid |
| 7,10,13,16-Docosicatetraenoic Acid | 22:4n6 | Docosicatetraenoic Acid |
| 4,7,10,13,16-Docosapentaenoic Acid | 22:5n6 | Docosapentaenoic Acid |

| **- U N U S U A L F A M E s -** | | |
|---|---|---|
| 9-Trans-Hexadecenoic Acid | t16:1n7 | Palmitelaidic Acid |
| 9-Trans-Octadecenoic Acid | t18:1n9 | Elaidic Acid |
| 8-Eicosaenoic Acid | 20:1n12 | - |
| 5-Eicosaenoic Acid | 20:1n15 | - |
| Plasmologen fatty acids | 16:0 | - |
| " | 18:0 | - |
| " | 18:1n7 | - |
| " | 18:1n9 | - |

| **- S T E R O L S -** | | |
|---|---|---|
| 5b-cholestan-3b-ol | C₂₇H₄₈O | coprostanol |
| 5a-cholestan-3b-ol | C₂₇H₄₈O | dihydrocholesterol |
| 5-cholesten-3b-ol | C₂₇H₄₆b | cholesterol |
| 5,24-cholestadien-3b-ol | C₂₇H₄₄O | desmosterol |
| 5-cholestan-25a-methyl-3b-ol | C₂₈H₄₂O | campesterol |
| 5-cholestan-24b-methyl-3b-ol | C₂₈H₄₂O | dihydrobrassicasterol |
| 5-cholesten-24b-ethyl-3b-ol | C₂₉H₅₀O | b-sitosterol |
| 5,22-cholestadien-24b-ethyl-3b-ol | C₂₉H₄₈O | stigmasterol |

Methods of integration, data processing, and data analysis used were essentially as described previously, for instance in co-owned international application PCT/US02/21426, entitled "GENERATING, VIEWING, INTERPRETING, AND UTILIZING A QUANTITATIVE DATABASE OF METABOLITES".

Mean, standard deviation and Student's t-test statistics were calculated for each test group. These calculations were used to compare the effects of treatment on lipid metabolite concentrations.

### Visualization

The results of the experiment were displayed in both table form (see, *e*.*g*., Tables 5-8) and with computer-based visual output systems. Representative example computer based visual output systems are described in co-owned provisional patent application 60/303,704, entitled "PROVISION OF A COMPREHENSIVE PANEL OF LIPID METABOLITES".

| Table 5: Rosiglitazone Treatment (Quantitative) | | | | | | | | | | | | | |
|---|---|---|---|---|---|---|---|---|---|---|---|---|---|
| Treatment | Tissue | | Lipid Class | 14:0 | 16:0 | 16:1n7 | 18:0 | 18:1n9 | 18:1n7 | Total nMole of FA | Saturates | Total n7 | Total n9 |
| **Control** | **Liver** | **Mean** | **PC** | **29.0** | **7035.2** | **186.2** | **5261.2** | **2385.6** | **846.6** | **13968.8** | **12419.3** | **1032.7** | **2538.0** |
| Control | Liver | SD | PC | 3.9 | 957.7 | 33.9 | 525.0 | 225.6 | 131.8 | 1645.7 | 1465.0 | 146.7 | 232.0 |
| **Treatment** | **Liver** | **Mean** | **PC** | **43.2** | **9572.4** | **501.3** | **2975.6** | **2767.4** | **1329.1** | **14390.8** | **12686.5** | **1830.5** | **2932.3** |
| Treatment | Liver | SD | PC | 4.1 | 577.0 | 58.4 | 164.4 | 311.2 | 156.3 | 740.6 | 635.6 | 210.4 | 328.1 |
| **Control** | **Liver** | **Mean** | **PS/I** | **20.1** | **554.9** | **42.2** | **3555.6** | **707.9** | **159.7** | **4831.4** | **4182.5** | **201.8** | **851.0** |
| Control | Liver | SD | PS/I | 4.6 | 107.8 | 6.7 | 605.2 | 172.6 | 17.6 | 736.2 | 704.1 | 18.5 | 181.6 |
| **Treatment** | **Liver** | **Mean** | **PS/I** | **18.9** | **1158.5** | **76.0** | **3348.9** | **693.2** | **303.2** | **5196.5** | **4590.1** | **379.2** | **854.3** |
| Treatment | Liver | SD | PS/I | 4.7 | 165.7 | 16.1 | 312.4 | 85.1 | 86.9 | 559.3 | 454.9 | 95.6 | 130.1 |
| **Control** | **Liver** | **Mean** | **PE** | **12.4** | **2412.1** | **67.6** | **2799.3** | **1094.4** | **270.5** | **6609.8** | **5262.8** | **338.1** | **1175.0** |
| Control | Liver | SD | PE | 5.2 | 433.5 | 22.7 | 422.2 | 280.0 | 57.3 | 1102.8 | 863.0 | 79.5 | 294.9 |
| **Treatment** | **Liver** | **Mean** | **PE** | **12.5** | **3461.1** | **191.0** | **2245.7** | **1119.7** | **574.2** | **7418.6** | **5762.3** | **765.1** | **1211.6** |
| Treatment | Liver | SD | PE | 4.9 | 389.5 | 34.9 | 163.1 | 215.3 | 88.1 | 806.9 | 534.8 | 120.6 | 226.6 |
| **Control** | **Liver** | **Mean** | **FFA** | **238.4** | **4254.7** | **1083.0** | **1027.3** | **6853.4** | **1009.7** | **23273.8** | **5639.3** | **2092.7** | **7007.1** |
| Control | Liver | SD | FFA | 37.0 | 538.3 | 110.4 | 173.8 | 1568.7 | 203.6 | 3155.9 | 754.5 | 311.9 | 1597.9 |
| **Treatment** | **Liver** | **Mean** | **FFA** | **236.5** | **4507.0** | **2121.2** | **691.5** | **5761.2** | **1466.0** | **22631.3** | **5541.5** | **3587.3** | **5909.9** |
| Treatment | Liver | SD | FFA | 34.9 | 587.9 | 331.1 | 132.4 | 1084.1 | 264.7 | 2523.8 | 741.0 | 564.6 | 1114.4 |
| **Control** | **Liver** | **Mean** | **TAG** | **1372.5** | **68556.9** | **9079.2** | **3009.0** | **107575.1** | **10159.7** | **81381.1** | **74293.0** | **19238.8** | **109608.7** |
| Control | Liver | SD | TAG | 292.2 | 14882.5 | 1711.6 | 840.3 | 24835.9 | 1854.0 | 19657.1 | 16342.5 | 3493.1 | 25303.7 |
| **Treatment** | **Liver** | **Mean** | **TAG** | **2450.8** | **122013.9** | **36411.0** | **2665.4** | **179035.8** | **26279.6** | **150412.8** | **129102.3** | **62690.6** | **183105.4** |
| Treatment | Liver | SD | TAG | 196.8 | 9975.2 | 4048.8 | 367.2 | 22720.9 | 2601.4 | 12221.3 | 10501.7 | 5405.3 | 23350.8 |
| **Control** | **Liver** | **Mean** | **CE** | **87.8** | **3053.5** | **368.4** | **273.1** | **1746.1** | **126.8** | **6697.0** | **3481.7** | **495.3** | **1776.9** |
| Control | Liver | SD | CE | 8.9 | 224.9 | 39.1 | 32.8 | 257.3 | 13.1 | 384.6 | 235.3 | 48.9 | 256.0 |
| **Treatment** | **Liver** | **Mean** | **CE** | **110.6** | **2765.2** | **678.5** | **204.4** | **1208.0** | **138.6** | **5830.7** | **3147.2** | **817.0** | **1239.5** |
| Treatment | Liver | SD | CE | 21.9 | 389.6 | 131.3 | 47.2 | 526.6 | 54.4 | 1221.5 | 459.6 | 169.4 | 533.0 |
| **Control** | **Plasma** | **Mean** | **PC** | **4.9** | **1689.0** | **23.5** | **1508.9** | **553.7** | **191.2** | **3624.2** | **3239.3** | **214.6** | **597.6** |
| Control | Plasma | SD | PC | 0.8 | 206.0 | 2.8 | 289.1 | 110.4 | 40.1 | 570.2 | 488.1 | 42.2 | 123.1 |
| **Treatment** | **Plasma** | **Mean** | **PC** | **5.7** | **1246.9** | **34.2** | **528.9** | **383.4** | **164.8** | **2027.9** | **1804.9** | **199.0** | **405.1** |
| Treatment | Plasma | SD | PC | 1.2 | 101.9 | 4.6 | 61.9 | 75.1 | 26.1 | 221.3 | 163.7 | 30.1 | 80.6 |
| **Control** | **Plasma** | **Mean** | **PE** | **3.8** | **43.6** | **3.3** | **45.4** | **44.0** | **5.1** | **141.5** | **96.7** | **8.3** | **45.9** |
| Control | Plasma | SD | PE | 2.1 | 8.2 | 2.7 | 9.1 | 38.7 | 2.7 | 42.1 | 18.8 | 5.4 | 37.6 |
| **Treatment** | **Plasma** | **Mean** | **PE** | **2.7** | **42.3** | **3.5** | **32.5** | **35.0** | **5.7** | **105.8** | **85.3** | **9.2** | **37.5** |
| Treatment | Plasma | SD | PE | 0.6 | 6.9 | 1.7 | 9.5 | 24.1 | 0.9 | 13.2 | 15.7 | 2.4 | 24.8 |
| **Control** | **Plasma** | **Mean** | **FFA** | **10.4** | **148.3** | **19.1** | **53.8** | **114.2** | **14.9** | **534.3** | **218.4** | **34.0** | **117.7** |
| Control | Plasma | SD | FFA | 6.7 | 43.6 | 6.3 | 19.7 | 34.7 | 3.7 | 145.9 | 70.4 | 9.8 | 35.0 |
| **Treatment** | **Plasma** | **Mean** | **FFA** | **15.9** | **148.4** | **41.4** | **37.3** | **90.2** | **15.2** | **478.4** | **208.8** | **56.7** | **92.7** |
| Treatment | Plasma | SD | FFA | 8.2 | 66.2 | 23.7 | 11.4 | 47.6 | 7.9 | 199.8 | 85.5 | 30.5 | 48.5 |
| **Control** | **Plasma** | **Mean** | **TAG** | **31.9** | **930.2** | **145.3** | **75.2** | **1549.0** | **146.0** | **1443.5** | **1059.3** | **291.4** | **1590.9** |
| Control | Plasma | SD | TAG | 5.2 | 171.6 | 24.6 | 14.7 | 320.3 | 26.6 | 243.7 | 193.7 | 49.4 | 330.5 |
| **Treatment** | **Plasma** | **Mean** | **TAG** | **17.1** | **315.8** | **102.5** | **24.9** | **398.8** | **66.1** | **484.3** | **368.7** | **168.5** | **410.2** |
| Treatment | Plasma | SD | TAG | 7.6 | 156.2 | 55.9 | 10.6 | 229.5 | 41.1 | 257.2 | 176.3 | 96.8 | 236.5 |
| **Control** | **Plasma** | **Mean** | **CE** | **12.7** | **140.4** | **114.1** | **10.2** | **305.9** | **24.9** | **4994.5** | **174.1** | **138.9** | **326.0** |
| Control | Plasma | SD | CE | 1.3 | 19.7 | 12.4 | 1.2 | 59.5 | 4.9 | 948.0 | 21.4 | 16.3 | 63.2 |
| **Treatment** | **Plasma** | **Mean** | **CE** | **16.8** | **113.0** | **200.8** | **7.1** | **189.1** | **22.7** | **3058.7** | **148.8** | **223.4** | **202.2** |
| Treatment | Plasma | SD | CE | 1.2 | 4.0 | 39.4 | 0.9 | 36.8 | 2.6 | 278.9 | 3.8 | 41.9 | 40.0 |
| **Control** | **Plasma** | **Mean** | **PL** | **5.9** | **2063.3** | **27.6** | **1893.1** | **649.5** | **222.3** | **4490.8** | **4042.1** | **249.8** | **751.3** |
| Control | Plasma | SD | PL | 0.6 | 278.9 | 4.3 | 341.6 | 137.3 | 49.6 | 712.6 | 619.1 | 51.9 | 159.0 |
| **Treatment** | **Plasma** | **Mean** | **PL** | **7.7** | **1533.6** | **43.0** | **604.7** | **384.8** | **195.0** | **2496.5** | **2191.4** | **238.0** | **449.9** |
| Treatment | Plasma | SD | PL | 0.3 | 162.0 | 6.3 | 306.4 | 200.2 | 33.0 | 376.0 | 389.9 | 39.2 | 202.2 |
| **Control** | **Heart** | **Mean** | **PC** | **40.4** | **7831.2** | **61.2** | **4898.9** | **1447.4** | **476.6** | **14180.4** | **12862.8** | **537.9** | **1506.2** |
| Control | Heart | SD | PC | 4.8 | 446.8 | 6.5 | 497.8 | 29.3 | 15.5 | 1085.3 | 921.4 | 17.6 | 28.1 |
| **Treatment** | **Heart** | **Mean** | **PC** | **68.3** | **8497.6** | **289.1** | **3392.4** | **3120.1** | **1154.7** | **14579.5** | **12059.6** | **1443.8** | **3184.2** |
| Treatment | Heart | SD | PC | 10.3 | 721.2 | 46.5 | 669.0 | 391.7 | 115.7 | 1694.0 | 1256.8 | 157.1 | 395.4 |
| **Control** | **Heart** | **Mean** | **PS/I** | **30.1** | **518.1** | **20.1** | **4177.6** | **494.0** | **101.2** | **6180.3** | **4808.4** | **121.3** | **566.8** |
| Control | Heart | SD | PS/I | 6.3 | 128.3 | 11.0 | 837.2 | 71.4 | 18.8 | 1284.7 | 948.0 | 24.4 | 71.4 |
| **Treatment** | **Heart** | **Mean** | **PS/I** | **25.3** | **515.0** | **36.4** | **3334.5** | **504.6** | **143.9** | **4967.1** | **3945.4** | **180.2** | **572.9** |
| Treatment | Heart | SD | PS/I | 8.0 | 124.1 | 6.8 | 562.0 | 68.3 | 23.7 | 738.9 | 607.7 | 29.6 | 81.3 |
| **Control** | **Heart** | **Mean** | **PE** | **19.1** | **2069.0** | **26.9** | **4042.4** | **840.7** | **288.9** | **9340.3** | **6171.8** | **315.8** | **883.7** |
| Control | Heart | SD | PE | 10.2 | 241.8 | 1.9 | 514.9 | 62.3 | 134.3 | 760.1 | 745.3 | 133.6 | 67.7 |
| **Treatment** | **Heart** | **Mean** | **PE** | **15.5** | **1969.3** | **109.5** | **4239.2** | **1327.0** | **580.9** | **9726.6** | **6267.3** | **690.3** | **1373.2** |
| Treatment | Heart | SD | PE | 4.8 | 242.4 | 17.6 | 718.4 | 232.3 | 256.4 | 1365.9 | 967.7 | 264.2 | 232.9 |
| **Control** | **Heart** | **Mean** | **FFA** | **66.1** | **983.6** | **63.4** | **384.7** | **1189.6** | **144.1** | **5055.3** | **1465.7** | **207.5** | **1247.0** |
| Control | Heart | SD | FFA | 8.3 | 210.7 | 15.9 | 89.4 | 400.8 | 32.9 | 1227.7 | 309.6 | 28.9 | 420.9 |
| **Treatment** | **Heart** | **Mean** | **FFA** | **52.4** | **560.7** | **73.6** | **206.9** | **466.4** | **90.0** | **2482.9** | **839.0** | **163.6** | **483.8** |
| Treatment | Heart | SD | FFA | 3.0 | 63.8 | 8.4 | 29.2 | 73.1 | 12.1 | 236.2 | 93.0 | 17.9 | 74.3 |
| **Control** | **Heart** | **Mean** | **TAG** | **106.5** | **1555.3** | **236.9** | **187.9** | **2024.8** | **211.2** | **2047.4** | **1918.1** | **448.2** | **2070.7** |
| Control | Heart | SD | TAG | 65.7 | 1116.1 | 217.5 | 96.0 | 1722.3 | 185.7 | 1517.7 | 1285.3 | 403.1 | 1739.9 |
| **Treatment** | **Heart** | **Mean** | **TAG** | **160.8** | **1597.3** | **316.4** | **173.5** | **1775.7** | **210.7** | **2014.7** | **1993.9** | **527.1** | **1813.1** |
| Treatment | Heart | SD | TAG | 135.0 | 1524.0 | 313.7 | 130.7 | 1817.5 | 216.6 | 1893.6 | 1811.4 | 529.7 | 1840.9 |
| **Control** | **Heart** | **Mean** | **CE** | **71.3** | **270.7** | **41.9** | **103.9** | **519.4** | **31.0** | **1773.1** | **492.5** | **72.9** | **540.0** |
| Control | Heart | SD | CE | 9.2 | 29.2 | 3.4 | 14.6 | 64.0 | 6.7 | 117.3 | 41.7 | 8.3 | 66.7 |
| **Treatment** | **Heart** | **Mean** | **CE** | **56.4** | **198.8** | **45.4** | **89.0** | **370.9** | **19.3** | **1358.5** | **379.2** | **64.8** | **383.4** |
| Treatment | Heart | SD | CE | 14.1 | 70.3 | 12.8 | 44.8 | 188.8 | 11.5 | 423.8 | 130.7 | 24.2 | 193.1 |
| **Control** | **Adipose** | **Mean** | **PL** | **178.8** | **1226.4** | **257.0** | **1143.1** | **2093.8** | **190.9** | **3991.0** | **2712.5** | **447.9** | **2183.4** |
| Control | Adipose | SD | PL | 66.3 | 185.8 | 29.5 | 243.6 | 253.9 | 14.7 | 497.9 | 491.6 | 38.7 | 248.7 |
| **Treatment** | **Adipose** | **Mean** | **PL** | **152.1** | **1706.0** | **338.8** | **1240.0** | **1679.9** | **261.8** | **4559.5** | **3281.6** | **600.6** | **1787.7** |
| Treatment | Adipose | SD | PL | 43.5 | 182.8 | 130.3 | 96.7 | 599.7 | 66.6 | 673.6 | 311.9 | 188.5 | 615.4 |
| **Control** | **Adipose** | **Mean** | **FFA** | **306.3** | **1548.6** | **382.6** | **464.7** | **3759.8** | **363.2** | **9896.0** | **2412.1** | **745.9** | **3823.9** |
| Control | Adipose | SD | FFA | 139.2 | 259.0 | 101.4 | 66.6 | 848.3 | 101.4 | 1915.6 | 376.0 | 202.4 | 866.0 |
| **Treatment** | **Adipose** | **Mean** | **FFA** | **391.9** | **2241.1** | **1073.7** | **487.0** | **4055.1** | **461.5** | **13373.4** | **3203.6** | **1535.2** | **4117.3** |
| Treatment | Adipose | SD | FFA | 20.5 | 267.2 | 128.8 | 81.1 | 374.1 | 52.7 | 1121.3 | 339.4 | 167.4 | 378.8 |
| **Control** | **Adipose** | **Mean** | **TAG** | **18475.7** | **616521.6** | **85441.5** | **68609.4** | **1254226.6** | **118808.8** | **1018833.4** | **711111.3** | **204250.3** | **1270899.1** |
| Control | Adipose | SD | TAG | 976.7 | 24505.5 | 7905.4 | 6317.7 | 98155.2 | 11466.9 | 19294.0 | 28595.6 | 17922.4 | 98611.6 |
| **Treatment** | **Adipose** | **Mean** | **TAG** | **52712.2** | **650079.8** | **210006.6** | **43888.4** | **880178.1** | **93310.6** | **962282.9** | **755758.5** | **303317.2** | **891269.3** |
| Treatment | Adipose | SD | TAG | 6816.3 | 31818.2 | 28290.3 | 8339.8 | 89604.0 | 9771.6 | 25731.3 | 36467.8 | 24199.3 | 91655.9 |
| **Control** | **Adipose** | **Mean** | **CE** | **143.0** | **344.6** | **263.5** | **96.2** | **1598.9** | **63.3** | **3607.1** | **726.1** | **326.8** | **1691.0** |
| Control | Adipose | SD | CE | 14.7 | 70.1 | 30.5 | 32.0 | 187.3 | 14.8 | 501.5 | 112.3 | 40.7 | 179.0 |
| **Treatment** | **Adipose** | **Mean** | **CE** | **131.5** | **361.1** | **248.8** | **128.3** | **1135.7** | **44.3** | **3405.8** | **833.5** | **293.1** | **1202.9** |
| Treatment | Adipose | SD | CE | 58.7 | 110.4 | 103.2 | 56.1 | 616.0 | 32.6 | 944.9 | 428.4 | 116.5 | 629.4 |

| Table 6: Rosiglitazone Treatment (Relative) | | | | | | | | | | | | | |
|---|---|---|---|---|---|---|---|---|---|---|---|---|---|
| Treatment | Tissue | | Lipid Class | 14:0 | 16:0 | 16:1n7 | 18:0 | 18:1n9 | 18:1n7 | Total | Saturates | Total n7 | Total n9 |
| **Control** | **Liver** | **Mean** | **PC** | **0.1** | **25.1** | **0.7** | **18.9** | **8.6** | **3.0** | **100.0** | **44.5** | **3.7** | **9.1** |
| Control | Liver | SD | PC | 0.0 | 0.7 | 0.1 | 0.7 | 0.4 | 0.4 | 0.0 | 0.5 | 0.4 | 0.5 |
| **Treatment** | **Liver** | **Mean** | **PC** | **0.2** | **33.3** | **1.7** | **10.3** | **9.6** | **4.6** | **100.0** | **44.1** | **6.4** | **10.2** |
| Treatment | Liver | SD | PC | 0.0 | 0.8 | 0.2 | 0.5 | 0.9 | 0.4 | 0.0 | 0.7 | 0.6 | 1.0 |
| **Control** | **Liver** | **Mean** | **PS/I** | **0.2** | **5.8** | **0.4** | **36.7** | **7.4** | **1.7** | **100.0** | **43.3** | **2.1** | **8.9** |
| Control | Liver | SD | PS/I | 0.1 | 0.8 | 0.1 | 1.5 | 1.7 | 0.3 | 0.0 | 2.1 | 0.4 | 1.8 |
| **Treatment** | **Liver** | **Mean** | **PS/I** | **0.2** | **11.1** | **0.7** | **32.3** | **6.7** | **2.9** | **100.0** | **44.2** | **3.6** | **8.2** |
| Treatment | Liver | SD | PS/I | 0.1 | 1.0 | 0.2 | 1.8 | 0.3 | 0.5 | 0.0 | 2.5 | 0.6 | 0.6 |
| **Control** | **Liver** | **Mean** | **PE** | **0.1** | **18.2** | **0.5** | **21.3** | **8.2** | **2.0** | **100.0** | **39.9** | **2.6** | **8.9** |
| Control | Liver | SD | PE | 0.0 | 0.8 | 0.1 | 1.2 | 1.4 | 0.3 | 0.0 | 1.7 | 0.4 | 1.5 |
| **Treatment** | **Liver** | **Mean** | **PE** | **0.1** | **23.3** | **1.3** | **15.2** | **7.5** | **3.9** | **100.0** | **38.9** | **5.1** | **8.1** |
| Treatment | Liver | SD | PE | 0.0 | 0.8 | 0.1 | 0.9 | 0.8 | 0.3 | 0.0 | 1.2 | 0.4 | 0.8 |
| **Control** | **Liver** | **Mean** | **CL** | **0.5** | **4.8** | **2.8** | **3.1** | **11.3** | **6.1** | **100.0** | **9.0** | **8.9** | **11.9** |
| Control | Liver | SD | CL | 0.1 | 0.8 | 0.2 | 0.6 | 1.6 | 0.3 | 0.0 | 1.1 | 0.3 | 1.5 |
| **Treatment** | **Liver** | **Mean** | **CL** | **0.5** | **5.7** | **6.3** | **3.6** | **10.0** | **5.5** | **100.0** | **10.6** | **11.8** | **10.5** |
| Treatment | Liver | SD | CL | 0.3 | 1.7 | 1.5 | 1.7 | 2.2 | 2.5 | 0.0 | 3.1 | 1.9 | 2.1 |
| **Control** | **Liver** | **Mean** | **FFA** | **1.0** | **18.3** | **4.7** | **4.4** | **29.2** | **4.3** | **100.0** | **24.3** | **9.0** | **29.9** |
| Control | Liver | SD | FFA | 0.1 | 1.0 | 0.4 | 0.2 | 2.9 | 0.5 | 0.0 | 1.2 | 0.8 | 3.0 |
| **Treatment** | **Liver** | **Mean** | **FFA** | **1.0** | **19.9** | **9.4** | **3.0** | **25.4** | **6.4** | **100.0** | **24.4** | **15.8** | **26.0** |
| Treatment | Liver | SD | FFA | 0.1 | 1.2 | 0.7 | 0.3 | 2.7 | 0.6 | 0.0 | 1.2 | 1.0 | 2.8 |
| **Control** | **Liver** | **Mean** | **TAG** | **0.6** | **28.2** | **3.8** | **1.2** | **44.2** | **4.2** | **100.0** | **30.6** | **8.0** | **45.0** |
| Control | Liver | SD | TAG | 0.0 | 1.0 | 0.4 | 0.1 | 2.2 | 0.4 | 0.0 | 1.0 | 0.7 | 2.2 |
| **Treatment** | **Liver** | **Mean** | **TAG** | **0.5** | **27.1** | **8.1** | **0.6** | **39.6** | **5.8** | **100.0** | **28.6** | **13.9** | **40.5** |
| Treatment | Liver | SD | TAG | 0.0 | 1.2 | 0.8 | 0.1 | 2.6 | 0.4 | 0.0 | 1.3 | 0.8 | 2.7 |
| **Control** | **Liver** | **Mean** | **CE** | **1.3** | **45.7** | **5.5** | **4.1** | **26.0** | **1.9** | **100.0** | **52.1** | **7.4** | **26.5** |
| Control | Liver | SD | CE | 0.2 | 3.6 | 0.3 | 0.5 | 2.9 | 0.2 | 0.0 | 4.0 | 0.4 | 2.8 |
| **Treatment** | **Liver** | **Mean** | **CE** | **1.9** | **48.0** | **11.8** | **3.5** | **19.9** | **2.3** | **100.0** | **54.6** | **14.1** | **20.5** |
| Treatment | Liver | SD | CE | 0.3 | 3.7 | 1.4 | 0.3 | 4.8 | 0.5 | 0.0 | 3.8 | 1.1 | 4.8 |
| **Control** | **Plasma** | **Mean** | **PC** | **0.1** | **23.4** | **0.3** | **20.7** | **7.6** | **2.6** | **100.0** | **44.7** | **3.0** | **8.2** |
| Control | Plasma | SD | PC | 0.0 | 1.2 | 0.0 | 0.9 | 0.4 | 0.2 | 0.0 | 0.4 | 0.2 | 0.5 |
| **Treatment** | **Plasma** | **Mean** | **PC** | **0.1** | **30.8** | **0.8** | **13.0** | **9.4** | **4.1** | **100.0** | **44.6** | **4.9** | **9.9** |
| Treatment | Plasma | SD | PC | 0.0 | 1.0 | 0.1 | 0.4 | 0.9 | 0.3 | 0.0 | 1.0 | 0.3 | 0.9 |
| **Control** | **Plasma** | **Mean** | **PE** | **1.3** | **15.7** | **1.1** | **16.4** | **14.0** | **1.7** | **100.0** | **34.8** | **2.8** | **14.7** |
| Control | Plasma | SD | PE | 0.3 | 1.5 | 0.5 | 1.6 | 7.0 | 0.3 | 0.0 | 2.9 | 0.8 | 6.5 |
| **Treatment** | **Plasma** | **Mean** | **PE** | **1.3** | **20.4** | **1.7** | **15.5** | **16.1** | **2.7** | **100.0** | **40.9** | **4.4** | **17.3** |
| Treatment | Plasma | SD | PE | 0.2 | 4.9 | 0.7 | 4.3 | 9.8 | 0.5 | 0.0 | 9.6 | 1.0 | 10.0 |
| **Control** | **Plasma** | **Mean** | **FFA** | **1.8** | **27.7** | **3.5** | **10.0** | **21.2** | **2.8** | **100.0** | **40.7** | **6.4** | **21.9** |
| Control | Plasma | SD | FFA | 0.7 | 1.3 | 0.6 | 1.5 | 2.2 | 0.2 | 0.0 | 3.3 | 0.6 | 2.2 |
| **Treatment** | **Plasma** | **Mean** | **FFA** | **3.3** | **30.9** | **8.3** | **8.1** | **18.4** | **3.2** | **100.0** | **43.8** | **11.5** | **18.9** |
| Treatment | Plasma | SD | FFA | 0.9 | 1.7 | 1.9 | 1.6 | 1.9 | 0.6 | 0.0 | 3.3 | 1.7 | 2.0 |
| **Control** | **Plasma** | **Mean** | **TAG** | **0.7** | **21.4** | **3.4** | **1.7** | **35.6** | **3.4** | **100.0** | **24.4** | **6.7** | **36.6** |
| Control | Plasma | SD | TAG | 0.0 | 0.8 | 0.2 | 0.1 | 3.2 | 0.2 | 0.0 | 0.9 | 0.2 | 3.3 |
| **Treatment** | **Plasma** | **Mean** | **TAG** | **1.2** | **21.9** | **7.0** | **1.8** | **27.2** | **4.5** | **100.0** | **25.8** | **11.5** | **27.9** |
| Treatment | Plasma | SD | TAG | 0.2 | 1.0 | 0.3 | 0.3 | 1.8 | 0.4 | 0.0 | 1.5 | 0.5 | 1.9 |
| **Control** | **Plasma** | **Mean** | **CE** | **0.3** | **2.8** | **2.3** | **0.2** | **6.2** | **0.5** | **100.0** | **3.5** | **2.8** | **6.6** |
| Control | Plasma | SD | CE | 0.0 | 0.2 | 0.2 | 0.0 | 0.8 | 0.1 | 0.0 | 0.3 | 0.2 | 0.7 |
| **Treatment** | **Plasma** | **Mean** | **CE** | **0.6** | **3.7** | **6.5** | **0.2** | **6.1** | **0.7** | **100.0** | **4.9** | **7.3** | **6.6** |
| Treatment | Plasma | SD | CE | 0.1 | 0.2 | 0.8 | 0.0 | 0.7 | 0.0 | 0.0 | 0.5 | 0.9 | 0.8 |
| **Control** | **Plasma** | **Mean** | **PL** | **0.1** | **23.0** | **0.3** | **21.0** | **7.2** | **2.5** | **100.0** | **45.0** | **2.8** | **8.3** |
| Control | Plasma | SD | PL | 0.0 | 0.8 | 0.0 | 0.7 | 0.5 | 0.3 | 0.0 | 0.3 | 0.2 | 0.5 |
| **Treatment** | **Plasma** | **Mean** | **PL** | **0.2** | **30.9** | **0.9** | **11.9** | **7.4** | **3.9** | **100.0** | **43.9** | **4.8** | **8.7** |
| Treatment | Plasma | SD | PL | 0.0 | 2.2 | 0.1 | 5.8 | 3.7 | 0.4 | 0.0 | 4.4 | 0.5 | 3.6 |
| **Control** | **Adipose** | **Mean** | **PL** | **2.2** | **15.3** | **3.3** | **14.3** | **26.3** | **2.4** | **100.0** | **33.8** | **5.7** | **27.4** |
| Control | Adipose | SD | PL | 0.5 | 0.4 | 0.5 | 1.7 | 1.9 | 0.3 | 0.0 | 2.1 | 0.7 | 1.9 |
| **Treatment** | **Adipose** | **Mean** | **PL** | **1.6** | **18.8** | **3.6** | **13.7** | **18.0** | **2.8** | **100.0** | **36.3** | **6.5** | **19.2** |
| Treatment | Adipose | SD | PL | 0.3 | 1.6 | 0.9 | 1.3 | 4.3 | 0.4 | 0.0 | 2.7 | 1.1 | 4.3 |
| **Control** | **Adipose** | **Mean** | **FFA** | **3.3** | **15.7** | **3.8** | **4.8** | **37.8** | **3.6** | **100.0** | **24.8** | **7.5** | **38.5** |
| Control | Adipose | SD | FFA | 2.0 | 1.2 | 0.4 | 1.0 | 2.2 | 0.4 | 0.0 | 4.4 | 0.7 | 2.3 |
| **Treatment** | **Adipose** | **Mean** | **FFA** | **3.0** | **16.7** | **8.0** | **3.6** | **30.4** | **3.4** | **100.0** | **23.9** | **11.5** | **30.8** |
| Treatment | Adipose | SD | FFA | 0.4 | 0.9 | 0.6 | 0.4 | 2.1 | 0.2 | 0.0 | 1.2 | 0.5 | 2.1 |
| **Control** | **Adipose** | **Mean** | **TAG** | **0.6** | **20.2** | **2.8** | **2.2** | **41.0** | **3.9** | **100.0** | **23.3** | **6.7** | **41.6** |
| Control | Adipose | SD | TAG | 0.0 | 0.6 | 0.2 | 0.2 | 3.0 | 0.4 | 0.0 | 0.8 | 0.5 | 3.0 |
| **Treatment** | **Adipose** | **Mean** | **TAG** | **1.8** | **22.5** | **7.3** | **1.5** | **30.5** | **3.2** | **100.0** | **26.2** | **10.5** | **30.8** |
| Treatment | Adipose | SD | TAG | 0.3 | 0.8 | 1.1 | 0.3 | 2.6 | 0.3 | 0.0 | 0.9 | 0.9 | 2.6 |
| **Control** | **Adipose** | **Mean** | **CE** | **4.0** | **9.5** | **7.4** | **2.7** | **44.5** | **1.7** | **100.0** | **20.2** | **9.1** | **47.1** |
| Control | Adipose | SD | CE | 0.5 | 1.2 | 0.7 | 1.1 | 2.1 | 0.2 | 0.0 | 2.9 | 0.6 | 2.4 |
| **Treatment** | **Adipose** | **Mean** | **CE** | **3.8** | **10.9** | **7.1** | **3.8** | **31.8** | **1.3** | **100.0** | **24.3** | **8.4** | **33.7** |
| Treatment | Adipose | SD | CE | 0.8 | 2.8 | 1.7 | 1.1 | 10.9 | 0.7 | 0.0 | 8.5 | 1.8 | 10.9 |
| **Control** | **Heart** | **Mean** | **PC** | **0.1** | **27.7** | **0.2** | **17.3** | **5.1** | **1.7** | **100.0** | **45.4** | **1.9** | **5.3** |
| Control | Heart | SD | PC | 0.0 | 0.7 | 0.0 | 0.7 | 0.5 | 0.1 | 0.0 | 0.7 | 0.2 | 0.5 |
| **Treatment** | **Heart** | **Mean** | **PC** | **0.2** | **29.2** | **1.0** | **11.6** | **10.7** | **4.0** | **100.0** | **41.4** | **5.0** | **10.9** |
| Treatment | Heart | SD | PC | 0.0 | 1.5 | 0.1 | 1.3 | 0.7 | 0.2 | 0.0 | 0.8 | 0.3 | 0.7 |
| **Control** | **Heart** | **Mean** | **PS/I** | **0.3** | **4.2** | **0.2** | **33.9** | **4.1** | **0.8** | **100.0** | **39.0** | **1.0** | **4.7** |
| Control | Heart | SD | PS/I | 0.1 | 0.4 | 0.1 | 1.6 | 0.7 | 0.1 | 0.0 | 1.6 | 0.1 | 0.7 |
| **Treatment** | **Heart** | **Mean** | **PS/I** | **0.3** | **5.2** | **0.4** | **33.6** | **5.1** | **1.4** | **100.0** | **39.7** | **1.8** | **5.8** |
| Treatment | Heart | SD | PS/I | 0.1 | 1.1 | 0.1 | 2.7 | 0.4 | 0.1 | 0.0 | 2.1 | 0.2 | 0.5 |
| **Control** | **Heart** | **Mean** | **PE** | **0.1** | **11.1** | **0.1** | **21.6** | **4.5** | **1.5** | **100.0** | **33.0** | **1.7** | **4.7** |
| Control | Heart | SD | PE | 0.1 | 0.6 | 0.0 | 1.2 | 0.2 | 0.7 | 0.0 | 1.5 | 0.7 | 0.1 |
| **Treatment** | **Heart** | **Mean** | **PE** | **0.1** | **10.1** | **0.6** | **21.7** | **6.8** | **2.9** | **100.0** | **32.2** | **3.5** | **7.0** |
| Treatment | Heart | SD | PE | 0.0 | 0.4 | 0.1 | 1.1 | 0.4 | 1.2 | 0.0 | 1.4 | 1.2 | 0.4 |
| **Control** | **Heart** | **Mean** | **CL** | **0.4** | **2.8** | **1.1** | **1.7** | **11.7** | **7.5** | **100.0** | **5.2** | **8.6** | **12.0** |
| Control | Heart | SD | CL | 0.1 | 0.2 | 0.1 | 0.4 | 0.8 | 0.5 | 0.0 | 0.5 | 0.4 | 0.8 |
| **Treatment** | **Heart** | **Mean** | **CL** | **0.2** | **1.3** | **2.0** | **2.4** | **5.0** | **3.0** | **100.0** | **4.1** | **5.1** | **5.3** |
| Treatment | Heart | SD | CL | 0.0 | 0.3 | 0.3 | 1.4 | 0.5 | 0.4 | 0.0 | 1.1 | 0.4 | 0.5 |
| **Control** | **Heart** | **Mean** | **FFA** | **1.4** | **19.7** | **1.4** | **7.7** | **22.9** | **2.9** | **100.0** | **29.4** | **4.3** | **24.0** |
| Control | Heart | SD | FFA | 0.5 | 1.4 | 0.9 | 0.6 | 3.2 | 0.2 | 0.0 | 2.3 | 1.1 | 3.4 |
| **Treatment** | **Heart** | **Mean** | **FFA** | **2.1** | **22.6** | **3.0** | **8.3** | **18.7** | **3.6** | **100.0** | **33.8** | **6.6** | **19.4** |
| Treatment | Heart | SD | FFA | 0.2 | 1.4 | 0.3 | 0.9 | 1.7 | 0.3 | 0.0 | 2.3 | 0.5 | 1.7 |
| **Control** | **Heart** | **Mean** | **TAG** | **1.8** | **25.4** | **3.5** | **3.5** | **31.0** | **3.2** | **100.0** | **32.1** | **6.7** | **31.9** |
| Control | Heart | SD | TAG | 0.3 | 1.3 | 0.7 | 1.2 | 3.8 | 0.4 | 0.0 | 2.8 | 1.1 | 3.7 |
| **Treatment** | **Heart** | **Mean** | **TAG** | **3.0** | **26.0** | **4.9** | **3.5** | **26.2** | **3.2** | **100.0** | **34.1** | **8.1** | **27.1** |
| Treatment | Heart | SD | TAG | 0.5 | 1.0 | 0.5 | 0.9 | 4.8 | 0.5 | 0.0 | 1.8 | 0.9 | 4.4 |
| **Control** | **Heart** | **Mean** | **CE** | **4.0** | **15.3** | **2.4** | **5.9** | **29.3** | **1.7** | **100.0** | **27.8** | **4.1** | **30.5** |
| Control | Heart | SD | CE | 0.6 | 1.6 | 0.1 | 0.6 | 3.4 | 0.3 | 0.0 | 2.3 | 0.4 | 3.6 |
| **Treatment** | **Heart** | **Mean** | **CE** | **4.3** | **14.6** | **3.4** | **6.4** | **25.5** | **1.3** | **100.0** | **27.9** | **4.7** | **26.4** |
| Treatment | Heart | SD | CE | 0.6 | 1.3 | 0.4 | 1.5 | 9.1 | 0.7 | 0.0 | 2.6 | 0.7 | 9.2 |

| Table 7: CL316,243 Treatment (Quantitative) | | | | | | | | | | | | | |
|---|---|---|---|---|---|---|---|---|---|---|---|---|---|
| Treatment | Tissue | | Lipid Class | 14:0 | 16:0 | 16:1n7 | 18:0 | 18: 1n9 | 18: 1n7 | Total nMole of FA | Saturates | Total n7 | Total n9 |
| **Control** | **Plasma** | **Mean** | **CE** | **22.3** | **144.8** | **228.6** | **12.6** | **265.4** | **22.1** | **3722.6** | **192.1** | **250.7** | **275.3** |
| Control | Plasma | SD | CE | 1.4 | 7.0 | 23.2 | 3.0 | 38.6 | 3.0 | 460.6 | 8.7 | 25.0 | 41.4 |
| **Treatment** | **Plasma** | **Mean** | **CE** | **21.2** | **114.4** | **84.9** | **16.5** | **194.2** | **13.5** | **2584.1** | **162.9** | **98.4** | **198.7** |
| Treatment | Plasma | SD | CE | 2.0 | 10.3 | 31.5 | 5.3 | 32.6 | 3.3 | 376.8 | 12.5 | 34.5 | 33.8 |
| **Control** | **Plasma** | **Mean** | **FFA** | **33.4** | **177.2** | **60.5** | **41.1** | **206.2** | **16.3** | **673.1** | **261.1** | **76.9** | **209.8** |
| Control | Plasma | SD | FFA | 10.0 | 25.0 | 19.8 | 5.2 | 171.5 | 9.4 | 227.3 | 34.0 | 27.0 | 173.0 |
| **Treatment** | **Plasma** | **Mean** | **FFA** | **22.1** | **102.3** | **15.4** | **36.9** | **160.6** | **9.8** | **424.7** | **167.7** | **25.2** | **163.1** |
| Treatment | Plasma | SD | FFA | 7.9 | 18.5 | 12.2 | 5.9 | 154.2 | 7.9 | 214.1 | 31.4 | 20.1 | 155.7 |
| **Control** | **Plasma** | **Mean** | **LysoPC** | **4.5** | **392.0** | **11.8** | **183.3** | **84.0** | **15.0** | **949.3** | **593.9** | **26.8** | **88.1** |
| Control | Plasma | SD | LysoPC | 2.0 | 42.7 | 2.4 | 24.2 | 16.0 | 0.8 | 79.4 | 42.9 | 1.7 | 16.6 |
| **Treatment** | **Plasma** | **Mean** | **LysoPC** | **2.9** | **241.6** | **4.8** | **127.4** | **47.5** | **6.9** | **643.1** | **381.9** | **11.8** | **49.4** |
| Treatment | Plasma | SD | LysoPC | 1.0 | 55.8 | 2.2 | 22.6 | 17.5 | 3.0 | 160.2 | 75.7 | 5.2 | 18.4 |
| **Control** | **Plasma** | **Mean** | **PC** | **7.4** | **1844.3** | **44.8** | **872.2** | **553.8** | **123.1** | **2930.5** | **2760.0** | **167.9** | **574.4** |
| Control | Plasma | SD | PC | 2.0 | 128.4 | 6.2 | 154.4 | 86.7 | 20.6 | 292.6 | 282.0 | 26.6 | 92.1 |
| **Treatment** | **Plasma** | **Mean** | **PC** | **5.5** | **1121.4** | **15.3** | **670.9** | **309.6** | **58.8** | **1928.9** | **1820.6** | **74.0** | **320.0** |
| Treatment | Plasma | SD | PC | 1.4 | 214.9 | 6.5 | 87.5 | 83.8 | 21.2 | 331.8 | 304.9 | 27.4 | 85.5 |
| **Control** | **Plasma** | **Mean** | **PE** | **4.4** | **81.8** | **6.4** | **64.8** | **57.3** | **6.8** | **208.0** | **161.2** | **13.1** | **62.3** |
| Control | Plasma | SD | PE | 4.0 | 26.9 | 5.1 | 20.1 | 58.5 | 3.1 | 72.4 | 51.8 | 7.9 | 60.0 |
| **Treatment** | **Plasma** | **Mean** | **PE** | **4.1** | **33.9** | **3.3** | **28.2** | **31.5** | **2.5** | **106.7** | **74.0** | **5.8** | **33.4** |
| Treatment | Plasma | SD | PE | 1.6 | 7.3 | 2.9 | 3.7 | 30.8 | 1.4 | 26.4 | 12.1 | 4.3 | 31.0 |
| **Control** | **Plasma** | **Mean** | **PL** | **9.2** | **2153.1** | **55.6** | **1143.0** | **665.0** | **144.9** | **3699.3** | **3375.8** | **200.5** | **726.8** |
| Control | Plasma | SD | PL | 0.5 | 136.3 | 7.0 | 195.9 | 92.1 | 26.7 | 401.0 | 324.9 | 33.3 | 100.6 |
| **Treatment** | **Plasma** | **Mean** | **PL** | **7.6** | **1315.0** | **20.3** | **847.2** | **380.8** | **69.7** | **2464.5** | **2221.2** | **90.0** | **429.4** |
| Treatment | Plasma | SD | PL | 1.0 | 204.1 | 6.4 | 71.5 | 63.3 | 21.6 | 305.3 | 273.1 | 27.9 | 74.7 |
| **Control** | **Plasma** | **Mean** | **TAG** | **51.6** | **1189.4** | **337.1** | **80.9** | **1459.0** | **183.9** | **1603.3** | **1346.6** | **521.0** | **1499.3** |
| Control | Plasma | SD | TAG | 4.8 | 91.5 | 51.9 | 8.6 | 91.4 | 17.8 | 126.8 | 96.7 | 67.6 | 89.7 |
| **Treatment** | **Plasma** | **Mean** | **TAG** | **17.6** | **219.3** | **33.1** | **45.7** | **268.3** | **23.7** | **344.8** | **295.4** | **56.8** | **276.2** |
| Treatment | Plasma | SD | TAG | 2.2 | 37.2 | 9.3 | 18.9 | 55.1 | 7.1 | 41.9 | 50.6 | 16.3 | 56.9 |
| **Control** | **Liver** | **Mean** | **CE** | **49.3** | **466.8** | **171.0** | **81.4** | **852.8** | **48.1** | **2184.8** | **642.7** | **219.1** | **884.1** |
| Control | Liver | SD | CE | 25.2 | 225.4 | 56.9 | 16.7 | 629.0 | 22.3 | 782.1 | 216.2 | 78.3 | 641.1 |
| **Treatment** | **Liver** | **Mean** | **CE** | **45.5** | **462.8** | **84.4** | **121.3** | **455.4** | **31.5** | **1764.6** | **668.6** | **115.9** | **482.4** |
| Treatment | Liver | SD | CE | 20.3 | 204.2 | 33.9 | 36.1 | 392.4 | 6.9 | 420.7 | 248.2 | 35.4 | 377.6 |
| **Control** | **Liver** | **Mean** | **CL** | **16.5** | **239.5** | **146.3** | **210.6** | **446.9** | **238.5** | **1064.8** | **497.3** | **384.8** | **483.6** |
| Control | Liver | SD | CL | 23.8 | 62.2 | 114.0 | 136.3 | 93.1 | 31.5 | 158.2 | 200.0 | 115.0 | 79.4 |
| **Treatment** | **Liver** | **Mean** | **CL** | **21.3** | **254.3** | **85.8** | **260.0** | **437.0** | **215.4** | **1255.5** | **556.4** | **301.2** | **473.0** |
| Treatment | Liver | SD | CL | 41.4 | 146.7 | 100.2 | 192.2 | 123.1 | 46.5 | 280.5 | 360.0 | 124.2 | 134.7 |
| **Control** | **Liver** | **Mean** | **FFA** | **165.5** | **2415.5** | **784.8** | **408.3** | **2732.3** | **467.7** | **10522.8** | **3036.2** | **1252.5** | **2805.1** |
| Control | Liver | SD | FFA | 51.2 | 332.9 | 127.1 | 65.9 | 395.9 | 76.3 | 1476.7 | 381.1 | 198.5 | 398.0 |
| **Treatment** | **Liver** | **Mean** | **FFA** | **152.9** | **2280.7** | **375.0** | **460.2** | **2293.4** | **273.5** | **9555.8** | **2951.3** | **648.5** | **2337.2** |
| Treatment | Liver | SD | FFA | 5.0 | 273.2 | 99.3 | 56.1 | 620.7 | 86.8 | 1596.7 | 301.6 | 183.5 | 638.1 |
| **Control** | **Liver** | **Mean** | **PC** | **55.9** | **11483.6** | **616.7** | **4498.9** | **3831.1** | **926.2** | **17936.6** | **16171.3** | **1542.9** | **3961.4** |
| Control | Liver | SD | PC | 3.7 | 887.7 | 56.6 | 534.8 | 366.8 | 93.8 | 975.6 | 890.6 | 94.4 | 388.0 |
| **Treatment** | **Liver** | **Mean** | **PC** | **69.0** | **11221.7** | **358.8** | **5889.5** | **3855.0** | **741.3** | **19052.2** | **17292.7** | **1100.1** | **3964.0** |
| Treatment | Liver | SD | PC | 6.9 | 1104.4 | 84.4 | 498.3 | 418.1 | 236.0 | 1477.5 | 1140.6 | 318.1 | 440.5 |
| **Control** | **Liver** | **Mean** | **PE** | **10.8** | **3934.4** | **182.1** | **3035.5** | **1137.8** | **306.5** | **8631.8** | **7042.4** | **488.6** | **1205.0** |
| Control | Liver | SD | PE | 1.5 | 411.8 | 20.2 | 236.6 | 134.0 | 22.3 | 621.4 | 543.4 | 33.9 | 142.6 |
| **Treatment** | **Liver** | **Mean** | **PE** | **13.1** | **4406.0** | **117.9** | **3777.4** | **1320.6** | **247.4** | **9924.9** | **8245.4** | **365.3** | **1376.1** |
| Treatment | Liver | SD | PE | 3.1 | 517.5 | 31.3 | 448.0 | 217.3 | 79.8 | 893.1 | 737.1 | 108.6 | 230.6 |
| **Control** | **Liver** | **Mean** | **PL** | **85.2** | **16916.1** | **1028.4** | **10230.1** | **5837.9** | **1591.1** | **33320.7** | **27796.2** | **2619.5** | **6298.4** |
| Control | Liver | SD | PL | 8.7 | 1495.8 | 61.1 | 526.1 | 180.4 | 110.2 | 1360.1 | 1552.6 | 124.7 | 196.3 |
| **Treatment** | **Liver** | **Mean** | **PL** | **91.0** | **16120.7** | **586.5** | **12438.5** | **5839.1** | **1242.7** | **34731.4** | **29247.6** | **1829.2** | **6261.0** |
| Treatment | Liver | SD | PL | 8.8 | 1452.8 | 136.0 | 1266.4 | 625.6 | 313.8 | 2715.5 | 2254.0 | 445.5 | 714.8 |
| **Control** | **Liver** | **Mean** | **PS/I** | **18.7** | **1048.6** | **59.3** | **3860.1** | **514.7** | **132.3** | **5260.4** | **4992.8** | **191.6** | **647.8** |
| Control | Liver | SD | PS/I | 2.3 | 148.1 | 11.7 | 173.1 | 122.3 | 22.4 | 359.6 | 313.3 | 34.0 | 154.8 |
| **Treatment** | **Liver** | **Mean** | **PS/I** | **19.9** | **898.1** | **37.8** | **4313.9** | **486.8** | **100.2** | **5625.0** | **5283.6** | **138.0** | **607.6** |
| Treatment | Liver | SD | PS/I | 2.7 | 84.0 | 6.1 | 217.2 | 53.3 | 13.1 | 361.6 | 282.7 | 18.0 | 69.4 |
| **Control** | **Liver** | **Mean** | **TAG** | **242.1** | **10322.3** | **1564.5** | **477.2** | **10542.3** | **1096.2** | **9614.7** | **11277.1** | **2660.7** | **10812.6** |
| Control | Liver | SD | TAG | 60.1 | 2941.5 | 475.2 | 109.2 | 3387.1 | 358.3 | 2698.6 | 3135.0 | 819.6 | 3452.2 |
| **Treatment** | **Liver** | **Mean** | **TAG** | **207.9** | **6580.5** | **675.6** | **668.7** | **7123.9** | **619.4** | **6910.6** | **7585.2** | **1295.0** | **7280.9** |
| Treatment | Liver | SD | TAG | 85.1 | 2972.9 | 372.8 | 366.8 | 3628.8 | 368.4 | 3270.4 | 3318.5 | 732.4 | 3660.1 |
| **Control** | **Heart** | **Mean** | **CE** | **44.2** | **137.4** | **33.0** | **68.8** | **223.4** | **10.5** | **1064.3** | **286.3** | **43.5** | **235.9** |
| Control | Heart | SD | CE | 14.7 | 44.1 | 11.3 | 33.7 | 156.1 | 8.0 | 247.6 | 93.6 | 18.6 | 160.5 |
| **Treatment** | **Heart** | **Mean** | **CE** | **59.3** | **187.4** | **36.2** | **85.3** | **389.5** | **19.6** | **1226.9** | **366.8** | **55.8** | **405.7** |
| Treatment | Heart | SD | CE | 10.6 | 60.9 | 13.6 | 32.2 | 195.1 | 10.8 | 421.1 | 119.8 | 24.1 | 197.9 |
| **Control** | **Heart** | **Mean** | **CL** | **58.4** | **299.8** | **291.5** | **706.0** | **1118.6** | **540.5** | **3547.4** | **1121.9** | **832.0** | **1205.4** |
| Control | Heart | SD | CL | 32.9 | 142.0 | 50.4 | 567.0 | 259.0 | 89.5 | 631.1 | 664.8 | 138.4 | 253.7 |
| **Treatment** | **Heart** | **Mean** | **CL** | **76.3** | **231.1** | **204.0** | **397.1** | **956.2** | **381.8** | **2975.1** | **776.3** | **585.8** | **1011.4** |
| Treatment | Heart | SD | CL | 40.4 | 116.7 | 21.8 | 242.4 | 225.3 | 53.8 | 306.5 | 272.4 | 73.5 | 214.9 |
| **Control** | **Heart** | **Mean** | **FFA** | **126.2** | **1291.1** | **158.2** | **510.6** | **1786.2** | **194.8** | **6404.9** | **1972.7** | **353.0** | **1870.1** |
| Control | Heart | SD | FFA | 13.7 | 170.5 | 21.5 | 111.2 | 286.1 | 39.3 | 1029.7 | 300.4 | 60.1 | 301.1 |
| **Treatment** | **Heart** | **Mean** | **FFA** | **181.4** | **1012.4** | **156.8** | **427.8** | **1964.0** | **139.4** | **6044.8** | **1683.2** | **296.1** | **2017.6** |
| Treatment | Heart | SD | FFA | 106.6 | 212.1 | 122.8 | 123.9 | 1374.9 | 70.7 | 2011.7 | 364.1 | 193.0 | 1382.2 |
| **Control** | **Heart** | **Mean** | **PC** | **70.6** | **8467.1** | **181.4** | **4309.3** | **2625.8** | **865.4** | **14730.7** | **12934.3** | **1046.8** | **2695.4** |
| Control | Heart | SD | PC | 3.8 | 287.5 | 17.0 | 351.8 | 121.3 | 39.8 | 719.6 | 555.8 | 54.1 | 124.7 |
| **Treatment** | **Heart** | **Mean** | **PC** | **94.6** | **8127.9** | **108.7** | **5235.9** | **1990.2** | **568.2** | **15087.3** | **13577.6** | **676.9** | **2052.1** |
| Treatment | Heart | SD | PC | 8.6 | 437.6 | 13.7 | 204.7 | 187.0 | 42.6 | 604.9 | 600.7 | 56.1 | 192.7 |
| **Control** | **Heart** | **Mean** | **PE** | **22.7** | **1990.1** | **107.3** | **4772.0** | **1161.9** | **440.5** | **10150.1** | **6833.9** | **547.8** | **1216.4** |
| Control | Heart | SD | PE | 9.3 | 256.8 | 42.3 | 1014.3 | 300.4 | 95.4 | 2087.9 | 1246.8 | 129.1 | 315.5 |
| **Treatment** | **Heart** | **Mean** | **PE** | **27.5** | **1814.0** | **59.6** | **5241.4** | **934.9** | **250.5** | **9823.7** | **7157.0** | **310.1** | **972.0** |
| Treatment | Heart | SD | PE | 11.4 | 145.5 | 13.7 | 767.3 | 117.5 | 44.0 | 1145.0 | 917.9 | 55.5 | 125.3 |
| **Control** | **Heart** | **Mean** | **PL** | **115.1** | **11023.2** | **472.5** | **11983.1** | **5114.0** | **1816.1** | **33815.3** | **23571.8** | **2288.6** | **5388.8** |
| Control | Heart | SD | PL | 9.3 | 458.2 | 52.5 | 571.7 | 379.3 | 136.7 | 1475.2 | 1048.3 | 186.3 | 394.0 |
| **Treatment** | **Heart** | **Mean** | **PL** | **170.5** | **10799.7** | **344.7** | **13320.8** | **4121.5** | **1181.0** | **33715.3** | **24932.5** | **1525.7** | **4384.8** |
| Treatment | Heart | SD | PL | 12.1 | 703.7 | 45.3 | 626.7 | 359.6 | 80.7 | 1699.7 | 1336.4 | 116.9 | 417.5 |
| **Control** | **Heart** | **Mean** | **PS/I** | **26.8** | **462.8** | **47.9** | **3005.7** | **559.8** | **107.8** | **4868.1** | **3556.2** | **155.7** | **610.4** |
| Control | Heart | SD | PS/I | 14.0 | 226.1 | 23.9 | 671.4 | 250.5 | 26.0 | 1073.2 | 866.7 | 49.1 | 262.3 |
| **Treatment** | **Heart** | **Mean** | **PS/I** | **33.4** | **302.1** | **48.6** | **2823.7** | **572.9** | **73.5** | **4276.3** | **3245.9** | **122.1** | **611.1** |
| Treatment | Heart | SD | PS/I | 5.7 | 26.7 | 10.0 | 149.7 | 146.3 | 11.0 | 263.4 | 160.1 | 20.5 | 150.3 |
| **Control** | **Heart** | **Mean** | **TAG** | **91.8** | **997.2** | **112.8** | **180.3** | **676.2** | **72.2** | **927.0** | **1324.0** | **185.0** | **701.3** |
| Control | Heart | SD | TAG | 9.8 | 176.6 | 13.2 | 16.7 | 135.6 | 1.8.8 | 135.4 | 189.5 | 21.6 | 143.6 |
| **Treatment** | **Heart** | **Mean** | **TAG** | **78.8** | **651.6** | **57.3** | **195.5** | **402.8** | **28.0** | **628.9** | **988.5** | **85.3** | **426.3** |
| Treatment | Heart | SD | TAG | 29.7 | 228.9 | 34.3 | 92.2 | 108.0 | 5.7 | 189.7 | 359.3 | 39.5 | 106.6 |
| **Control** | **Adipose** | **Mean** | **CE** | **52.2** | **260.7** | **50.2** | **82.5** | **359.4** | **42.5** | **1280.1** | **430.5** | **92.8** | **397.2** |
| Control | Adipose | SD | CE | 23.4 | 120.6 | 34.5 | 29.1 | 140.6 | 17.3 | 506.2 | 183.1 | 47.2 | 157.4 |
| **Treatment** | **Adipose** | **Mean** | **CE** | **65.2** | **326.2** | **85.7** | **72.1** | **344.4** | **50.6** | **1340.0** | **491.1** | **136.3** | **385.7** |
| Treatment | Adipose | SD | CE | 15.2 | 116.4 | 53.6 | 13.9 | 119.1 | 12.3 | 418.3 | 143.5 | 65.0 | 122.4 |
| **Control** | **Adipose** | **Mean** | **FFA** | **794.6** | **5399.0** | **1670.1** | **2502.1** | **14460.3** | **699.3** | **36569.3** | **8966.6** | **2369.4** | **14766.7** |
| Control | Adipose | SD | FFA | 189.7 | 1500.9 | 531.1 | 587.5 | 3839.9 | 160.1 | 8994.8 | 2347.7 | 688.6 | 3924.2 |
| **Treatment** | **Adipose** | **Mean** | **FFA** | **833.3** | **6822.9** | **2652.0** | **1467.5** | **9845.4** | **864.3** | **29061.0** | **9306.7** | **3516.3** | **10077.8** |
| Treatment | Adipose | SD | FFA | 381.6 | 4195.0 | 865.6 | 872.7 | 5015.6 | 462.7 | 14417.5 | 5558.2 | 1316.1 | 5164.8 |
| **Control** | **Adipose** | **Mean** | **PL** | **68.9** | **2605.8** | **327.5** | **5741.2** | **3090.8** | **292.2** | **12024.1** | **8643.6** | **619.6** | **3174.2** |
| Control | Adipose | SD | PL | 11.6 | 286.5 | 80.3 | 726.9 | 632.6 | 67.4 | 1208.6 | 976.8 | 145.7 | 637.7 |
| **Treatment** | **Adipose** | **Mean** | **PL** | **74.4** | **1403.3** | **291.2** | **1249.1** | **794.7** | **118.1** | **3468.8** | **2850.0** | **409.3** | **845.8** |
| Treatment | Adipose | SD | PL | 19.0 | 272.8 | 129.3 | 172.2 | 182.6 | 25.6 | 616.5 | 443.5 | 154.7 | 194.5 |
| **Control** | **Adipose** | **Mean** | **TAG** | **43154.8** | **415224.2** | **57170.5** | **123491.3** | **533199.8** | **26555.4** | **548254.5** | **599292.9** | **83725.9** | **542927.3** |
| Control | Adipose | SD | TAG | 6511.3 | 80767.9 | 9812.5 | 53298.1 | 52045.2 | 2991.8 | 83554.9 | 147566.6 | 9354.2 | 55209.6 |
| **Treatment** | **Adipose** | **Mean** | **TAG** | **79501.6** | **666399.1** | **265948.0** | **73800.5** | **793227.6** | **49837.1** | **824978.7** | **824049.8** | **315785.2** | **802281.4** |
| Treatment | Adipose | SD | TAG | 9211.2 | 78602.1 | 20379.9 | 18401.0 | 148042.1 | 24507.1 | 95124.5 | 94536.6 | 36771.4 | 150656.2 |

| Table 8: CL316,243 Treatment (Relative) | | | | | | | | | | | | | |
|---|---|---|---|---|---|---|---|---|---|---|---|---|---|
| Treatment | Tissue | | Lipid Class | 14:0 | 16:0. | 16:1n7 | 18:0. | 18:1n9 | 18:1n7 | Total | Saturates | Total n7 | Total n9 |
| **Control** | **Adipose** | **Mean** | **CE** | **3.9** | **20.8** | **4.3** | **6.5** | **28.4** | **3.3** | **100.0.** | **33.7** | **7.5** | **31.1** |
| Control | Adipose | SD | CE | 0.6 | 4.1 | 2.2 | 0.8 | 1.3 | 0.3 | 0.0 | 3.4 | 2.1 | 1.7 |
| **Treatment** | **Adipose** | **Mean** | **CE** | **4.5** | **24.4** | **6.8** | **5.1** | **26.2** | **3.5** | **100.0** | **35.7** | **10.3** | **28.8** |
| Treatment | Adipose | SD | CE | 1.1 | 2.1 | 2.2 | 1.0 | 3.0 | 0.7 | 0.0 | 3.5 | 1.9 | 2.6 |
| **Control** | **Adipose** | **Mean** | **FFA** | **2.7** | **16.7** | **4.6** | **7.8** | **35.8** | **1.8** | **100.0** | **28.6** | **6.5** | **36.9** |
| Control | Adipose | SD | FFA | 1.3 | 5.0 | 0.9 | 2.3 | 9.4 | 0.3 | 0.0 | 10.0 | 1.1 | 8.8 |
| **Treatment** | **Adipose** | **Mean** | **FFA** | **2.6** | **21.7** | **9.2** | **6.8** | **30.7** | **2.8** | **100.0** | **31.8** | **12.0** | **31.5** |
| Treatment | Adipose | SD | FFA | 0.8 | 3.4 | 2.6 | 5.2 | 8.0 | 0.4 | 0.0 | 4.7 | 2.9 | 7.9 |
| **Control** | **Adipose** | **Mean** | **PL** | **1.1** | **13.3** | **2.0** | **22.2** | **13.,1** | **1.1** | **100.0** | **38.6** | **3.1** | **13.9** |
| Control | Adipose | SD | PL | 1.9 | 6.2 | 1.5 | 4.1 | 1.7 | 0.3 | 0.0 | 6.8 | 1.3 | 2.4 |
| **Treatment** | **Adipose** | **Mean** | **PL** | **1.4** | **19.9** | **5.5** | **16.1** | **14.6** | **1.9** | **100.0** | **39.0** | **7.4** | **15.3** |
| Treatment | Adipose | SD | PL | 0.8 | 1.2 | 4.0 | 5.5 | 8.7 | 0.4 | 0.0 | 6.0 | 4.5 | 8.5 |
| **Control** | **Adipose** | **Mean** | **TAG** | **2.6** | **25.1** | **3.6** | **7.3** | **32.7** | **1.6** | **100.0** | **36.1** | **5.3** | **33.2** |
| Control | Adipose | SD | TAG | 0.2 | 1.3 | 1.2 | 2.2 | 2.1 | 0.2 | 0.0 | 3.9 | 1.3 | 2.0 |
| **Treatment** | **Adipose** | **Mean** | **TAG** | **3.3** | **26.9** | **10.8** | **2.9** | **31.9** | **2.0** | **100.0** | **33.3** | **12.9** | **32.2** |
| Treatment | Adipose | SD | TAG | 0.6 | 1.0 | 1.3 | 0.5 | 3.2 | 0.9 | 0.0 | 1.2 | 1.8 | 3.2 |
| **Control** | **Heart** | **Mean** | **CE** | **4.2** | **12.9** | **3.1** | **6.5** | **19.9** | **0.9** | **100.0** | **27.0** | **4.0** | **21.1** |
| Control | Heart | SD | CE | 1.1 | 2.9 | 0.4 | 3.0 | 8.7 | 0.5 | 0.0 | 7.3 | 0.8 | 8.9 |
| **Treatment** | **Heart** | **Mean** | **CE** | **5.1** | **15.4** | **2.9** | **7.0** | **30.1** | **1.5** | **100.0** | **30.3** | **4.4** | **31.5** |
| Treatment | Heart | SD | CE | 1.0 | 1.3 | 0.1 | 0.9 | 7.1 | 0.5 | 0.0 | 3.2 | 0.6 | 6.9 |
| **Control** | **Heart** | **Mean** | **CL** | **0.4** | **2.2** | **2.1** | **4.9** | **7.9** | **3.8** | **25.0** | **8.0** | **5.9** | **8.5** |
| Control | Heart | SD | CL | 0.3 | 1.2 | 0.2 | 3.6 | 1.0 | 0.4 | 0.0 | 4.3 | 0.5 | 1.2 |
| **Treatment** | **Heart** | **Mean** | **CL** | **0.6** | **2.0** | **1.7** | **3.3** | **8.0** | **3.2** | **25.0** | **6.5** | **4.9** | **8.5** |
| Treatment | Heart | SD | CL | 0.3 | 1.0 | 0.2 | 1.7 | 1.7 | 0.4 | 0.0 | 2.2 | 0.6 | 1.6 |
| **Control** | **Heart** | **Mean** | **FFA** | **2.0** | **20.4** | **2.5** | **8.0** | **27.9** | **3.0** | **100.0** | **31.1** | **5.5** | **29.2** |
| Control | Heart | SD | FFA | 0.2 | 2.6 | 0.1 | 1.4 | 1.3 | 0.2 | 0.0 | 3.9 | 0.2 | 1.1 |
| **Treatment** | **Heart** | **Mean** | **FFA** | **2.9** | **17.8** | **2.3** | **7.4** | **29.9** | **2.2** | **100.0** | **29.2** | **4.5** | **30.8** |
| Treatment | Heart | SD | FFA | 1.0 | 4.5 | 1.2 | 1.9 | 12.0 | 0.5 | 0.0 | 5.7 | 1.6 | 11.9 |
| **Control** | **Heart** | **Mean** | **PC** | **0.2** | **28.8** | **0.6** | **14.6** | **8.9** | **2.9** | **50.0** | **43.9** | **3.6** | **9.2** |
| Control | Heart | SD | PC | 0.0 | 1.0 | 0.1 | 0.6 | 0.5 | 0.2 | 0.0 | 0.6 | 0.3 | 0.5 |
| **Treatment** | **Heart** | **Mean** | **PC** | **0.3** | **26.9** | **0.4** | **17.4** | **6.6** | **1.9** | **50.0** | **45.0** | **2.2** | **6.8** |
| Treatment | Heart | SD | PC | 0.0 | 0.7 | 0.1 | 0.3 | 0.8 | 0.2 | 0.0 | 0.5 | 0.2 | 0.8 |
| **Control** | **Heart** | **Mean** | **PE** | **0.1** | **10.0** | **0.5** | **23.5** | **5.7** | **2.2** | **50.0** | **33.9** | **2.7** | **5.9** |
| Control | Heart | SD | PE | 0.0 | 1.6 | 0.1 | 1.8 | 0.4 | 0.2 | 0.0 | 2.7 | 0.2 | 0.4 |
| **Treatment** | **Heart** | **Mean** | **PE** | **0.1** | **9.3** | **0.3** | **26.7** | **4.8** | **1.3** | **50.0** | **36.5** | **1.6** | **5.0** |
| Treatment | Heart | SD | PE | 0.1 | 1.0 | 0.0 | 2.6 | 0.6 | 0.1 | 0.0 | 3.5 | 0.2 | 0.6 |
| **Control** | **Heart** | **Mean** | **PL** | **0.2** | **16.3** | **0.7** | **17.7** | **7.6** | **2.7** | **50.0** | **34.9** | **3.4** | **8.0** |
| Control | Heart | SD | PL | 0.0 | 0.1 | 0.1 | 0.2 | 0.4 | 0.1 | 0.0 | 0.2 | 0.2 | 0.4 |
| **Treatment** | **Heart** | **Mean** | **PL** | **0.3** | **16.0** | **0.5** | **19.8** | **6.1** | **1.8** | **50.0** | **37.0** | **2.3** | **6.5** |
| Treatment | Heart | SD | PL | 0.0 | 0.7 | 0.1 | 0.5 | 0.8 | 0.2 | 0.0 | 1.2 | 0.2 | 0.9 |
| **Control** | **Heart** | **Mean** | **PS/I** | **0.3** | **4.6** | **0.5** | **30.9** | **5.6** | **1.1** | **50.0** | **36.4** | **1.6** | **6.2** |
| Control | Heart | SD | PS/I | 0.1 | 1.4 | 0.2 | 1.9 | 1.4 | 0.1 | 0.0 | 2.4 | 0.3 | 1.4 |
| **Treatment** | **Heart** | **Mean** | **PS/I** | **0.4** | **3.5** | **0.6** | **33.1** | **6.7** | **0.9** | **50.0** | **38.0** | **1.4** | **7.1** |
| Treatment | Heart | SD | PS/I | 0.1 | 0.2 | 0.1 | 2.1 | 1.5 | 0.1 | 0.0 | 2.2 | 0.2 | 1.6 |
| **Control** | **Heart** | **Mean** | **TAG** | **3.3** | **35.7** | **4.1** | **6.5** | **24.2** | **2.6** | **33.3** | **47.6** | **6.7** | **25.1** |
| Control | Heart | SD | TAG | 0.5 | 1.4 | 0.7 | 0.8 | 1.3 | 0.3 | 0.0 | 1.3 | 0.5 | 1.5 |
| **Treatment** | **Heart** | **Mean** | **TAG** | **4.1** | **34.2** | **2.9** | **10.1** | **21.5** | **1.5** | **33.3** | **51.9** | **4.4** | **22.9** |
| Treatment | Heart | SD | TAG | 0.4 | 3.0 | 1.0 | 2.2 | 1.4 | 0.2 | 0.0 | 5.2 | 0.9 | 2.2 |
| **Control** | **Liver** | **Mean** | **CE** | **2.2** | **24.7** | **7.9** | **4.1** | **35.5** | **2.1** | **100.0** | **33.0** | **10.0** | **36.9** |
| Control | Liver | SD | CE | 0.5 | 14.1 | 0.7 | 1.5 | 13.7 | 0.2 | 0.0 | 14.9 | 0.8 | 14.0 |
| **Treatment** | **Liver** | **Mean** | **CE** | **2.5** | **27.3** | **4.9** | **7.1** | **25.4** | **1.8** | **100.0** | **39.0** | **6.7** | **26.9** |
| Treatment | Liver | SD | CE | 0.5 | 12.0 | 1.8 | 2.2 | 18.0 | 0.3 | 0.0 | 14.2 | 1.8 | 16.9 |
| **Control** | **Liver** | **Mean** | **CL** | **0.4** | **5.6** | **3.5** | **4.7** | **10.5** | **5.6** | **25.0** | **11.5** | **9.1** | **11.4** |
| Control | Liver | SD | CL | 0.6 | 0.9 | 2.8 | 2.2 | 1.4 | 0.4 | 0.0 | 3.2 | 2.8 | 1.1 |
| **Treatment** | **Liver** | **Mean** | **CL** | **0.4** | **4.9** | **1.5** | **4.7** | **8.7** | **4.4** | **25.0** | **10.4** | **5.9** | **9.4** |
| Treatment | Liver | SD | CL | 0.7 | 1.9 | 1.7 | 2.6 | 1.3 | 0.8 | 0.0 | 4.8 | 1.3 | 1.1 |
| **Control** | **Liver** | **Mean** | **FFA** | **1.6** | **23.0** | **7.4** | **3.9** | **26.1** | **4.4** | **100.0** | **28.9** | **11.9** | **26.8** |
| Control | Liver | SD | FFA | 0.5 | 1.8 | 0.4 | 0.3 | 3.0 | 0.4 | 0.0 | 1.7 | 0.7 | 3.0 |
| **Treatment** | **Liver** | **Mean** | **FFA** | **1.6** | **24.0** | **3.9** | **4.9** | **23.7** | **2.8** | **100.0** | **31.2** | **6.7** | **24.2** |
| Treatment | Liver | SD | FFA | 0.3 | 1.4 | 0.5 | 0.7 | 2.7 | 0.5 | 0.0 | 2.2 | 0.9 | 2.8 |
| **Control** | **Liver** | **Mean** | **PC** | **0.2** | **32.0** | **1.7** | **12.5** | **10.7** | **2.6** | **50.0** | **45.1** | **4.3** | **11.0** |
| Control | Liver | SD | PC | 0.0 | 1.8 | 0.1 | 1.3 | 0.6 | 0.2 | 0.0 | 0.5 | 0.2 | 0.6 |
| **Treatment** | **Liver** | **Mean** | **PC** | **0.2** | **29.4** | **0.9** | **15.5** | **10.1** | **1.9** | **50.0** | **45.4** | **2.9** | **10.4** |
| Treatment | Liver | SD | PC | 0.0 | 0.9 | 0.2 | 1.8 | 0.7 | 0.5 | 0.0 | 1.1 | 0.7 | 0.7 |
| **Control** | **Liver** | **Mean** | **PE** | **0.1** | **22.8** | **1.1** | **17.6** | **6.6** | **1.8** | **50.0** | **40.8** | **2.8** | **7.0** |
| Control | Liver | SD | PE | 0.0 | 1.4 | 0.1 | 0.8 | 0.6 | 0.1 | 0.0 | 0.7 | 0.2 | 0.6 |
| **Treatment** | **Liver** | **Mean** | **PE** | **0.1** | **22.2** | **0.6** | **19.1** | **6.6** | **1.2** | **50.0** | **41.5** | **1.8** | **6.9** |
| Treatment | Liver | SD | PE | 0.0 | 1.3 | 0.1 | 2.0 | 0.8 | 0.3 | 0.0 | 1.2 | 0.5 | 0.9 |
| **Control** | **Liver** | **Mean** | **PL** | **0.1** | **25.4** | **1.5** | **15.4** | **8.8** | **2.4** | **50.0** | **41.7** | **3.9** | **9.5** |
| Control | Liver | SD | PL | 0.0 | 1.5 | 0.0 | 0.9 | 0.2 | 0.2 | 0.0 | 0.9 | 0.2 | 0.2 |
| **Treatment** | **Liver** | **Mean** | **PL** | **0.1** | **23.2** | **0.8** | **17.9** | **8.4** | **1.8** | **50.0** | **42.1** | **2.6** | **9.0** |
| Treatment | Liver | SD | PL | 0.0 | 0.5 | 0.2 | 1.7 | 0.7 | 0.4 | 0.0 | 1.1 | 0.6 | 0.8 |
| **Control** | **Liver** | **Mean** | **PS/I** | **0.2** | **9.9** | **0.6** | **36.7** | **4.9** | **1.3** | **50.0** | **47.5** | **1.8** | **6.1** |
| Control | Liver | SD | PS/I | 0.0 | 0.9 | 0.1 | 1.0 | 0.8 | 0.2 | 0.0 | 1.0 | 0.2 | 1.1 |
| **Treatment** | **Liver** | **Mean** | **PS/I** | **0.2** | **8.0** | **0.3** | **38.4** | **4.3** | **0.9** | **50.0** | **47.0** | **1.2** | **5.4** |
| Treatment | Liver | SD | PS/I | 0.0 | 0.4 | 0.0 | 1.1 | 0.3 | 0.1 | 0.0 | 0.9 | 0.1 | 0.5 |
| **Control** | **Liver** | **Mean** | **TAG** | **0.9** | **35.8** | **5.4** | **1.7** | **36.2** | **3.8** | **33.3** | **39.1** | **9.2** | **37.2** |
| Control | Liver | SD | TAG | 0.2 | 0.4 | 0.5 | 0.2 | 2.9 | 0.3. | 0.0 | 0.7 | 0.5 | 2.9 |
| **Treatment** | **Liver** | **Mean** | **TAG** | **1.1** | **32.1** | **3.2** | **3.3** | **34.1** | **2.9** | **33.3** | **37.1** | **6.1** | **35.0** |
| Treatment | Liver | SD | TAG | 0.2 | 1.5 | 0.4 | 1.2 | 2.3 | 0.4 | 0.0 | 1.3 | 0.6 | 2.4 |
| **Control** | **Plasma** | **Mean** | **CE** | **0.6** | **3.9** | **6.2** | **0.3** | **7.1** | **0.6** | **100.0** | **5.2** | **6.8** | **7.4** |
| Control | Plasma | SD | CE | 0.1 | 0.4 | 0.5 | 0.1 | 0.7 | 0.1 | 0.0 | 0.6 | 0.5 | 0.7 |
| **Treatment** | **Plasma** | **Mean** | **CE** | **0.8** | **4.5** | **3.2** | **0.7** | **7.5** | **0.5** | **100.0** | **6.4** | **3.7** | **7.7** |
| Treatment | Plasma | SD | CE | 0.1 | 0.4 | 0.8 | 0.3 | 0.8 | 0.1 | 0.0 | 0.8 | 0.8 | 0.8 |
| C**ontrol** | **Plasma** | **Mean** | **FFA** | **5.0** | **28.1** | **9.1** | **6.6** | **27.3** | **2.3** | **100.0** | **41.2** | **11.4** | **27.9** |
| Control | Plasma | SD | FFA | 0.9 | 7.3 | 1.8 | 2.2 | 14.0 | 0.6 | 0.0 | 9.7 | 1.4 | 14.1 |
| **Treatment** | **Plasma** | **Mean** | **FFA** | **5.7** | **28.1** | **3.2** | **10.0** | **31.0** | **2.1** | **100.0** | **45.4** | **5.3** | **31.5** |
| Treatment | Plasma | SD | FFA | 1.9 | 11.5 | 1.0 | 3.6 | 19.9 | 0.7 | 0.0 | 17.0 | 1.7 | 20.0 |
| **Control** | **Plasma** | **Mean** | **LY** | **0.5** | **41.5** | **1.3** | **19.3** | **8.8** | **1.6** | **100.0** | **62.7** | **2.8** | **9.2** |
| Control | Plasma | SD | LY | 0.2 | 5.7 | 0.3 | 1.3 | 0.9 | 0.1 | 0.0 | 4.1 | 0.3 | 1.0 |
| **Treatment** | **Plasma** | **Mean** | **LY** | **0.5** | **37.8** | **0.7** | **20.1** | **7.2** | **1.0** | **100.0** | **60.0** | **1.8** | **7.5** |
| Treatment | Plasma | SD | LY | 0.2 | 5.0 | 0.2 | 2.0 | 1.1 | 0.2 | 0.0 | 4.9 | 0.4 | 1.2 |
| **Control** | **Plasma** | **Mean** | **PC** | **0.1** | **31.6** | **0.8** | **14.8** | **9.4** | **2.1** | **50.0** | **47.1** | **2.9** | **9.8** |
| Control | Plasma | SD | PC | 0.0 | 1.1 | 0.0 | 1.2 | 0.6 | 0.2 | 0.0 | 0.4 | 0.2 | 0.6 |
| **Treatment** | **Plasma** | **Mean** | **PC** | **0.1** | **29.0** | **0.4** | **17.5** | **7.9** | **1.5** | **50.0** | **47.2** | **1.9** | **8.2** |
| Treatment | Plasma | SD | PC | 0.0 | 0.8 | 0.1 | 0.9 | 1.0 | 0.3 | 0.0 | 0.4 | 0.4 | 1.0 |
| **Control** | **Plasma** | **Mean** | **PE** | **1.0** | **19.8** | **1.4** | **15.8** | **12.0** | **1.6** | **50.0** | **39.0** | **3.0** | **13.3** |
| Control | Plasma | SD | PE | 0.5 | 1.4 | 0.7 | 2.0 | 8.5 | 0.2 | 0.0 | 3.2 | 0.9 | 8.4 |
| **Treatment** | **Plasma** | **Mean** | **PE** | **1.9** | **16.1** | **1.4** | **13.5** | **13.1** | **1.1** | **50.0** | **35.3** | **2.5** | **14.1** |
| Treatment | Plasma | SD | PE | 0.4 | 1.8 | 1.0 | 1.7 | 10.2 | 0.4 | 0.0 | 3.8 | 1.3 | 10.1 |
| **Control** | **Plasma** | **Mean** | **PL** | **0.1** | **29.2** | **0.8** | **15.4** | **9.0** | **1.9** | **50.0** | **45.7** | **2.7** | **9.8** |
| Control | Plasma | SD | PL | 0.0 | 1.4 | 0.0 | 1.1 | 0.3 | 0.2 | 0.0 | 0.7 | 0.2 | 0.4 |
| **Treatment** | **Plasma** | **Mean** | **PL** | **0.2** | **26.6** | **0.4** | **17.3** | **7.7** | **1.4** | **50.0** | **45.1** | **1.8** | **8.7** |
| Treatment | Plasma | SD | PL | 0.0 | 1.0 | 0.1 | 1.0 | 0.5 | 0.3 | 0.0 | 0.4 | 0.4 | 0.6 |
| **Control** | **Plasma** | **Mean** | **TAG** | **1.1** | **24.7** | **7.0** | **1.7** | **30.4** | **3.8** | **33.3** | **28.0** | **10.8** | **31.3** |
| Control | Plasma | SD | TAG | 0.1 | 0.3 | 0.8 | 0.2 | 2.0 | 0.4 | 0.0 | 0.5 | 1.0 | 2.1 |
| **Treatment** | **Plasma** | **Mean** | **TAG** | **1.7** | **21.1** | **3.2** | **4.4** | **25.8** | **2.3** | **33.3** | **28.5** | **5.4** | **26.6** |
| Treatment | Plasma | SD | TAG | 0.2 | 1.1 | 0.6 | 1.9 | 3.1 | 0.5 | 0.0 | 2.9 | 1.1 | 3.2 |

### Results:

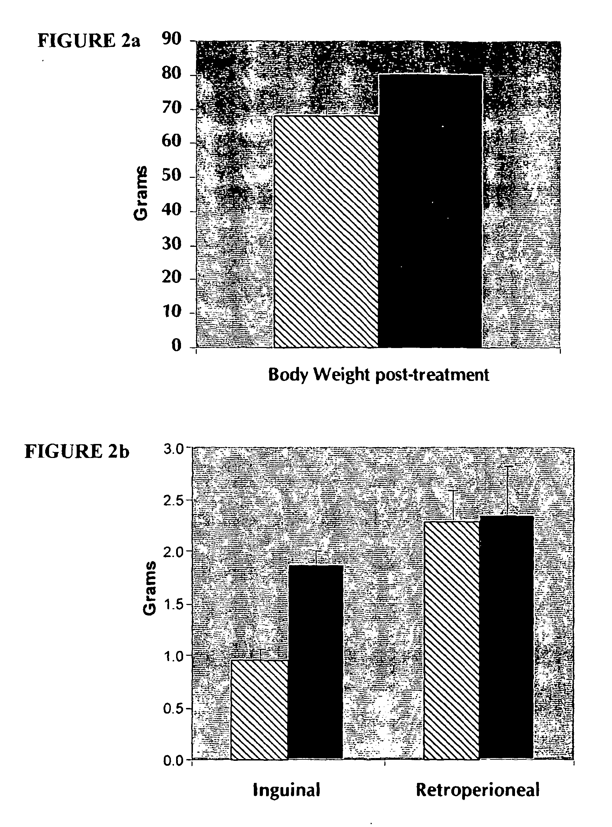

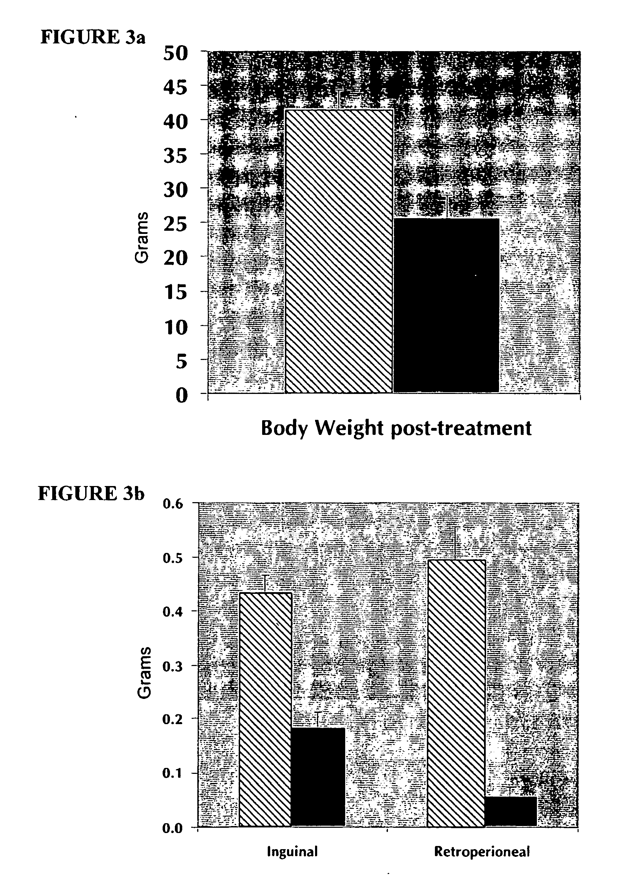

Tables 5 and 6 show the concentrations of lipid metabolites present in adipose, plasma, heart, and liver tissues in mice treated with rosiglitazone and their controls. Tables 7 and 8 show the concentrations of lipid metabolites present in adipose, plasma, heart and liver tissues in mice treated with CL316,243 and their controls. In Tables 5 and 7, the data are expressed as nanomoles per gram of tissue or plasma. In tables 6 and 8, the data are expressed as a percentage of total fatty acids within each lipid class. Figures 2 and 3 show the body (FIGS 2a and 3a) and adipose tissue (FIGS 2b and 3b) weight gain associated with rosiglitazone and CL316,243 treatments, respectively.

It is clear that the rosiglitazone induced a substantial increase in palmitoleic acid concentrations in most lipid classes in heart, liver, adipose, and plasma (Table 5). Additionally, the vaccenic and myristic acid concentrations in these lipid classes were typically increased. Corresponding to the increased palmitoleic acid, and thus increased *de novo* fatty acid synthesis, was a significant increase in total body (FIG 2a) and adipose tissue (FIG 2b) weight with rosiglitazone treatment. Importantly, even though total cholesterol esters and phosphatidylcholine were decreased from the plasma of rosiglitazone-treated mice, the concentration of palmitoleic acid in cholesterol ester and phosphatidylcholine increased both quantitatively (Table 5) and in relational terms (Table 6). This indicates that plasma palmitoleic acid is a useful marker of tissue *de novo* fatty acid synthesis independent of triacylglyceride synthesis. Additionally, the concentration of palmitoleic acid in plasma free fatty acids was reflective of an increased concentration of palmitoleic acid within adipose triacylglycerides. The concentration of plasma palmitoleic acid within cholesterol esters, triacylglycerides and phospholipids therefore serves as a plasma diagnostic for hepatic *de novo* fatty acid synthesis and overall weight gain. The concentration of plasma palmitoleic acid within free fatty acids therefore serves as a plasma diagnostic for adipose *de novo* fatty acid synthesis, adipose mass accumulation, and weight gain. Additionally, plasma and tissue palmitoleic acid concentrations may be used as a marker or as part of a profile of markers identifying the actions or activity of PPARγ agonists including thiazolidinediones. In each of these applications, vaccenic acid (18:1n7), oleic acid (18:1n9), palmitic acid (16:0) and myristic acid (14:0) also proved to be valuable, but slightly less consistent markers.

The mole percentage data supports these findings, as the palmitoleic, vaccenic, palmitic, myristic and oleic acids were increased with rosiglitazone treatment relative to other fatty acids in each lipid class in heart, liver and plasma (Table 6). This indicates an increased contribution of these fatty acids to the global lipid metabolite pool. Thus, the mole percentage data was capable of determining the increased de novo fatty acid synthesis and predicting weight gain in rosiglitazone-treated mice.

Data supporting the application of these markers to humans comes from clinical studies that find that patients taking rosiglitazone gain weight (Fuchtenbebusch et al., Exp. Clin. Endocrinol. Diabetes 108:1551-163, 2000).

Treatment of mice with CL316,243 induced a substantial decrease in palmitoleic acid concentrations in most lipid classes in heart, liver, adipose and plasma (Table 7 and Table 8). Additionally, the vaccenic acid concentrations in these lipid classes were typically decreased. Corresponding to the decreased palmitoleic acid, and thus decreased de novo fatty acid synthesis was a significant decrease in mouse body (FIG 3a) and adipose (FIG 3b) tissue weight with CL316,243 treatment. In mice treated with CL316,243, the decreased lipid concentrations in plasma were partially the result of decreased lipid synthesis in tissues, which can be observed in Table 7. This indicates again that plasma palmitoleic acid is a useful marker of tissue *de novo* fatty acid synthesis. The concentration of plasma palmitoleic acid within cholesterol esters, triacylglycerides, and phospholipids therefore serves as a plasma diagnostic for hepatic *de novo* fatty acid synthesis and overall weight gain. The concentration of plasma palmitoleic acid within free fatty acids therefore serves as a plasma diagnostic for adipose *de novo* fatty acid synthesis, adipose mass accumulation, and weight gain. Additionally, plasma and tissue palmitoleic acid concentrations may be used as a marker or as part of a profile of markers identifying the actions or activity of β3-adrenergic agonists including CL316,243.

### Example 2: Identification of Therapeutic Compounds

The linkage of specific lipid metabolites to *de novo* fatty acid synthesis and related conditions as disclosed herein can be used to identify compounds that are useful in treating, reducing, or preventing such conditions, including for instance diabetes, weight gain, weight loss (*e*.*g*., wasting), obesity, hypo- and hyper-thyroidism, menopause, immuno-tolerance, auto-immunity, aging, and/or cardiovascular disease. These marker molecules can be used alone or in combination, for instance in sets of two or more that are linked to a particular condition, for instance in a condition-related profile or fingerprint. Specific provided marker molecules include the following lipid metabolites, assessed either as free fatty acids or as components of a lipid class (as discussed throughout): palmitoleic acid (16:1n7), vaccenic acid (18:1n7), palmitic acid (16:0), stearic acid (18:0), oleic acid (18:1n9), all n7 fatty acids, all n9 fatty acids, all saturated fatty acids, or a combination of two or more thereof, and including ratios between specific molecules or molecule categories as provided herein (such as palmitoleic to palmitic, n7 to saturated, n7 to n9, and oleic to stearic). In particular examples, these lipid metabolites are measured from liver, plasma, adipose, heart, or other biological samples, and are specifically measured in one or more of the following lipid classes: triacylglycerides, free fatty acids, cholesterol esters, or diacylglycerides.

By way of example, a test compound is applied to a cell, for instance a test cell, and at least one *de novo* fatty acid synthesis marker level in the cell is measured and compared to the equivalent measurement from a test cell (or from the same cell prior to application of the test compound). If application of the compound alters the level of marker molecule (for instance by increasing or decreasing that level), or changes an entire *de novo* fatty acid synthesis profile to be more like a profile that is indicative of a condition, then that compound is selected as a likely candidate for further characterization. In particular examples, a test agent that opposes or inhibits a *de novo* fatty acid synthesis-related change is selected for further study, for example by exposing the agent to a cell *in vitro*, or to an animal such as an animal model, to determine whether *de novo* fatty acid synthesis is inhibited, and/or whether weight gain is prevented or reversed. Such identified compounds may be useful in treating, reducing, or preventing weight gain or development or progression of obesity or another related, weight-gain attendant condition.

Methods for identifying such compounds optionally can include the generation of a *de novo* fatty acid synthesis-related lipid metabolomic profile, as described herein. Example control profiles useful for comparison in such methods may be constructed from normal biological samples such as those taken from a cell not contacted with the test agent, or an animal not subjected to the condition or intervention being tested.

### Example 3: Profiles (Fingerprints)

With the provision herein of specific molecules the levels of which, or proportional levels of which, are linked to *de novo* fatty acid synthesis, profiles that provide information on the *de novo* fatty acid synthesis-state of a subject are now enabled.

*De novo* fatty acid synthesis-related profiles comprise the distinct and identifiable pattern of (or level) of sets of lipid metabolites, for instance a pattern of high and low levels of a defined set of fatty acids and/or fatty acids in particular lipid classes. The set of molecules in a particular profile will usually include at least one of the following: palmitoleic acid, vaccenic acid, palmitic acid, stearic acid, oleic acid, all n7 fatty acids, all n9 fatty acids, or all saturated fatty acids. Particular profiles include ratios between specific metabolites or metabolite categories as provided herein (such as palmitoleic to palmitic, n7 to saturated, n7 to n9, and oleic to stearic). In particular examples, the lipid metabolites included in a profile are measured from liver, plasma, adipose, heart, or other biological samples, and are specifically measured in one or more of the following lipid classes: triacylglycerides, free fatty acids, cholesterol esters, or diacylglycerides.

Specific profiles may be for a particular disease (*e*.*g*., diabetes, obesity, cardiovascular disease, and so forth), a specific condition (*e*.*g*., menopause), treatment regimen (*e*.*g*., treatment with a known drug or agent, toxin exposure, and so forth), growth or disease progression (*e*.*g*., increase in weight, decrease in weight, wasting, etc), or other categories. Thus, representative profiles that are linked to *de novo* fatty acid synthesis can be established for a biological sample taken from a lean individual (*i*.*e*., normal as regards weight), and a biological taken from an overweight or obese individual, or paired subjects one of which is taking a certain drug or other therapeutic intervention, and so forth. Each of these profiles includes information on the level of at least one, but usually two or more, lipid metabolites that are linked to *de novo* fatty acid synthesis (*e*.*g*., positively, such that the lipid metabolite level is high in those samples where *de novo* fatty acid synthesis is high, or negatively, such that the lipid metabolite level is low in those samples where the *de novo* fatty acid synthesis is high). Information provided in a profile can include relative as well as absolute levels of specific metabolites. Results from the *de novo* fatty acid synthesis profiles of an individual are often viewed in the context of a test sample compared to a baseline or control sample profile, or in comparison to a database of profiles from other individuals of the same or different species.

The levels of lipid metabolites that make up a profile can be measured in any of various known ways, which may be specific for the type of molecule being measured, including specific methods provided herein. Many ways to collect quantitative or relational data on lipid metabolites are known to those of ordinary skill in the art, and the analytical methodology does not affect the utility of metabolite concentrations in predicting phenotype or assessing metabolism. One method described herein for generating quantitative and mole percentage data on fatty acids in lipid classes involves gas chromatography coupled with flame ionization detection. Other methods for generating data on lipid metabolites include but are not limited to high-performance liquid chromatography, mass spectrometry, capillary electrophoresis, thin layer chromatography, immunoassay, RNA switches, nuclear magnetic resonance, etc.

Optionally, a subject's *de novo* fatty acid synthesis profile can be correlated with one or more appropriate treatments, which may be correlated with a control (or set of control) profile(s) for a disease or condition linked to or associated with *de novo* fatty acid synthesis, for instance.

It will be apparent that the precise details of the methods described may be varied or modified without departing from the described invention. We claim all such modifications and variations that fall within the scope of the claims below.

## Claims

1. A method of assessing *de novo* fatty acid synthesis in a cell, an organism or a tissue of an organism, comprising quantifying a marker of *de novo* fatty acid synthesis in a biological sample from the organism, wherein the marker of *de novo* fatty acid synthesis - comprises palmitoleic acid, vaccenic acid, palmitic acid, stearic acid, oleic acid, myristic acid, n7 fatty acids, n9 fatty acids, all saturated fatty acids, or a combination of any two or more of these, wherein the marker of *de novo* fatty acid synthesis is measured in a specific lipid category, and wherein the organism is not a rosiglitazone-treated mouse.

2. The method of claim 1, wherein the lipid category is triacylglycerides, cholesterol esters, or free fatty acids.

3. The method of claim 1, wherein the method is a method of assessing *de novo* fatty acid synthesis in a cell, and the cell is a cultured cell; in an organism; in a tissue of an organism; or in adipose tissue, liver tissue or muscle tissue.

4. The method of claim 3, wherein the organism is a research animal, a companion animal, or a human.

5. The method of claim 1, wherein the biological sample is a liver sample, a plasma sample, an adipose sample, a heart sample, blood or a blood product.

6. The method of claim 5, wherein the biological sample is blood or a blood product and the marker of *de novo* fatty acid synthesis is quantified from the free fatty acid fraction of the blood or blood product and the method is a method to assess *de novo* fatty acid synthesis in adipose tissue.

7. The method of claim 5, wherein the biological sample is blood or a blood product and the marker of *de novo* fatty acid synthesis is quantified from the phosphatidylcholine, triacylglyceride, or cholesterol ester fraction of the blood or blood product, and the method is a method to assess *de novo* fatty acid synthesis in liver tissue.

8. The method of claim 7, wherein the marker of *de novo* fatty acid synthesis is quantified from the cholesterol ester fraction of the blood or blood product.

9. The method of claim 1, comprising quantifying palmitoleic acid and palmitic acid in a biological sample from the organism.

10. The method of claim 9, further comprising generating a ratio indicator of *de novo* fatty acid synthesis, wherein the ratio indicator is the ratio of the quantity of palmitoleic acid to the quantity of palmitic acid.

11. The method of claim 10, further comprising comparing the ratio indicator from the biological sample with a ratio indicator from a baseline or control sample.

12. The method of claim 1, comprising quantifying total n7 fatty acids and total saturated fatty acids in a biological sample from the organism.

13. The method of claim 12, further comprising generating a ratio indicator of *de novo* fatty acid synthesis, wherein the ratio indicator is the ratio of the quantity of total n7 fatty acids to the quantity of total saturated fatty acids.

14. The method of claim 13, further comprising comparing the ratio indicator from the biological sample with a ratio indicator from a baseline or control sample.

15. The method of claim 1, comprising quantifying total n7 fatty acids and total n9 fatty acids in a biological sample from the organism.

16. The method of claim 15, further comprising generating a ratio indicator of *de novo* fatty acid synthesis, wherein the ratio indicator is the ratio of the quantity of total n7 fatty acids to the quantity of total n9 fatty acids.

17. The method of claim 16, further comprising comparing the ratio indicator from the biological sample with a ratio indicator from a baseline or control sample.

18. The method of claim 1, wherein the method is
(1) a method to determine if a pharmaceutical, nutritional, genetic, toxicological or environmental treatment, regimen or dosage influences *de novo* fatty acid synthesis;
(2) a method to assess a therapeutic or pharmaceutical agent for its potential effectiveness, efficacy or side effects relating to *de novo* fatty acid synthesis; or
(3) a method to screen individuals for compatibility or incompatibility with a pharmaceutical, nutritional, toxicological or environmental treatment.

19. The method of claim 1, comprising quantifying palmitoleic acid in a biological sample from the organism, preferably the biological sample is blood or a blood product.

20. The method of claim 1, comprising quantifying stearic acid and palmitic acid in a biological sample from the organism.

21. The method of claim 20, further comprising generating a ratio indicator of *de novo* fatty acid synthesis, wherein the ratio indicator is the ratio of the quantity of stearic acid to the quantity of palmitic acid.

22. The method of claim 1, wherein the method is a method of assessing a change in the *de novo* fatty acid synthesis in the organism, and wherein the method comprises taking at least two biological samples from the organism, wherein the two samples are taken before and after an event.

23. The method of claim 22, wherein the event comprises passage of time, treatment with a therapeutic agent, treatment with a pharmaceutical agent, treatment with a nutritional regimen, treatment with a genetic modification, exposure to a toxic or potentially toxic compound, exposure to an environmental condition, treatment with a laboratory procedure, exercise, or the appearance of a phenotypic state.

24. The method of claim 1, wherein the quantity of the marker of *de novo* fatty acid synthesis is correlated to a propensity, risk, or metabolic basis for weight gain or loss and/or obesity and/or diabetes and/or cardiovascular disease and/or hormonal dysregulation of the organism, and the method is a method for determining the propensity, risk, or metabolic basis for weight gain or loss and/or obesity and/or diabetes and/or cardiovascular disease and/or hormonal dysregulation of the organism.

25. The method of claim 24, further comprising correlating the quantity of the marker of *de novo* fatty acid synthesis with *de novo* fatty acid synthesis in adipose tissue, wherein the marker of *de novo* fatty acid synthesis is quantified from the free fatty acid fraction of blood or a blood product.

26. The method of claim 25, further comprising correlating the quantity of the marker of *de novo* fatty acid synthesis with *de novo* fatty acid synthesis in the liver, wherein the marker of *de novo* fatty acid synthesis is quantified from the phosphatidylcholine, triacylglyceride, or cholesterol ester fraction of blood or a blood product.

27. The method of claim 24, which is a method of determining whether a treatment or intervention will cause weight gain or loss and/or obesity and/or influence cardiovascular disease and/or cause hormonal dysregulation, further comprising taking at least two biological samples from the organism, wherein the two samples are taken before and after a nutritional, pharmacological, genetic, environmental or toxicological treatment or intervention, and wherein a change in the quantity of the marker of *de novo* fatty acid synthesis is correlated with a likelihood of weight gain or loss and/or obesity and/or a change in diabetic state of an organism and/or influencing cardiovascular disease.

28. The method of claim 24, further comprising comparing the assessment of *de novo* fatty acid synthesis from the organism to an assessment of *de novo* fatty acid synthesis from another organism or compiled for a population of organisms.

29. The method of claim 24, wherein the quantity of the marker of *de novo* fatty acid synthesis is reported as an absolute or relative concentration.

30. The method of claim 29, wherein correlating the quantity of the marker of *de novo* fatty acid synthesis comprises using the absolute or relative concentration of the marker of *de novo* fatty acid synthesis in a mathematical or statistical equation for determining the amount of *de novo* fatty acid synthesis.

31. The method of claim 1, wherein the method is a method of assessing an activity of at least one enzyme involved in *de novo* fatty acid synthesis, further comprising correlating the quantity of the marker with the activity of the at least one enzyme.

32. The method of claim 31, wherein the at least one enzyme involved in *de novo* fatty acid synthesis is fatty acid synthase.

33. The method of any one of claims 1 through 32, further comprising generating a printed report.

## Patentansprüche

1. Verfahren zum Bewerten der *de novo* Fettsäuresynthese in einer Zelle, einem Organismus oder einem Gewebe eines Organismus, umfassend das Quantifizieren eines Markers der *de novo* Fettsäuresynthese in einer biologischen Probe aus dem Organismus, wobei der Marker der *de novo* Fettsäuresynthese Palmitoleinsäure, Vaccensäure, Palmitinsäure, Stearinsäure, Oleinsäure, Myristinsäure, n7 Fettsäuren, n9 Fettsäuren, vollständig gesättigte Fettsäuren oder eine Kombination von jedweden zwei oder mehreren von diesen umfaßt, wobei der Marker der *de novo* Fettsäurensynthese in einer spezifischen Lipidkategorie gemessen wird und wobei der Organismus nicht eine Rosiglitazon-behandelte Maus ist.

2. Verfahren gemäß Anspruch 1, wobei die Lipidkategorie Triacylglyceride, Cholesterinester oder freie Fettsäuren ist.

3. Verfahren gemäß Anspruch 1, wobei das Verfahren ein Verfahren des Bewertens der *de novo* Fettsäuresynthese in einer Zelle ist und die Zelle eine kultivierte Zelle ist, in einem Organismus, in einem Gewebe eines Organismus oder in einem Fettgewebe, Lebergewebe oder Muskelgewebe.

4. Verfahren gemäß Anspruch 3, wobei der Organismus ein Forschungstier, ein Haustier oder ein Mensch ist.

5. Verfahren gemäß Anspruch 1, wobei die biologische Probe eine Leberprobe, eine Plasmaprobe, eine Fettprobe, eine Herzprobe, Blut oder ein Blutprodukt ist.

6. Verfahren gemäß Anspruch 5, wobei die biologische Probe Blut oder ein Blutprodukt ist und der Marker der *de novo* Fettsäuresynthese aus der freien Fettsäurefraktion des Bluts oder Blutprodukts quantifiziert wird und das Verfahren ein Verfahren zur Berwertung der *de novo* Fettsäuresynthese in Fettgewebe ist.

7. Verfahren gemäß Anspruch 5, wobei die biologische Probe Blut oder ein Blutprodukt ist und der Marker der *de novo* Fettsäuresynthese aus der Phosphatidylcholin-, Triacylglycerid- oder Cholesterinesterfraktion des Bluts oder Blutprodukts quantifiziert wird und das Verfahren ein Verfahren zur Bewertung der *de novo* Fettsäuresynthese in Lebergewebe ist.

8. Verfahren gemäß Anspruch 7, wobei der Marker der *de novo* Fettsäuresynthese aus der Cholesterinesterfraktion des Bluts oder Blutprodukts quantifiziert wird.

9. Verfahren gemäß Anspruch 1, umfassend das Quantifizieren von Palmitoleinsäure und Palmitinsäure in einer biologischen Probe aus dem Organismus.

10. Verfahren gemäß Anspruch 9, weiter umfassend das Generieren eines Verhältnisindikators der *de novo* Fettsäuresynthese, wobei der Verhältnisindikator das Verhältnis der Menge an Palmitoleinsäure zu der Menge an Palmitinsäure ist.

11. Verfahren gemäß Anspruch 10, weiter umfassend das Vergleichen des Verhältnisindikators aus der biologischen Probe mit einem Verhältnisindikator aus einer Basislinie oder Kontrollprobe.

12. Verfahren gemäß Anspruch 1, umfassend das Quantifizieren sämtlicher n7 Fettsäuren und sämtlicher gesättigter Fettsäuren in einer biologischen Probe aus dem Organismus.

13. Verfahren gemäß Anspruch 12, weiter umfassend das Generieren eines Verhältnisindikators der *de novo* Fettsäuresynthese, wobei der Verhältnisindikator das Verhältnis der Menge an sämtlichen n7 Fettsäuren zu der Menge an sämtlichen gesättigten Fettsäuren ist.

14. Verfahren gemäß Anspruch 13, weiter umfassend das Vergleichen des Verhältnisindikators aus der biologischen Probe mit einem Verhältnisindikator aus einer Basislinie oder Kontrollprobe.

15. Verfahren gemäß Anspruch 1, umfassend das Quantifizieren sämtlicher n7 Fettsäuren und sämtlicher n9 Fettsäuren in einer biologischen Probe aus dem Organismus.

16. Verfahren gemäß Anspruch 15, weiter umfassend das Generieren eines Verhältnisindikators der de *novo* Fettsäuresynthese, wobei der Verhältnisindikator das Verhältnis der Menge an sämtlichen n7 Fettsäuren zu der Menge an sämtlichen n9 Fettsäuren ist.

17. Verfahren gemäß Anspruch 16, weiter umfassend das Vergleichen des Verhältnisindikators aus der biologischen Probe mit einem Verhältnisindikator aus einer Basislinie oder Kontrollprobe.

18. Verfahren gemäß Anspruch 1, wobei das Verfahren ist
(1) ein Verfahren zum Bestimmen, ob eine pharmazeutische, Ernährungs-, genetische, toxikologische oder Umweltbehandlung, Verabreichungsart oder Dosierung die *de novo* Fettsäuresynthese beeinflußt;
(2) ein Verfahren zum Untersuchen eines therapeutischen oder pharmazeutischen Mittels auf dessen potentielle Wirksamkeit, Wirkungskraft oder Nebeneffekte bezüglich der *de novo* Fettsäuresynthese oder
(3) ein Verfahren zum Screenen von Einzelpersonen hinsichtlich der Kompatibilität oder Inkompatibilität mit einer pharmazeutischen, Ernährungs-, toxikologischen oder Umweltbehandlung.

19. Verfahren gemäß Anspruch 1, umfassend das Quantifizieren von Palmitoleinsäure in einer biologischen Probe aus dem Organismus, wobei vorzugsweise die biologische Probe Blut oder ein Blutprodukt ist.

20. Verfahren gemäß Anspruch 1, umfassendes das Quantifizieren von Stearinsäure und Palmitinsäure in einer biologischen Probe aus dem Organismus;

21. Verfahren gemäß Anspruch 20, weiter umfassend das Generieren eines Verhältnisindikators der *de novo* Fettsäuresynthese, wobei der Verhältnisindikator das Verhältnis der Menge an Stearinsäure zu der Menge an Palmitinsäure ist.

22. Verfahren gemäß Anspruch 1, wobei das Verfahren ein Verfahren zum Bewerten einer Änderung in der *de novo* Fettsäuresynthese in dem Organismus ist und wobei das Verfahren das Nehmen von mindestens zwei biologischen Proben aus dem Organismus umfaßt, wobei die zwei Proben vor und nach einem Ereignis genommen werden.

23. Verfahren gemäß Anspruch 22, wobei das Ereignis Zeitfortdauer, Behandlung mit einem therapeutischen Mittel, Behandlung mit einem pharmazeutischen Mittel, Behandlung mit einem Ernährungsprogramm, Behandlung mit einer genetischen Modifikation, Aussetzen einer toxischen oder potentiell toxischen Verbindung, Aussetzen einer Umweltbedingung, Behandlung mit einem Laborvorgang, Bewegung oder das Erscheinen eines phänotypischen Zustands umfaßt.

24. Verfahren gemäß Anspruch 1, wobei die Menge des Markers der *de novo* Fettsäuresynthese zu einer Neigung, Risiko oder metabolischen Basis für Gewichtszunahme oder -verlust und/oder Fettleibigkeit und/oder Diabetes und/oder kardiovaskulärer Krankheit und/oder hormonaler Dysregulierung des Organismus korreliert und das Verfahren ein Verfahren zum Bestimmen der Neigung, des Risikos oder metabolischen Basis für Gewichtszunahme oder - verlust und/oder Fettleibigkeit und/oder Diabetes und/oder kardiovaskuläre Krankheit und/oder hormonaler Dysregulierungsorganismus ist.

25. Verfahren gemäß Anspruch 24, weiter umfassend das Korrelieren der Quantität des Markers der *de novo* Fettsäuresynthese mit der *de novo* Fettsäuresynthese in Fettgewebe, wobei der Marker der *de novo* Fettsäuresynthese aus der freien Fettsäurefraktion von Blut oder einem Blutprodukt quantifiziert wird.

26. Verfahren gemäß Anspruch 25, weiter umfassend das Korrelieren der Quantität des Markers der *de novo* Fettsäuresynthese mit der *de novo* Fettsäuresynthese in der Leber, wobei der Marker der *de novo* Fettsäuresynthese aus der Phosphatidylcholin-, Triacylglycerid- oder Cholesterinesterfraktion von Blut oder einem Blutprodukt quantifiziert wird.

27. Verfahren gemäß Anpruch 24, welches ein Verfahren zum Bestimmen ist, ob eine Behandlung oder Intervention Gewichtszunahme oder -verlust und/oder Fettleibigkeit bewirkt und/oder kardiovaskuläre Krankheit beeinflußt und/oder hormonale Dysregulierung bewirkt, weiter umfassend das Nehmen von mindestens zwei biologischen Proben aus dem Organismus, wobei die zwei Proben vor und nach einer Ernährungs-, pharmakologischen, genetischen, Umwelt- oder toxikologischen Behandlung oder Intervention genommen werden, wobei eine Änderung in der Quantität des Markers der *de novo* Fettsäuresynthese mit der Wahrscheinlichkeit von Gewichtszunahme oder -verlust und/oder Fettleibigkeit und/oder einer Änderung im diabetischen Zustand eines Organismus und/oder dem Beeinflussen einer kardiovaskulären Krankheit korreliert wird.

28. Verfahren gemäß Anspruch 24, weiter umfassend das Vergleichen der Bewertung der *de novo* Fettsäuresynthese aus dem Organismus mit einer Bewertung der *de novo* Fettsäuresynthese aus einem anderen Organismus oder aufgestellt für eine Population von Organismen.

29. Verfahren gemäß Anspruch 24, wobei die Quantität des Markers der *de novo* Fettsäuresynthese als eine absolute oder relative Konzentration berichtet wird.

30. Verfahren gemäß Anspruch 29, wobei das Korrelieren der Quantität des Markers der *de novo* Fettsäuresynthese das Verwenden der absoluten oder relativen Konzentration des Markers der *de novo* Fettsäuresynthese in einer mathematischen oder statistischen Gleichung zum Bestimmen der Menge der *de novo* Fettsäuresynthese umfaßt.

31. Verfahren gemäß Anspruch 1, wobei das Verfahren ein Verfahren zum Bewerten einer Aktivität von mindestens einem Enzym, das in der *de novo* Fettsäuresynthese involviert ist, ist, weiter umfassend das Korrelieren der Menge des Markers mit der Aktivität des mindestens einen Enzyms.

32. Verfahren gemäß Anspruch 31, wobei das mindestens eine, in der *de novo* Fettsäuresynthese involvierte Enzym Fettsäuresynthase ist.

33. Verfahren gemäß einem der Ansprüche 1 bis 32, weiter umfassend das Generieren eines gedruckten Berichts.

## Revendications

1. Procédé d'évaluation de la synthèse *de novo* d'acides gras dans une cellule, un organisme ou un tissu d'un organisme, comprenant la quantification d'un marqueur de la synthèse *de novo* d'acides gras dans un échantillon biologique provenant de l'organisme, dans lequel le marqueur de la synthèse *de novo* d'acides gras comprend l'acide palmitoléique, l'acide vaccénique, l'acide palmitique, l'acide stéarique, l'acide oléique, l'acide myristique, les acides gras n-7, les acides gras n-9, tous les acides gras saturés, ou une combinaison de deux éléments quelconques ou plus de ceux-ci, dans lequel le marqueur de la synthèse *de novo* d'acides gras est mesuré dans une catégorie de lipides spécifique, et dans lequel l'organisme n'est pas une souris traitée à la rosiglitazone.

2. Procédé selon la revendication 1, dans lequel la catégorie de lipides est constituée par les triacylglycérides, les esters de cholestérol, ou les acides gras libres.

3. Procédé selon la revendication 1, lequel procédé est un procédé d'évaluation de la synthèse *de novo* d'acides gras dans une cellule, et la cellule est une cellule cultivée ; dans un organisme ; dans un tissu d'un organisme ; ou dans du tissu adipeux, du tissu hépatique ou du tissu musculaire.

4. Procédé selon la revendication 3, dans lequel l'organisme est un animal de laboratoire, un animal de compagnie, ou un être humain.

5. Procédé selon la revendication 1, dans lequel l'échantillon biologique est un échantillon de foie, un échantillon de plasma, un échantillon de tissu adipeux, un échantillon de coeur, du sang ou un produit sanguin.

6. Procédé selon la revendication 5, dans lequel l'échantillon biologique est du sang ou un produit sanguin et le marqueur de la synthèse *de novo* d'acides gras est quantifié à partir de la fraction d'acides gras libres du sang ou produit sanguin et le procédé est un procédé permettant d'évaluer la synthèse *de novo* d'acides gras dans du tissu adipeux.

7. Procédé selon la revendication 5, dans lequel l'échantillon biologique est du sang ou un produit sanguin et le marqueur de la synthèse *de novo* d'acides gras est quantifié à partir de la fraction de phosphatidylcholines, de triacylglycérides ou d'esters de cholestérol du sang ou produit sanguin, et le procédé est un procédé permettant d'évaluer la synthèse *de novo* d'acides gras dans du tissu hépatique.

8. Procédé selon la revendication 7, dans lequel le marqueur de la synthèse *de novo* d'acides gras est quantifié à partir de la fraction d'esters de cholestérol du sang ou produit sanguin.

9. Procédé selon la revendication 9, comprenant la quantification de l'acide palmitoléique et de l'acide palmitique dans un échantillon biologique provenant de l'organisme.

10. Procédé selon la revendication 9, comprenant en outre la production d'un indicateur de rapport de la synthèse *de novo* d'acides gras, dans lequel l'indicateur de rapport est le rapport de la quantité d'acide palmitoléique à la quantité d'acide palmitique.

11. Procédé selon la revendication 10, comprenant en outre la comparaison de l'indicateur de rapport de l'échantillon biologique avec un indicateur de rapport d'un échantillon de base ou témoin.

12. Procédé selon la revendication 1, comprenant la quantification des acides gras n-7 totaux et des acides gras saturés totaux dans un échantillon biologique provenant de l'organisme.

13. Procédé selon la revendication 12, comprenant en outre la production d'un indicateur de rapport de la synthèse *de novo* d'acides gras, dans lequel l'indicateur de rapport est le rapport de la quantité d'acides gras n-7 totaux à la quantité d'acides gras saturés totaux.

14. Procédé selon la revendication 13, comprenant en outre la comparaison de l'indicateur de rapport de l'échantillon biologique avec un indicateur de rapport d'un échantillon de base ou témoin.

15. Procédé selon la revendication 1, comprenant la quantification des acides gras n-7 totaux et des acides gras n-9 totaux dans un échantillon biologique provenant de l'organisme.

16. Procédé selon la revendication 15, comprenant en outre la production d'un indicateur de rapport de la synthèse *de novo* d'acides gras, dans lequel l'indicateur de rapport est le rapport de la quantité d'acides gras n-7 totaux à la quantité d'acides gras n-9 totaux.

17. Procédé selon la revendication 16, comprenant en outre la comparaison de l'indicateur de rapport de l'échantillon biologique avec un indicateur de rapport d'un échantillon de base ou témoin.

18. Procédé selon la revendication 1, lequel procédé est
(1) un procédé permettant de déterminer si un traitement, régime ou dosage pharmaceutique, nutritionnel, génétique, toxicologique ou environnemental influe sur la synthèse *de novo* d'acides gras ;
(2) un procédé permettant d'évaluer l'efficacité, l'activité ou les effets secondaires d'un agent thérapeutique ou pharmaceutique en matière de synthèse *de novo* d'acides gras ; ou
(3) un procédé permettant de dépister les individus présentant une compatibilité ou incompatibilité avec un traitement pharmaceutique, nutritionnel, toxicologique ou environnemental.

19. Procédé selon la revendication 1, comprenant la quantification de l'acide palmitoléique dans un échantillon biologique provenant de l'organisme, l'échantillon biologique étant de préférence du sang ou un produit sanguin.

20. Procédé selon la revendication 1, comprenant la quantification de l'acide stéarique et de l'acide palmitique dans un échantillon biologique provenant de l'organisme.

21. Procédé selon la revendication 20, comprenant en outre la production d'un indicateur de rapport de la synthèse *de novo* d'acides gras, dans lequel l'indicateur de rapport est le rapport de la quantité d'acide stéarique à la quantité d'acide palmitique.

22. Procédé selon la revendication 1, lequel procédé est un procédé permettant d'évaluer une variation de la synthèse *de novo* d'acides gras dans l'organisme, et lequel procédé comprend le prélèvement d'au moins deux échantillons biologiques dans l'organisme, dans lequel les deux échantillons sont prélevés avant et après un événement.

23. Procédé selon la revendication 22, dans lequel l'événement comprend un certain laps de temps, un traitement avec un agent thérapeutique, un traitement avec un agent pharmaceutique, un traitement avec un régime nutritionnel, un traitement avec une modification génétique, une exposition à un composé toxique ou potentiellement toxique, une exposition à une condition environnementale, un traitement avec une procédure de laboratoire, un effort, ou l'apparition d'un état phénotypique.

24. Procédé selon la revendication 1, dans lequel la quantité du marqueur de la synthèse *de novo* d'acides gras est corrélée à une propension, un risque, ou un fondement métabolique en matière de gain ou de perte de poids et/ou d'obésité et/ou de diabète et/ou de maladie cardiovasculaire et/ou de dysrégulation hormonale de l'organisme, et le procédé est un procédé permettant de déterminer la propension, le risque, ou le fondement métabolique en matière de gain ou de perte de poids et/ou d'obésité et/ou de diabète et/ou de maladie cardiovasculaire et/ou de dysrégulation hormonale de l'organisme.

25. Procédé selon la revendication 24, comprenant en outre la corrélation de la quantité du marqueur de la synthèse *de novo* d'acides gras avec la synthèse *de novo* d'acides gras dans le tissu adipeux, dans lequel le marqueur de la synthèse *de novo* d'acides gras est quantifié à partir de la fraction d'acides gras libres du sang ou d'un produit sanguin.

26. Procédé selon la revendication 25, comprenant en outre la corrélation de la quantité du marqueur de la synthèse *de novo* d'acides gras avec la synthèse *de novo* d'acides gras dans le foie, dans lequel le marqueur de la synthèse *de novo* d'acides gras est quantifié à partir de la fraction de phosphatidylcholines, de triacylglycérides ou d'esters de cholestérol du sang ou d'un produit sanguin.

27. Procédé selon la revendication 24, lequel est un procédé permettant de déterminer si un traitement ou une intervention entraînera un gain ou une perte de poids et/ou une obésité et/ou influera sur une maladie cardiovasculaire et/ou entraînera une dysrégulation hormonale, comprenant en outre le prélèvement d'au moins deux échantillons biologiques dans l'organisme, dans lequel les deux échantillons sont prélevés avant et après un traitement ou intervention nutritionnel, pharmacologique, génétique, environnemental ou toxicologique, et dans lequel une variation de la quantité du marqueur de la synthèse *de novo* d'acides gras est corrélée avec une probabilité de gain ou de perte de poids et/ou d'obésité et/ou de variation de l'état diabétique d'un organisme et/ou d'influence sur une maladie cardiovasculaire.

28. Procédé selon la revendication 24, comprenant en outre la comparaison de l'évaluation de la synthèse *de novo* d'acides gras de l'organisme à une évaluation de la synthèse *de novo* d'acides gras d'un autre organisme ou compilée pour une population d'organismes.

29. Procédé selon la revendication 24, dans lequel la quantité du marqueur de la synthèse *de novo* d'acides gras est rapportée sous forme de concentration absolue ou relative.

30. Procédé selon la revendication 29, dans lequel la corrélation de la quantité du marqueur de la synthèse *de novo* d'acides gras comprend l'utilisation de la concentration absolue ou relative du marqueur de la synthèse *de novo* d'acides gras dans une équation mathématique ou statistique pour déterminer la quantité de synthèse *de novo* d'acides gras.

31. Procédé selon la revendication 1, lequel procédé est un procédé d'évaluation de l'activité d'au moins une enzyme impliquée dans la synthèse *de novo* d'acides gras, comprenant en outre la corrélation de la quantité du marqueur avec l'activité de ladite au moins une enzyme.

32. Procédé selon la revendication 31, dans lequel ladite au moins une enzyme impliquée dans la synthèse *de novo* d'acides gras est une synthase d'acide gras.

33. Procédé selon l'une quelconque des revendications 1 à 32, comprenant en outre la production d'un rapport imprimé.
